# NEW EUROPEAN PATENT SPECIFICATION

(11) **EP 1 787 991 B2**
(45) Date of publication and mention of the opposition decision: **24.06.2020**
(45) Mention of the grant of the patent: 05.10.2011
(21) Application number: 05768664.4
(22) Date of filing: 28.07.2005
(51) Int. Cl.: C07D 471/04, A61K 31/437, A61K 31/444, A61P 1/00, A61P 1/04, A61P 1/06, A61P 7/04, A61P 31/04, A61P 35/00, A61P 43/00, A61K 31/5377, A61K 31/4436

(54) **PYRROLO[2,3-c]PYRIDINE COMPOUND, PROCESS FOR PRODUCING THE SAME, AND USE**
PYRROLO[2,3-c]PYRIDINVERBINDUNG, VERFAHREN ZU DEREN HERSTELLUNG UND ANWENDUNG
DERIVES DE PYRROLO[2,3-c]PYRIDINE, PROCEDE POUR LEUR PREPARATION ET LEUR UTILISATION

(30) Priority: 28.07.2004 JP 2004220788
(43) Date of publication of application: 23.05.2007
(73) Proprietor: Takeda Pharmaceutical Company Limited, Osaka-shi, Osaka 541-0045 (JP)
(72) Inventor: HASUOKA, Atsushi, c/o Takeda Pharmaceutical Co. Ltd., Osaka-shi, Osaka 5328686 (JP); ARIKAWA, Yasuyoshi, c/o Takeda Pharmaceutical Co. Ltd., Osaka-shi, Osaka 5328686 (JP)
(74) Representative: Jones, Nicholas Andrew
(86) International application number: PCT/JP2005/014263
(87) International publication number: WO 2006/011670

(56) References cited:
- EP-A- 1 209 158
- EP-B1- 1 784 404
- WO-A-03/082280
- WO-A-2005/097129
- WO-A1-2005/121140
- WO-A2-01/58869
- JP-A- 6 247 967
- JP-A- H06 247 967
- JP-A- 2001 294 587
- JP-A- 2001 512 477
- JP-A- 2001 525 322
- JP-A- 2004 502 642
- US-A- 4 241 064
- US-A- 6 124 306
- US-A1- 2003 096 825
- KAMINSKI J J ET AL: "ANTIULCER AGENTS. 2. GASTRIC ANTISECRETORY, CYTOPROTECTIVE, AND METABOLIC PROPERTIES OF SUBSTITUTED IMIDAZOÚ1,2-A 3/4 PYRIDINES AND ANALOGUES" JOURNAL OF MEDICINAL CHEMISTRY, US AMERICAN CHEMICAL SOCIETY. WASHINGTON, vol. 30, no. 11, 1 January 1987 (1987-01-01), pages 2031-2046, XP002008621 ISSN: 0022-2623
- KAMINSKI J J ET AL: "ANTIULCER AGENTS. 5. INHIBITION OF GASTRIC H+/K+-ATPASE BY SUBSITUTED IMIDAZOL not 1,2-A 3/4 PYRIDINES AND RELATED ANALOGUES AND ITS IMPLICATION IN MODELING THE HIGH AFFINITY POTASSIUM ION BINDING SITE OF THE GASTRIC PROTON PUMP ENZYME" JOURNAL OF MEDICINAL CHEMISTRY, US AMERICAN CHEMICAL SOCIETY. WASHINGTON, vol. 34, 1 January 1991 (1991-01-01), pages 533-541, XP000919185 ISSN: 0022-2623
- SONG Y ET AL: "beta-Carbolines as specific inhibitors of cyclin-dependent kinases" BIOORGANIC & MEDICINAL CHEMISTRY LETTERS, OXFORD, GB, vol. 12, no. 7, 8 April 2002 (2002-04-08), pages 1129-1132, XP002330760 ISSN: 0960-894X

## Description

### Technical Field

The present invention relates to a pyrrolo[2,3-c]pyridine compound, a production method thereof and use thereof.

### Background Art

For the aim of treating peptic ulcer and reflux esophagitis and the like, proton pump inhibitors represented by omeprazole that suppresses secretion of gastric acid have been widely used in clinical situations. However, existing proton pump inhibitors have problems in terms of effect and side effect. To be specific, since existing proton pump inhibitors are unstable under acidic conditions, they are often formulated as enteric-coated preparations, and in that case, several hours are necessary for the expression of action. In addition, since existing proton pump inhibitors are concerned to show inconsistent treatment effects based on metabolic enzyme polymorphism and drug interaction with pharmaceutical agents such as diazepam and the like, improvements thereof are desired.

As a pyrrolo[2,3-c]pyridine compound having a proton pump inhibitory action, JP-A-6-247967 discloses a compound represented by the formula:

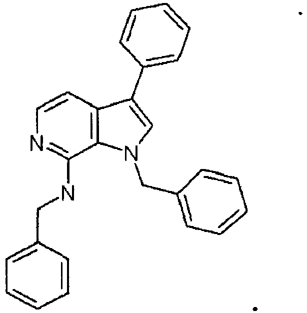

In addition, as an imidazo[1,2-a]pyridine compound having a proton pump inhibitory action, WO03/018582 discloses a compound represented by the formula:

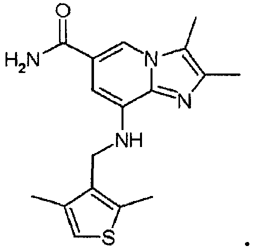

WO03/082280 discloses pyrrolo[2,3-c]pyridines said to have glucocorticoid activity. The disclosed compounds do not have substituents at the 7-position. WO99/28322 describes gastric acid secretion inhibitors having heterocyclic ring moieties other than pyrrolo[2,3-c]pyridine. The same is true of WO98/37080, Kaminski et al. (J. Med. Chem (1987) 30, 2031) and Kaminski et al. (J.Med.Chem (1991) 34, 533). US 6,124,306 discloses a pyrrolo[2,3-c]pyridine said to be useful in the treatment of HIV. WO01/58869 discloses pyrrolo[2,3-c]pyridines as cannabinoid receptor modulators. WO2005/097129 (prior art under Art. 54(3) EPC) discloses similar compounds as kinase inhibitors. EP1209158 and Song et al. (Bioorg. Med. Chem Lett (2002) 12, 1129) disclose β-carbolines and their use as kinase inhibitors. Further tricyclic molecules are disclosed in US 4,241,064, specifically as inhibitors of xanthine oxidase.

### Disclosure of the Invention

A pharmaceutical agent which, like known proton pump inhibitors, effectively suppresses gastric acid secretion and has improved instability under acidic conditions, inconsistent effect based on metabolic enzyme polymorphism and interaction of pharmaceutical agents, which are the problems of known proton pump inhibitors, is expected to provide a more superior treatment effect on peptic ulcer and reflux esophagitis and the like. At present, however, a proton pump inhibitor that fully satisfies these requirements has not been found. Accordingly, an object of the present invention is to provide a compound that has improved these problems and has a proton pump inhibitory action, as well as a production method thereof and use thereof.

The present inventors have conducted various studies and first succeeded in generating a compound represented by the formula (I): 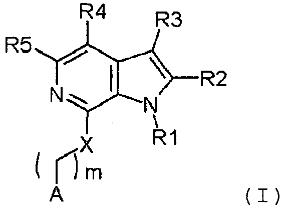 wherein R1 is (i) a hydrogen atom, or (ii) a hydrocarbon group selected from a C₁₋₆ alkyl group, a C₂₋₆ alkenyl group, a C₂₋₆ alkynyl group, a C₃₋₇ cycloalkyl group, a C₆₋₁₄ aryl group, a C₇₋₁₆ aralkyl group, and a group represented by the formula: 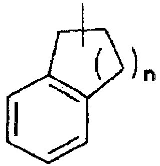 wherein n is 1 or 2, each of which is optionally substituted by substituent(s) selected from (1) halogen atom, (2) nitro, (3) cyano, (4) hydroxy, (5) C₁₋₆ alkoxy optionally having 1 to 3 halogen atoms, (6) C₆₋₁₄ aryloxy, (7) C₇₋₁₆ aralkyloxy, (8) mercapto, (9) C₁₋₆ alkylthio optionally having 1 to 3 halogen atoms, (10) C₆₋₁₄ arylthio, (11) C₇₋₁₆ aralkylthio, (12) amino, (13) mono-C₁₋₆ alkylamino, (14) mono-C₆₋₁₄ arylamino, (15) mono-C₇₋₁₆ aralkylamino, (16) di-C₁₋₆ alkylamino, (17) di-C₆₋₁₄ arylamino, (18) di-C₇₋₁₆ aralkylamino, (19) formyl, (20) C₁₋₆ alkyl-carbonyl, (21) C₆₋₁₄ aryl-carbonyl, (22) carboxyl, (23) C₁₋₆ alkoxy-carbonyl, (24) C₆₋₁₄ aryloxy-carbonyl, (25) carbamoyl, (26) thiocarbamoyl, (27) mono-C₁₋₆ alkyl-carbamoyl, (28) di-C₁₋₆ alkyl-carbamoyl, (29) C₆₋₁₄ aryl-carbamoyl, (30) C₁₋₆ alkylsulfonyl, (31) C₆₋₁₄ arylsulfonyl, (32) C₁₋₆ alkylsulfinyl, (33) C₆₋₁₄ arylsulfinyl, (34) formylamino, (35) C₁₋₆ alkyl-carbonylamino, (36) C₆₋₁₄ aryl-carbonylamino, (37) C₁₋₆ alkoxy-carbonylamino, (38) C₁₋₆ alkylsulfonylamino, (39) C₆₋₁₄ arylsulfonylamino, (40) C₁₋₆ alkyl-carbonyloxy, (41) C₆₋₁₄ aryl-carbonyloxy, (42) C₁₋₆ alkoxy-carbonyloxy, (43) mono-C₁₋₆ alkyl-carbamoyloxy, (44) di-C₁₋₆ alkyl-carbamoyloxy, (45) C₆₋₁₄ aryl-carbamoyloxy, (46) a 5- to 10-membered heterocyclic group containing, besides carbon atom, one or two kinds of 1 to 4 hetero atoms selected from nitrogen atom, sulfur atom and oxygen atom, (47) C₁₋₃ alkylenedioxy, (48) C₃₋₇ cycloalkyl,
R2 is a C₁₋₄ alkyl group,
R3 is a C₁₋₄ alkyl group,
R4 and R5 are the same or different and each is a hydrogen atom, or a C₁₋₆ alkyl group,
X is a bond, O, or 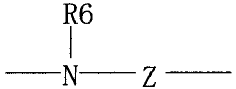 [wherein R6 is a hydrogen atom or a C₁₋₆ alkyl group, and
Z is a bond or -CO-],
m is 0 or 1,
A is a) a hydrocarbon group selected from i) a C₆₋₁₀ aryl group optionally substituted by 1 to 4 substituents selected from C₁₋₆ alkyl optionally substituted by 1 to 5 halogens, C₁₋₆ alkoxy, cyano and halogen atom, ii) C₇₋₁₆ aralkyl group optionally substituted by 1 to 4 substituents selected from C₁₋₆ alkyl optionally substituted by 1 to 5 halogens, C₁₋₆ alkoxy, cyano and halogen atom, and iii) a group represented by the formula: 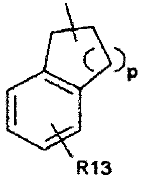 wherein p is 1 or 2, and R13 is (i) a hydrogen atom or (ii) a C₁₋₆ alkyl group optionally substituted by 1 to 4 substituents selected from hydroxy, cyano and C₁₋₆ alkoxy, or b) a 5- to 14-membered monocyclic or bicyclic heterocyclic group containing, besides carbon atom, one or two kinds of 1 to 4 hetero atoms selected from nitrogen atom, oxygen atom and sulfur atom, optionally substituted by 1 to 3 substituent(s) selected from (1) halogen atom, (2) nitro, (3) cyano, (4) C₁₋₆ alkyl optionally having 1 to 3 halogen atoms, (5) C₆₋₁₄ aryl, (6) hydroxy, (7) C₁₋₆ alkoxy optionally having 1 to 3 halogen atoms, (8) C₆₋₁₄ aryloxy, (9) C₇₋₁₆ aralkyloxy, (10) mercapto, (11) C₁₋₆ alkylthio optionally having 1 to 3 halogen atoms, (12) C₆₋₁₄ arylthio, (13) C₇₋₁₆ aralkylthio, (14) amino, (15) mono-C₁₋₆ alkylamino, (16) mono-C₆₋₁₄ arylamino, (17) mono-C₇₋₁₆ aralkylamino, (18) di-C₁₋₆ alkylamino, (19) di-C₆₋₁₄ arylamino, (20) di-C₇₋₁₆ aralkylamino, (21) formyl, (22) C₁₋₆ alkyl-carbonyl, (23) C₆₋₁₄ aryl-carbonyl, (24) carboxyl, (25) C₁₋₆ alkoxy-carbonyl, (26) C₆₋₁₄ aryloxy-carbonyl, (27) carbamoyl, (28) thiocarbamoyl, (29) mono-C₁₋₆ alkyl-carbamoyl, (30) di-C₁₋₆ alkyl-carbamoyl, (31) C₆₋₁₄ aryl-carbamoyl, (32) C₁₋₆ alkylsulfonyl, (33) C₆₋₁₄ arylsulfonyl, (34) C₁₋₆ alkylsulfinyl, (35) C₆₋₁₄ arylsulfinyl, (36) formylamino, (37) C₁₋₆ alkyl-carbonylamino, (38) C₆₋₁₄ aryl-carbonylamino, (39) C₁₋₆ alkoxy-carbonylamino, (40) C₁₋₆ alkylsulfonylamino, (41) C₆₋₁₄ arylsulfonylamino, (42) C₁₋₆ alkyl-carbonyloxy, (43) C₆₋₁₄ aryl-carbonyloxy, (44) C₁₋₆ alkoxy-carbonyloxy, (45) mono-C₁₋₆ alkyl-carbamoyloxy, (46) di-C₁₋₆ alkyl-carbamoyloxy, (47) C₆₋₁₄ aryl-carbamoyloxy, (48) 5- to 7-membered saturated cyclic amino optionally containing one nitrogen atom and, besides carbon atom, one or two kinds of 1 to 4 hetero atoms selected from nitrogen atom, sulfur atom and oxygen atom, (49) a 5- to 10-membered aromatic heterocyclic group containing, besides carbon atom, one or two kinds of 1 to 4 hetero atoms selected from nitrogen atom, sulfur atom and oxygen atom, (50) C₁₋₃ alkylenedioxy, (51) C₃₋₇ cycloalkyl] or a salt thereof [hereinafter to be abbreviated as compound (I)], and further, a proton pump inhibitor as defined in appended claim 9, and found that the compound unexpectedly has a very potent proton pump inhibitory action and is fully satisfactory as a pharmaceutical agent. Based on these findings, they completed the present invention.

Accordingly, the present invention relates to
[1] compound (I),
[2] The compound of the above-mentioned [1], wherein R1 is i) a hydrogen atom, ii) a C₁₋₆ alkyl group optionally substituted by substituent(s) selected from halogen atom, hydroxy, mono-C₁₋₆ alkylamino, di-C₁₋₆ alkylamino, C₁₋₆ alkoxy, C₇₋₁₆ aralkyloxy, C₃₋₇ cycloalkyl and 5- or 6-membered heterocyclic group, iii) a C₂₋₆ alkenyl group or iv) a C₇₋₁₆ aralkyl group optionally substituted by C₁₋₆ alkoxy,
[3] The compound of the above-mentioned [1], wherein m is 1,
[4] The compound of the above-mentioned [1], wherein A is i) a C₆₋₁₄ aryl group optionally substituted by substituent(s) selected from C₁₋₆ alkyl optionally substituted by halogen, C₁₋₆ alkoxy, cyano and halogen atom, ii) a 5- or 6-membered heterocyclic group optionally substituted by substituent(s) selected from C₁₋₆ alkyl, C₁₋₆ alkoxy, cyano and halogen atom, iii) a 2,3-dihydro-1H-inden-1-yl group or iv) a 1,2,3,4-tetrahydronaphthalen-1-yl group,
[5] The compound of the above-mentioned [1], which is selected from
   N-(2,3-dihydro-1H-inden-1-yl)-2,3-dimethyl-1H-pyrrolo[2,3-c]pyridine-7-amine,
   N-(4-fluoro-2-methylbenzyl)-2,3-dimethyl-1-propyl-1H-pyrrolo[2,3-c]pyridine-7-amine,
      and
   N-[7-(2,3-dimethyl-1H-pyrrolo[2,3-c]pyridyl)]benzamide,
[6] A production method of a compound represented by the formula: 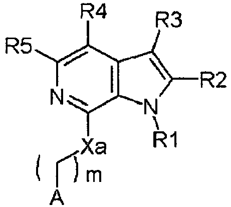 Wherein R1, R2, R3, R4, R5, m and A are as defined in the above-mentioned [1],
   Xa is O, or 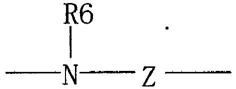 [wherein R6 is a hydrogen atom or a C₁₋₆ alkyl group, and Z is a bond or -CO-], or a salt thereof, which comprises reacting a compound represented by the formula: 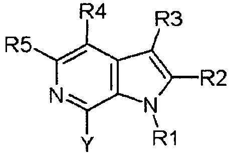 wherein Y is a leaving group, and other symbols are as defined in the above-mentioned [1], or a salt thereof, with a compound represented by formula: 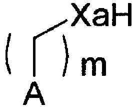 wherein Xa is as defined above, and m and A are as defined in the above-mentioned [1], or a salt thereof,
[7] A compound represented by the formula: 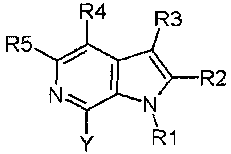 wherein Y is a leaving group, and other symbols are as defined in the above-mentioned [1], or a salt thereof,
[8] A pharmaceutical agent comprising the compound of the above-mentioned [1],
[9] A proton pump inhibitor comprising a compound represented by the formula (I): 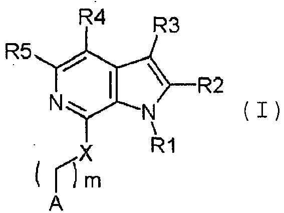 as defined in the above-mentioned [1], or a salt thereof,
[10] The pharmaceutical agent of the above-mentioned [8], which is an agent for the treatment or prophylaxis of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress; a Helicobacter pylori eradication agent; or a suppressant of upper gastrointestinal hemorrhage due to peptic ulcer, acute stress ulcer, hemorrhagic gastritis or invasive stress,
[11] Use of the compound of the above-mentioned [1] for the production of an agent for the treatment or prophylaxis of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress; a Helicobacter pylori eradication agent; or a suppressant of upper gastrointestinal hemorrhage due to peptic ulcer, acute stress ulcer, hemorrhagic gastritis or invasive stress.

Since compound (I) shows a proton pump inhibitory action, it can provide a clinically useful agent for the prophylaxis or treatment of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress ; a Helicobacter pylori eradication agent; or a suppressant of upper gastrointestinal hemorrhage due to peptic ulcer, acute stress ulcer, hemorrhagic gastritis or invasive stress. Since compound (I) shows low toxicity and is superior in water-solubility, in vivo kinetics and efficacy expression, they are useful as pharmaceutical agents. Moreover, since compound (I) is stable even under acidic conditions, it can be orally administered as normal tablets without forming an enteric coated preparation. As a result, since the preparation such as tablet can be downsized, an advantage of easy administration to sick people with weak swallowing power, particularly the elderly and children can be afforded. Moreover, since a sustained release effect like that of an enteric coated preparation is not provided, a gastric acid secretion-inhibitory action is rapidly expressed and the symptoms such as pain are rapidly improved.

### Best Mode for Embodying the Invention

As the "hydrocarbon group" for R1, alkyl, alkenyl, alkynyl, cycloalkyl, aryl, aralkyl, a group represented by the formula: 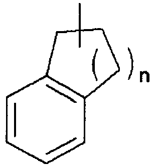 wherein n is 1 or 2, etc. are included.

The "alkyl" is C₁₋₆ alkyl (e.g., methyl, ethyl, propyl, isopropyl, butyl, isobutyl, sec-butyl, tert-butyl, pentyl, hexyl etc.).

The "alkenyl" is C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl etc.).

The "alkynyl" is C₂₋₆ alkynyl (e.g., ethynyl, propargyl, 1-butynyl, 2-butynyl, 3-butynyl, 1-hexynyl etc.).

The "cycloalkyl" is C₃₋₇ cycloalkyl (e.g., cyclopropyl, cyclobutyl, cyclopentyl, cyclohexyl, cycloheptyl etc.).

The "aryl" is C₆₋₁₄ aryl (e.g., phenyl, 1-naphthyl, 2-naphthyl, 2-biphenylyl, 3-biphenylyl, 4-biphenylyl, 2-anthryl etc.).

The "aralkyl" is C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 1-phenylethyl etc.).

As the group represented by the formula: 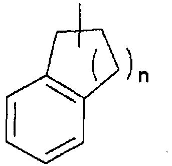 wherein the symbols in the formula are as defined above, 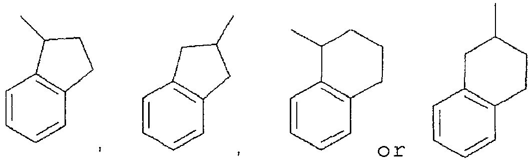 can be mentioned.

As the substituent of the "hydrocarbon group" for R1, 1 to 3 selected from
(1) halogen atom (e.g., fluorine atom, chlorine atom, bromine atom, iodine atom),
(2) nitro,
(3) cyano,
(4) hydroxy,
(5) C₁₋₆ alkoxy (e.g., methoxy, ethoxy, propoxy, isopropoxy, butoxy, isobutoxy, sec-butoxy, pentyloxy, hexyloxy, fluoromethoxy etc.) optionally having 1 to 3 halogen atoms (e.g., fluorine, chlorine, bromine, iodine),
(6) C₆₋₁₄ aryloxy (e.g., phenyloxy, naphthyloxy etc.),
(7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.),
(8) mercapto,
(9) C₁₋₆ alkylthio (e.g., methylthio, difluoromethylthio, trifluoromethylthio, ethylthio, propylthio, isopropylthio, butylthio, 4,4,4-trifluorobutylthio, pentylthio, hexylthio etc.) optionally having 1 to 3 halogen atoms (e.g., fluorine, chlorine, bromine, iodine),
(10) C₆₋₁₄ arylthio (e.g., phenylthio, naphthylthio etc.),
(11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.),
(12) amino,
(13) mono-C₁₋₆ alkylamino (e.g., methylamino, ethylamino etc.),
(14) mono-C₆₋₁₄ arylamino (e.g., phenylamino, 1-naphthylamino, 2-naphthylamino etc.),
(15) mono-C₇₋₁₆ aralkylamino (e.g., benzylamino etc.),
(16) di-C₁₋₆ alkylamino (e.g., dimethylamino, diethylamino etc.),
(17) di-C₆₋₁₄ arylamino (e.g., diphenylamino etc.),
(18) di-C₇₋₁₆ aralkylamino (e.g., dibenzylamino etc.),
(19) formyl,
(20) C₁₋₆ alkyl-carbonyl (e.g., acetyl, propionyl etc.),
(21) C₆₋₁₄ aryl-carbonyl (e.g., benzoyl, 1-naphthoyl, 2-naphthoyl etc.),
(22) carboxyl,
(23) C₁₋₆ alkoxy-carbonyl (e.g., methoxycarbonyl, ethoxycarbonyl, propoxycarbonyl, tert-butoxycarbonyl etc.),
(24) C₆₋₁₄ aryloxy-carbonyl (e.g., phenoxycarbonyl etc.),
(25) carbamoyl,
(26) thiocarbamoyl,
(27) mono-C₁₋₆ alkyl-carbamoyl (e.g., methylcarbamoyl, ethylcarbamoyl etc.),
(28) di-C₁₋₆ alkyl-carbamoyl (e.g., dimethylcarbamoyl, diethylcarbamoyl, ethylmethylcarbamoyl etc.),
(29) C₆₋₁₄ aryl-carbamoyl (e.g., phenylcarbamoyl, 1-naphthylcarbamoyl, 2-naphthylcarbamoyl etc.),
(30) C₁₋₆ alkylsulfonyl (e.g., methylsulfonyl, ethylsulfonyl etc.),
(31) C₆₋₁₄ arylsulfonyl (e.g., phenylsulfonyl, 1-naphthylsulfonyl, 2-naphthylsulfonyl etc.),
(32) C₁₋₆ alkylsulfinyl (e.g., methylsulfinyl, ethylsulfinyl etc.),
(33) C₆₋₁₄ arylsulfinyl (e.g., phenylsulfinyl, 1-naphthylsulfinyl, 2-naphthylsulfinyl etc.),
(34) formylamino,
(35) C₁₋₆ alkyl-carbonylamino (e.g., acetylamino etc.),
(36) C₆₋₁₄ aryl-carbonylamino (e.g., benzoylamino, naphthoylamino etc.),
(37) C₁₋₆ alkoxy-carbonylamino (e.g., methoxycarbonylamino, ethoxycarbonylamino, propoxycarbonylamino, butoxycarbonylamino etc.),
(38) C₁₋₆ alkylsulfonylamino (e.g., methylsulfonylamino, ethylsulfonylamino etc.),
(39) C₆₋₁₄ arylsulfonylamino (e.g., phenylsulfonylamino, 2-naphthylsulfonylamino, 1-naphthylsulfonylamino etc.),
(40) C₁₋₆ alkyl-carbonyloxy (e.g., acetoxy, propionyloxy etc.),
(41) C₆₋₁₄ aryl-carbonyloxy (e.g., benzoyloxy, naphthylcarbonyloxy etc.),
(42) C₁₋₆ alkoxy-carbonyloxy (e.g., methoxycarbonyloxy, ethoxycarbonyloxy, propoxycarbonyloxy, butoxycarbonyloxy etc.),
(43) mono-C₁₋₆ alkyl-carbamoyloxy (e.g., methylcarbamoyloxy, ethylcarbamoyloxy etc.),
(44) di-C₁₋₆ alkyl-carbamoyloxy (e.g., dimethylcarbamoyloxy, diethylcarbamoyloxy etc.),
(45) C₆₋₁₄ aryl-carbamoyloxy (e.g., phenylcarbamoyloxy, naphthylcarbamoyloxy etc.),
(46) a 5- to 10-membered heterocyclic group containing, besides carbon atom, one or two kinds of 1 to 4 hetero atoms selected from nitrogen atom, sulfur atom and oxygen atom (e.g., non-aromatic heterocyclic groups such as pyrrolidin-1-yl, piperidino, piperazin-1-yl, morpholino, thiomorpholino, hexahydroazepin-1-yl ; aromatic heterocyclic group such as 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl , , preferably a 5- or 6-membered heterocyclic group),
(47) C₁₋₃ alkylenedioxy (e.g., methylenedioxy, ethylenedioxy etc.),
(48) C₃₋₇ cycloalkyl (e.g., cyclopropyl, cyclobutyl, cyclopentyl, cyclohexyl, cycloheptyl etc.)
are optional substituents.

In the present specification, the substituent of the "hydrocarbon group" of the "hydrocarbon group optionally substituted by substituent(s)" does not include an oxo group.

As the "hydrocarbon group" for R1, (i) a C₁₋₆ alkyl group optionally substituted by substituent(s) selected from halogen atom, hydroxy, mono-C₁₋₆ alkylamino, di-C₁₋₆ alkylamino, C₁₋₆ alkoxy, C₇₋₁₆ aralkyloxy, C₃₋₇ cycloalkyl and 5- or 6-membered heterocyclic group (e.g., piperazin-1-yl, morpholino, 2-pyridyl etc.), (ii) a C₂₋₆ alkenyl group or (iii) a C₇₋₁₆ aralkyl group optionally substituted by C₁₋₆ alkoxy are preferable.

The "heterocyclic group" of the "optionally substituted heterocyclic group" for A is a 5- to 14-membered (preferably 5- to 10-membered, more preferably 5- or 6-membered) monocyclic or bicyclic heterocyclic group containing, besides carbon atom, one or two kinds of 1 to 4 (preferably 1 to 3) hetero atoms selected from nitrogen atom, oxygen atom and sulfur atom.

For example, a 5-membered cyclic group containing, besides carbon atom, 1 to 4 hetero atoms selected from oxygen atom, sulfur atom and nitrogen atom such as 2- or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3- or 4-pyrazolidinyl, 2-, 4- or 5-imidazolyl, 1,2,3-triazolyl, 1,2,4-triazolyl, 1H- or 2H-tetrazolyl , a 6-membered cyclic group containing, besides carbon atom, 1 to 4 hetero atoms selected from oxygen atom, sulfur atom and nitrogen atom such as 2-, 3- or 4-pyridyl, N-oxido-2-, 3- or 4-pyridyl, 2-, 4- or 5-pyrimidinyl, N-oxido-2-, 4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, N-oxido-3- or 4-pyridazinyl , a group formed by condensing a 5- or 6-membered cyclic group containing, besides carbon atom, 1 to 4 hetero atoms selected from oxygen atom, sulfur atom and nitrogen atom and one 5- or 6-membered ring (e.g., benzene ring etc.) containing, besides carbon atom, 1 to 4 hetero atoms selected from oxygen atom, sulfur atom and nitrogen atom, such as indolyl, benzofuryl, benzothiazolyl, benzoxazolyl, benzimidazolyl, quinolyl, isoquinolyl, phthalazinyl, quinazolinyl, quinoxalinyl, indolizinyl, quinolizinyl, 1,8-naphthyridinyl, chromanyl, phenothiazinyl, phenoxazinyl , can be mentioned.

Of these, a 5- or 6-membered heterocyclic group containing, besides carbon atom, 1 to 3 hetero atoms selected from oxygen atom, sulfur atom and nitrogen atom are preferable.

As the substituent of the aforementioned "heterocyclic group", 1 to 3 selected from
(1) halogen atom (e.g., fluorine, chlorine, bromine, iodine),
(2) nitro,
(3) cyano,
(4) C₁₋₆ alkyl optionally having 1 to 3 halogen atoms (e.g., fluorine, chlorine, bromine, iodine) (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl etc.),
(5) C₆₋₁₄ aryl (e.g., phenyl, 1-naphthyl, 2-naphthyl, biphenylyl, 2-anthryl etc.),
(6) hydroxy,
(7) C₁₋₆ alkoxy optionally having 1 to 3 halogen atoms (e.g., fluorine, chlorine, bromine, iodine) (e.g., methoxy, ethoxy, propoxy, isopropoxy, butoxy, isobutoxy, sec-butoxy, pentyloxy, hexyloxy, fluoromethoxy etc.),
(8) C₆₋₁₄ aryloxy (e.g., phenyloxy, naphthyloxy etc.),
(9) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.),
(10) mercapto,
(11) C₁₋₆ alkylthio optionally having 1 to 3 halogen atoms (e.g., fluorine, chlorine, bromine, iodine) (e.g., methylthio, difluoromethylthio, trifluoromethylthio, ethylthio, propylthio, isopropylthio, butylthio, 4,4,4-trifluorobutylthio, pentylthio, hexylthio etc.),
(12) C₆₋₁₄ arylthio (e.g., phenylthio, naphthylthio etc.),
(13) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.),
(14) amino,
(15) mono-C₁₋₆ alkylamino (e.g., methylamino, ethylamino etc.),
(16) mono-C₆₋₁₄ arylamino (e.g., phenylamino, 1-naphthylamino, 2-naphthylamino etc.),
(17) mono-C₇₋₁₆ aralkylamino (e.g., benzylamino etc.),
(18) di-C₁₋₆ alkylamino (e.g., dimethylamino, diethylamino etc.),
(19) di-C₆₋₁₄ arylamino (e.g., diphenylamino etc.),
(20) di-C₇₋₁₆ aralkylamino (e.g., dibenzylamino etc.),
(21) formyl,
(22) C₁₋₆ alkyl-carbonyl (e.g., acetyl, propionyl etc.),
(23) C₆₋₁₄ aryl-carbonyl (e.g., benzoyl, 1-naphthoyl, 2-naphthoyl etc.),
(24) carboxyl,
(25) C₁₋₆ alkoxy-carbonyl (e.g., methoxycarbonyl, ethoxycarbonyl, propoxycarbonyl, tert-butoxycarbonyl etc.),
(26) C₆₋₁₄ aryloxy-carbonyl (e.g., phenoxycarbonyl etc.),
(27) carbamoyl,
(28) thiocarbamoyl,
(29) mono-C₁₋₆ alkyl-carbamoyl (e.g., methylcarbamoyl, ethylcarbamoyl etc.),
(30) di-C₁₋₆ alkyl-carbamoyl (e.g., dimethylcarbamoyl, diethylcarbamoyl, ethylmethylcarbamoyl etc.),
(31) C₆₋₁₄ aryl-carbamoyl (e.g., phenylcarbamoyl, 1-naphthylcarbamoyl, 2-naphthylcarbamoyl etc.),
(32) C₁₋₆ alkylsulfonyl (e.g., methylsulfonyl, ethylsulfonyl etc.),
(33) C₆₋₁₄ arylsulfonyl (e.g., phenylsulfonyl, 1-naphthylsulfonyl, 2-naphthylsulfonyl etc.),
(34) C₁₋₆ alkylsulfinyl (e.g., methylsulfinyl, ethylsulfinyl etc.),
(35) C₆₋₁₄ arylsulfinyl (e.g., phenylsulfinyl, 1-naphthylsulfinyl, 2-naphthylsulfinyl etc.),
(36) formylamino,
(37) C₁₋₆ alkyl-carbonylamino (e.g., acetylamino etc.),
(38) C₆₋₁₄ aryl-carbonylamino (e.g., benzoylamino, naphthoylamino etc.),
(39) C₁₋₆ alkoxy-carbonylamino (e.g., methoxycarbonylamino, ethoxycarbonylamino, propoxycarbonylamino, butoxycarbonylamino etc.),
(40) C₁₋₆ alkylsulfonylamino (e.g., methylsulfonylamino, ethylsulfonylamino etc.),
(41) C₆₋₁₄ arylsulfonylamino (e.g., phenylsulfonylamino, 2-naphthylsulfonylamino, 1-naphthylsulfonylamino etc.),
(42) C₁₋₆ alkyl-carbonyloxy (e.g., acetoxy, propionyloxy etc.),
(43) C₆₋₁₄ aryl-carbonyloxy (e.g., benzoyloxy, naphthylcarbonyloxy etc.),
(44) C₁₋₆ alkoxy-carbonyloxy (e.g., methoxycarbonyloxy, ethoxycarbonyloxy, propoxycarbonyloxy, butoxycarbonyloxy etc.),
(45) mono-C₁₋₆ alkyl-carbamoyloxy (e.g., methylcarbamoyloxy, ethylcarbamoyloxy etc.),
(46) di-C₁₋₆ alkyl-carbamoyloxy (e.g., dimethylcarbamoyloxy, diethylcarbamoyloxy etc.),
(47) C₆₋₁₄ aryl-carbamoyloxy (e.g., phenylcarbamoyloxy, naphthylcarbamoyloxy etc.),
(48) 5- to 7-membered saturated cyclic amino optionally containing one nitrogen atom and, besides carbon atom, one or two kinds of 1 to 4 hetero atoms selected from nitrogen atom, sulfur atom and oxygen atom (e.g., pyrrolidin-1-yl, piperidino, piperazin-1-yl, morpholino, thiomorpholino, hexahydroazepin-1-yl etc.),
(49) a - to 10-membered aromatic heterocyclic group containing, besides carbon atom, one or two kinds of 1 to 4 hetero atoms selected from nitrogen atom, sulfur atom and oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.),
(50) C₁₋₃ alkylenedioxy (e.g., methylenedioxy, ethylenedioxy etc.),
(51) C₃₋₇ cycloalkyl (e.g., cyclopropyl, cyclobutyl, cyclopentyl, cyclohexyl, cycloheptyl etc.)
are optional substituents.

As R1, i) a hydrogen atom, ii) a C₁₋₆ alkyl group optionally substituted by 1 to 3 substituents selected from halogen atom, hydroxy, mono-C₁₋₆ alkylamino, di-C₁₋₆ alkylamino, C₁₋₆ alkoxy, C₇₋₁₆ aralkyloxy, C₃₋₇ cycloalkyl and 5- or 6-membered heterocyclic group, iii) a C₂₋₆ alkenyl group or iv) a C₇₋₁₆ aralkyl group optionally substituted by 1 to 3 C₁₋₆ alkoxy are preferable.

As R1, particularly, i) a hydrogen atom, ii) a C₁₋₆ alkyl group optionally substituted by 1 to 3 substituents selected from hydroxy, mono-C₁₋₆ alkylamino, di-C₁₋₆ alkylamino, C₁₋₆ alkoxy and C₃₋₇ cycloalkyl, are widely used, of which i) hydrogen atom and ii) a C₁₋₆ alkyl group optionally substituted by 1 to 3 substituents selected from C₁₋₆ alkoxy and C₃₋₇ cycloalkyl, particularly, a C₁₋₄ alkyl group (e.g., methyl, ethyl, propyl etc.), are preferable.

R2 is a C₁₋₄ alkyl group (e.g., methyl, ethyl, propyl etc.).

R3 is a C₁₋₄ alkyl group (e.g., methyl, ethyl, propyl etc.).

R4 and R5 are the same or different and each is a hydrogen atom or a C₁₋₆ alkyl group.

X is a bond, O or 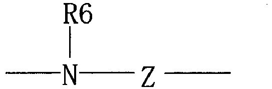 wherein R6 is a hydrogen atom or a C₁₋₆ alkyl group, and Z is a bond or -CO-. Of these, a bond, O or NH is preferable.

In the aforementioned formula (I), m is 0 or 1, and particularly, m is preferably 1.

As A, i) C₆₋₁₀ aryl group (e.g., phenyl, naphthyl etc.) optionally substituted by 1 to 4 substituents selected from C₁₋₆ alkyl optionally substituted by 1 to 5 halogens, C₁₋₆ alkoxy, cyano and halogen atom, ii) C₇₋₁₆ aralkyl group optionally substituted by 1 to 4 substituents selected from C₁₋₆ alkyl optionally substituted by 1 to 5 halogens, C₁₋₆ alkoxy, cyano and halogen atom or iii) a group represented by the formula: 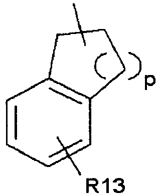 wherein p is 1 or 2, R13 is as defined above, is widely used. Of these, a phenyl group is preferable.

R13 is i) a hydrogen atom or ii) a C₁₋₆ alkyl group optionally substituted by 1 to 4 substituents selected from hydroxy, cyano and C₁₋₆ alkoxy.

As A, a 5- or 6-membered heterocyclic group (e.g., thienyl, furyl, pyridyl etc.) optionally having 1 to 4 substituents selected from cyano, halogen atom (e.g., chlorine, fluorine etc.), C₁₋₆ alkyl (e.g., methyl, ethyl etc.), C₁₋₆ alkoxy (e.g., methoxy, ethoxy etc.), C₇₋₁₂ aralkyloxy-carbonyl (e.g., benzyloxycarbonyl etc.), are preferable.

As A, i) a C₆₋₁₀ aryl group (e.g., phenyl group etc.) optionally substituted by 1 to 4 substituents selected from C₁₋₆ alkyl, C₁₋₆ alkoxy, cyano and halogen atom, ii) a group represented by 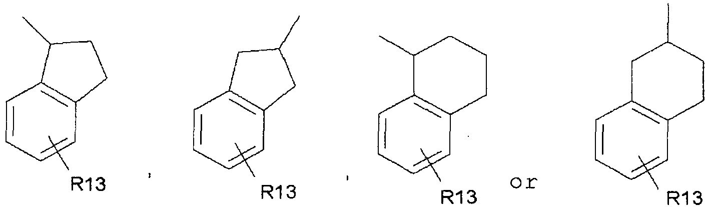 wherein R13 is i) a hydrogen atom or ii) a C₁₋₆ alkyl group optionally substituted by 1 to 4 substituents selected from hydroxy, cyano and C₁₋₆ alkoxy, iii) 5- or 6-membered heterocyclic group (e.g., thienyl group, furyl group, pyridyl group etc.) optionally substituted by 1 to 4 substituents selected from C₁₋₆ alkyl, C₁₋₆ alkoxy, cyano and halogen atom, is widely used. Particularly, phenyl group optionally substituted by 1 to 4 substituents selected from C₁₋₆ alkyl, C₁₋₆ alkoxy, cyano and halogen atom, and a thienyl group optionally substituted by 1 to 4 substituents selected from C₁₋₆ alkyl, C₁₋₆ alkoxy, cyano and halogen atom are preferable.

As compound (I), for example,
N-(2,3-dihydro-1H-inden-1-yl)-2,3-dimethyl-1H-pyrrolo[2,3-c]pyridine-7-amine,
N-(4-fluoro-2-methylbenzyl)-2,3-dimethyl-1-propyl-1H-pyrrolo[2,3-c]pyridine-7-amine,
N-[7-(2,3-dimethyl-1H-pyrrolo[2,3-c]pyridyl)]benzamide,
N-(2,6-dimethylbenzyl)-2,3-dimethyl-1H-pyrrolo[2,3-c]pyridine-7-amine, N-benzyl-2,3-dimethyl-1H-pyrrolo[2,3-c]pyridine-7-amine are preferable.

As a salt of compound (I), for example, metal salts, ammonium salts, salts with organic bases, salts with inorganic acids, salts with organic acids, salts with basic or acidic amino acids can be mentioned. As preferable examples of the metal salt, alkali metal salts such as sodium salt, potassium salt; alkaline earth metal salts such as calcium salt, magnesium salt, barium salt; aluminum salt can be mentioned. As preferable examples of the salts with organic bases, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine can be mentioned. As preferable examples of the salts with inorganic acids, salts with hydrochloric acid, hydrobromic acid, nitric acid, sulfuric acid, phosphoric acid can be mentioned. As preferable examples of the salts with organic acids, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid can be mentioned. As preferable examples of the salts with basic amino acids, salts with arginine, lysine, ornithine can be mentioned. As preferable examples of the salts with acidic amino acids, salts with aspartic acid, glutamic acid can be mentioned.

Of those, pharmaceutically acceptable salts are preferable. For example, when a compound has an acidic functional group therein, inorganic salts such as alkali metal salts (e.g., sodium salt, potassium salt), alkaline earth metal salts (e.g., calcium salt, magnesium salt, barium salt), ammonium salt can be mentioned. When a compound has a basic functional group therein, salts with inorganic acids such as hydrochloric acid, hydrobromic acid, nitric acid, sulfuric acid, phosphoric acid, and salts with organic acids such as acetic acid, phthalic acid, fumaric acid, oxalic acid, tartaric acid, maleic acid, citric acid, succinic acid, methanesulfonic acid, p-toluenesulfonic acid can be mentioned.

The production method of compound (I) of the present invention is described in more detail by referring to the production methods of the following compound (Ia), compound (Ib), compound (Ic) and compound (Id) .

Compound (Ia), compound (Ib), compound (Ic) and compound (Id) of the present invention can be produced, for example, by the method shown by the following reaction scheme or a method according thereto.

The compound of the formula includes one in the form of a salt, and as such salt, for example, a salt similar to the salt of compound (I) are used.

While the compound obtained in each step can be used directly as a reaction mixture or a crude product and used for the next reaction, it can also be isolated from a reaction mixture according to a conventional method, and easily purified by a separation means such as recrystallization, distillation, chromatography .

A simplified reaction scheme is shown in the following, wherein R2, R3, R4, R5, m, A and Y are as defined above.

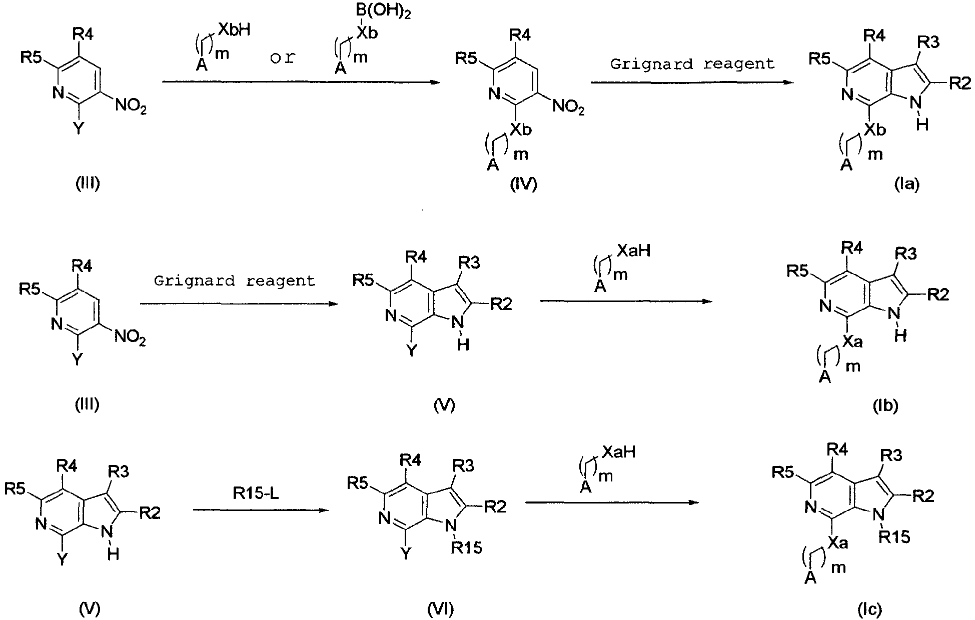

Compound (III) wherein Y is a leaving group such as a halogen atom (e.g., fluorine, chlorine, bromine, iodine), a phenoxy group , and other symbols are as defined above, can be produced by a method known *per se,* for example, the method described in Chemical And Pharmaceutical Bulletin (Chem. Pharm. Bull.), vol. 36, page 2244 (1988), Journal of Heterocyclic Chemistry (J. Heterocyclic. Chem.), vol. 33, page 287 (1996), or a method analogous thereto.

Compound (IV) wherein Xb is O, S or NR6 (wherein R6 is as defined above) can be produced by reacting compound (III) with a compound represented by the formula: 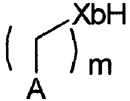 wherein Xb is O, S or NR6 (wherein R6 is as defined above), and other symbols are as defined above.

The latter compound is used in an amount of about 1.0 - about 100 mol, preferably about 1.0 - about 10.0 mol, per 1 mol of compound (III).

This reaction is advantageously carried out without solvent or using a solvent inert to the reaction. While such solvent is not particularly limited as long as the reaction proceeds, solvents such as alcohols (e.g., methanol, ethanol, propanol ), hydrocarbons (e.g., benzene, toluene, cyclohexane, hexane ), amides (e.g., N,N-dimethylformamide, N,N-dimethylacetamide ), a mixed solvent thereof are preferable.

For this reaction, the use of a base is sometimes effective. As the base, for example, inorganic base such as sodium hydroxide, potassium hydroxide , basic salts such as sodium carbonate, potassium carbonate, cesium carbonate, sodium hydrogen carbonate , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine , can be mentioned. The amount of such base to be used is about 0.1 - about 10.0 mol, preferably about 0.1 - about 5.0 mol, per 1 mol of compound (III).

While the reaction time varies depending on the reagent and solvent to be used, it is generally about 30 min - about 24 hr, preferably about 30 min - about 8 hr.

The reaction temperature is generally about 0°C to about 250°C, preferably about 25°C to about 100°C.

Compound (IV) wherein Xb is a bond or CH₂ can be produced from compound (III) wherein Y is a halogen atom such as chlorine atom, bromine atom or iodine atom, , and other symbols are as defined above, and a boronic acid derivative represented by the formula: 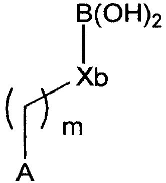 wherein Xb is a bond or CH₂, and other symbols are as defined above, according to a method known *per se,* for example, the method described in Tetrahedron, vol. 58, page 1465 (2002), or a method analogous thereto.

Then, compound (IV) wherein Xb is a bond, O, S, CH₂ or NR6 (wherein R6 is as defined above), and other symbols are as defined above, is reacted with a vinyl Grignard reagent represented by the formula: 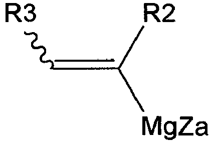 wherein Za is a chlorine atom or a bromine atom, and other symbols are as defined above, such as isopropenylmagnesium halide, 1-methyl-1-propenylmagnesium halide , whereby compound (Ia) can be produced.

In this reaction, the Grignard reagent is used in an amount of about 1.0 - about 5.0 mol, preferably about 3.0 - about 4.0 mol, per 1 mol of compound (IV).

This reaction is advantageously carried out using a solvent inert to the reaction. While such solvent is not particularly limited as long as the reaction proceeds, solvents such as hydrocarbons such as benzene, toluene, cyclohexane, hexane , tetrahydrofuran, a mixed solvent thereof are preferable.

While the reaction time varies depending on the reagent and solvent to be used, it is generally about 30 min - about 24 hr, preferably about 30 min - about 8 hr.

The reaction temperature is generally about -78°C to about 50°C, preferably about -78°C to about 0°C.

Compound (III) wherein Y is a halogen atom such as chlorine atom, bromine atom or iodine atom , and other symbols are as defined above, can be converted to compound (V) by a method similar to the aforementioned method for producing compound (Ia) from compound (IV).

Then, compound (V) is reacted with a compound represented by the formula: 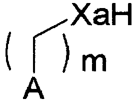 wherein Xa is O, S or 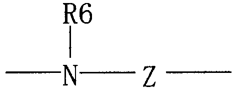 wherein R6 and Z are as defined in compound (I) above, and other symbols are as defined above, whereby compound (Ib) can be produced.

The latter compound is used in an amount of about 1 - about 50 mol, preferably about 1 - about 10 mol, per 1 mol of compound (V).

For this reaction, the coexistence of a base is sometimes effective. As the base, for example, inorganic base such as sodium hydroxide, potassium hydroxide, sodium hydride , basic salts such as sodium carbonate, potassium carbonate, cesium carbonate, sodium hydrogen carbonate , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine, metal alkoxides such as sodium methoxide, sodium ethoxide, sodium tert-butoxide, potassium tert-butoxide , , can be mentioned. The amount of such base is about 1.0 - about 10.0 mol, preferably about 1.5 - about 3.0 mol, per 1 mol of compound (V).

Where necessary, for example, a catalyst such as copper, copper salt may be used, and a catalyst such as palladium, nickel and a ligand (e.g., phosphine, pyridines ) may be used according to the method described in Chemistry Letters, page 927 (1983).

As the "copper catalyst", copper, halogenated copper (CuI, CuBr, CuCl ), copper oxide (CuO) can be mentioned. The amount of these copper catalysts to be used is about 0.1 to about 10.0 mol, preferably about 0.5 to about 2.0 mol, per 1 mol of compound (V).

As the "ligand", phosphine is preferable, and trialkylphosphine, triarylphosphine (2,2'-bis(diphenylphosphino)-1,1'-binaphthyl, 1,1'-bis(diphenylphosphino)ferrocene, 9,9-dimethyl-4,5-bis(diphenylphosphino)xanthine etc.), trialkoxyphosphine can be mentioned. The amount of these ligands to be used is about 0.001 to about 10.0 mol, preferably about 0.01 to about 1.0 mol, per 1 mol of compound (V).

As the "palladium catalyst", palladium acetate, palladium chloride, tetrakis(triphenylphosphine) palladium, tris(dibenzylideneacetone) dipalladium can be mentioned. The amount of these palladium catalysts to be used is about 0.001 to about 5.0 mol, preferably about 0.01 to about 0.5 mol, per 1 mol of compound (V).

This reaction sometimes proceeds advantageously using a solvent inert to the reaction. While such solvent is not particularly limited as long as the reaction proceeds, for example, solvents such as hydrocarbons (e.g., benzene, toluene ), amides (e.g., N,N-dimethylformamide, N,N-dimethylacetamide ), halogenated hydrocarbons (e.g., dichloromethane, chloroform, carbon tetrachloride, 1,2-dichloroethane ) or tetrahydrofuran or a mixed solvent thereof are preferable.

While the reaction time varies depending on the reagent and solvent to be used, it is generally about 5 min - about 48 hr, preferably about 5 min - about 16 hr.

The reaction temperature is generally about 0°C to about 250°C, preferably about 25°C to about 200°C.

Compound (VI) wherein Y is a leaving group such as a halogen atom (e.g., fluorine, chlorine, bromine, iodine), a phenoxy group , R15 is a hydrocarbon group as as defined herein for R1 and other symbols are as defined above, can be produced by reacting compound (V) wherein Y is a leaving group such as a halogen atom (e.g., fluorine, chlorine, bromine, iodine), phenoxy group , and other symbols are as defined above, with a compound represented by the formula: R15-L wherein L is a leaving group such as a halogen atom, alkylsulfonyl, alkylsulfonyloxy or arylsulfonyloxy , and R15 is as defined above, in the presence of a base.

As the halogen atom for L, fluorine atom, chlorine atom, bromine atom and iodine atom can be mentioned.

As alkylsulfonyl for L, for example, C₁₋₆ alkylsulfonyl such as methylsulfonyl, ethylsulfonyl, propylsulfonyl can be mentioned.

As alkylsulfonyloxy for L, for example, C₁₋₆ alkylsulfonyloxy such as methylsulfonyloxy, ethylsulfonyloxy, propylsulfonyloxy can be mentioned.

As arylsulfonyloxy for L, for example, C₆₋₁₀ arylsulfonyloxy such as phenylsulfonyloxy can be mentioned.

R15-L is used in an amount of about 1.0 - about 5.0 mol, preferably about 1.5 - about 3.0 mol, per 1 mol of compound (V).

As the base, for example, inorganic bases such as sodium hydroxide, potassium hydroxide, sodium hydride , basic salts such as sodium carbonate, potassium carbonate, cesium carbonate, sodium hydrogen carbonate , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine , can be mentioned.

The amount of these bases to be used is about 1.0 - about 10.0 mol, preferably about 1.5 - about 3.0 mol, per 1 mol of compound (V).

This reaction proceeds advantageously using a solvent inert to the reaction. While such solvent is not particularly limited as long as the reaction proceeds, for example, solvents such as hydrocarbons (e.g., benzene, toluene ), amides (e.g., N,N-dimethylformamide, N,N-dimethylacetamide ), halogenated hydrocarbons (e.g., dichloromethane, chloroform, carbon tetrachloride, 1,2-dichloroethane ) or tetrahydrofuran or a mixed solvent thereof are preferable.

While the reaction time varies depending on the reagent and solvent to be used, it is generally about 30 min - about 24 hr, preferably about 30 min - about 8 hr.

The reaction temperature is generally about 0°C to about 150°C, preferably about 0°C to about 100°C.

Compound (VI) wherein Y is a leaving group such as a halogen atom (e.g., fluorine, chlorine, bromine, iodine), a phenoxy group , and other symbols are as defined above, can be converted to compound (Ic) by a method similar to the aforementioned method for producing compound (Ib) from compound (V).

A compound obtained by the reaction step to obtain the aforementioned compound (Ib) and compound (Ic), which is represented by the formula: 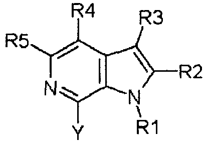 wherein Y is a leaving group such as a halogen atom (e.g., fluorine, chlorine, bromine, iodine), a phenoxy group , R1, R2, R3, R4 and R5 are as defined above, or a salt thereof, more specifically, a compound represented by the formula (V) : 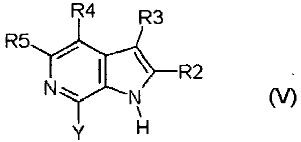 wherein each symbol is as defined above, or a salt thereof, or the formula (VI) : 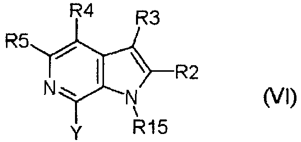 wherein each symbol is as defined above, or a salt thereof, is a novel compound, and can be used as a starting material of the compound of the present invention. As preferable compounds, 7-chloro-2,3-dimethylpyrrolo[2,3-c]pyridine and a salt thereof can be mentioned.

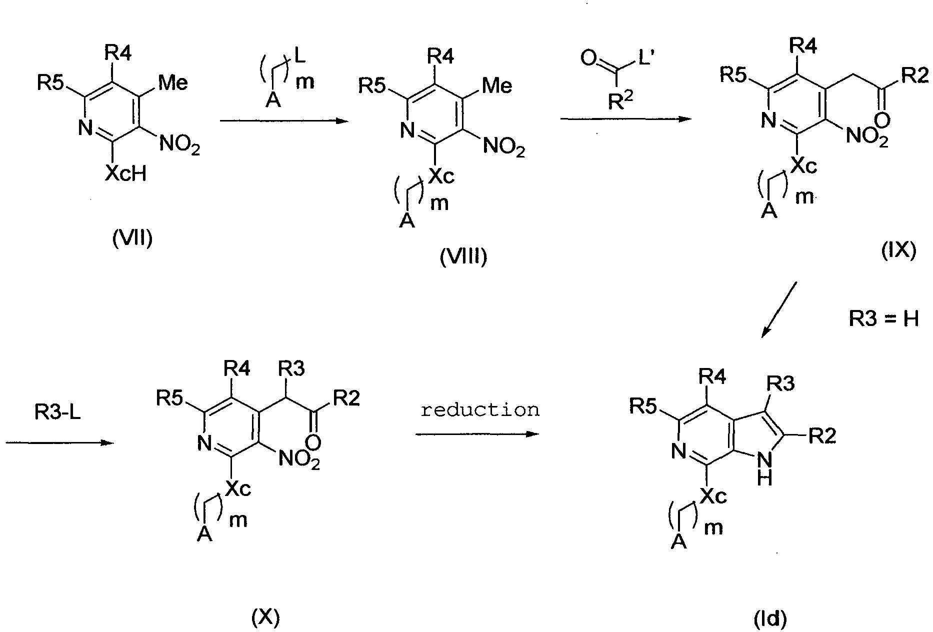

Compound (VII) wherein Xc is O, S or NR6' wherein R6' is as defined for R6 above, or N-protecting group, and other symbols are as defined above, can be produced according to a method known per se, for example, the method described in Heterocycles, vol. 57, page 2335 (2002), or a method analogous thereto.

As the N-protecting group for R6', tert-butoxycarbonyl group [BOC group], benzyloxycarbonyl group (Cbz group) can be mentioned.

Compound (VIII) wherein each symbol is as defined above can be produced by reacting compound (VII) with a compound represented by the formula: 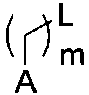 wherein L is a leaving group as mentioned above, such as a halogen atom, alkylsulfonyl, alkylsulfonyloxy or arylsulfonyloxy , and other symbols are as defined above, in the presence of a base.

The latter compound is preferably used in an amount of about 1.0 - about 10 mol, preferably about 1.0 - about 2.0 mol, per 1 mol of compound (VII).

This reaction is advantageously carried out without solvent or using a solvent inert to the reaction. While such solvent is not particularly limited as long as the reaction proceeds, solvents such as ethers (e.g., diethyl ether, tetrahydrofuran ), alcohols (e.g., methanol, ethanol, propanol ), hydrocarbons (e.g., benzene, toluene, cyclohexane, hexane ), amides (e.g., N,N-dimethylformamide, N,N-dimethylacetamide ) , a mixed solvent thereof are preferable.

As the base, for example, inorganic bases such as sodium hydroxide, potassium hydroxide, sodium hydride , basic salts such as sodium carbonate, potassium carbonate, cesium carbonate, sodium hydrogen carbonate , metal bases such as butyllithium, potassium ethoxide, potassium tert-butoxide, sodium methoxide, sodium ethoxide , metal amides such as lithium diisopropylamide, lithium hexamethyldisilazide , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine , can be mentioned. The amount of these bases to be used is about 0.1 - about 10.0 mol, preferably about 0.1 - about 5.0 mol, per 1 mol of compound (VII).

While the reaction time varies depending on the reagent and solvent to be used, it is generally about 30 min - about 24 hr, preferably about 30 min - about 8 hr.

The reaction temperature is generally about -78°C to about 100°C, preferably about 0°C to about 50°C.

Compound (IX) wherein symbol is as defined above can be produced by reacting compound (VIII) with the formula: 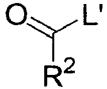 wherein L' is a leaving group such as a halogen atom, alkoxy, 2-methyl-1-aziridinyl, N,O-dimethylhydroxyamino, morpholino , and other symbols are as defined above, in the presence of a base.

The latter compound is preferably used in an amount of about 1.0 - about 10 mol, preferably about 1.0 - about 2.0 mol, per 1 mol of compound (VIII).

This reaction is advantageously carried out without solvent or using a solvent inert to the reaction. While such solvent is not particularly limited as long as the reaction proceeds, solvents such as ethers (e.g., diethyl ether, tetrahydrofuran ), hydrocarbons (e.g., benzene, toluene, cyclohexane, hexane ), amides (e.g., N,N-dimethylformamide, N,N-dimethylacetamide ) , a mixed solvent thereof are preferable.

As the base, for example, inorganic bases such as sodium hydroxide, potassium hydroxide, sodium hydride , metal bases such as butyllithium, potassium ethoxide, potassium tert-butoxide , metal amides such as lithium diisopropylamide, lithium hexamethyldisilazide , basic salts such as sodium carbonate, potassium carbonate, cesium carbonate, sodium hydrogen carbonate , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine , can be mentioned. The amount of these bases to be used is about 0.1 - about 10.0 mol, preferably about 0.1 - about 5.0 mol, per 1 mol of compound (VIII).

While the reaction time varies depending on the reagent and solvent to be used, it is generally about 30 min - about 24 hr, preferably about 30 min - about 8 hr.

The reaction temperature is generally about -78°C to about 100°C, preferably about 0°C to about 50°C.

Compound (X) wherein the symbol is as defined above, can be produced by reacting compound (IX) with a compound represented by the formula:

**R3-L**

wherein R3 is as defined above, L is a leaving group as mentioned above, such as a halogen atom, alkylcarboxy, alkylsulfonyl, alkylsulfonyloxy or arylsulfonyloxy .

The latter compound is preferably used in an amount of about 1.0 - about 10 mol, preferably about 1.0 - about 2.0 mol, per 1 mol of compound (IX).

This reaction is advantageously carried out without solvent or using a solvent inert to the reaction. While such solvent is not particularly limited as long as the reaction proceeds, solvents such as ethers (e.g., diethyl ether, tetrahydrofuran ), hydrocarbons (e.g., benzene, toluene, cyclohexane, hexane ), amides (e.g., N,N-dimethylformamide, N,N-dimethylacetamide ) , a mixed solvent thereof are preferable.

In this reaction, use of a base is sometimes effective. As the base, for example, inorganic bases such as sodium hydroxide, potassium hydroxide, sodium hydride , metal bases such as butyllithium, potassium ethoxide, potassium t-butoxide , metal amides such as lithium diisopropylamide, lithium hexamethyldisilazide , basic salts such as sodium carbonate, potassium carbonate, cesium carbonate, sodium hydrogen carbonate , aromatic amines such as pyridine, lutidine , tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine , can be mentioned. The amount of these bases to be used is about 0.1 - about 10.0 mol, preferably about 0.1 - about 5.0 mol, per 1 mol of compound (IX).

While the reaction time varies depending on the reagent and solvent to be used, it is generally about 30 min - about 24 hr, preferably about 30 min - about 8 hr.

The reaction temperature is generally about -78°C to about 100°C , preferably about 0°C to about 50°C.

Compound (Id) can be produced by reducing compound (IX) or (X). As the reduction method, a method known *per se,* for example, the method described in SHINJIKKEN KAGAKU KOUZA 15, "Oxidation and Reduction II" (edited by The Chemical Society of Japan) Maruzen and a method analogous thereto can be mentioned.

Compound (Id) wherein Xc is NR6' (R6' is an N-protecting group) can be led to the compound of the present invention by eliminating the protecting group, for example, by the method described in "Protective Groups in Organic Synthesis, 3rd Ed." Theodora W. Greene, Peter G. M. Wuts, page 494 - page 653, Wiley-Interscience (1999) .

In each of the aforementioned reactions, when the starting compound has an amino group, a carboxyl group or a hydroxyl group as a substituent, these groups may be protected by a protecting group generally used in peptide chemistry . In this case, the object compound can be obtained by eliminating the protecting group as necessary after the reaction. These protecting groups may be introduced or eliminated according to a method known *per se,* for example, the method described in "Protective Groups in Organic Synthesis, 3rd Ed." Theodora W. Greene, Peter G. M. Wuts, Wiley-Interscience (1999).

Compound (I) can also be produced by combining the above-mentioned reaction with, when desired, any one of or two or more of known hydrolysis reaction, deprotection reaction, acylation reaction, alkylation reaction, oxidation reaction, cyclization reaction, carbon chain extension reaction and substituent exchange reaction.

Compound (I) can be isolated and purified by a means known *per se,* such as phase transfer, concentration, solvent extraction, fractionation, liquid conversion, crystallization, recrystallization, chromatography.

When compound (I) is obtained as a free compound, it can be converted to a desired salt by a method known *per se* or a method analogous thereto; conversely, when compound (I) is obtained as a salt, it can be converted to a free form or other desired salt by a method known *per se* or a method analogous thereto.

When compound (I) has isomers such as optical isomer, stereoisomer, positional isomer, rotational isomer , and any isomers and mixtures are encompassed in compound (I). For example, when compound (I) has an optical isomer, an optical isomer resolved from a racemate is also encompassed in compound (I). These isomers can be obtained as independent products by a synthesis means or a separation means (concentration, solvent extraction, column chromatography, recrystallization) known *per se.*

Compound (I) may be a crystal, and both a single crystal and crystal mixtures are encompassed in compound (I). Crystals can be produced by crystallization according to crystallization methods known *per se.*

Compound (I) may be a solvate (e.g., hydrate etc.) or a non-solvate, both of which are encompassed in compound (I) .

A compound labeled with an isotope (e.g., ³H, ¹⁴C, ³⁵S, ¹²⁵I) is also encompassed in compound (I).

Compound (I) of the present invention (hereinafter sometimes to be abbreviated as the compound of the present invention) has a proton pump inhibitory action, and effectively suppresses gastric acid secretion. Since the compound shows low toxicity (e.g., acute toxicity, chronic toxicity, genetic toxicity, reproductive toxicity, cardiotoxicity, drug interaction, carcinogenicity etc.) and high water-solubility, and is also superior in the stability, *in vivo* kinetics (absorption, distribution, metabolism, excretion etc.) and efficacy expression, it is useful as a pharmaceutical agent.

Compound (I) or a salt thereof of the present invention is useful for the treatment or prophylaxis of peptic ulcer (e.g., gastric ulcer, postoperative stress-induced gastric ulcer, duodenal ulcer, anastomotic ulcer, non-steroidal anti-inflammatory drug-induced ulcer etc.); gastritis; reflux esophagitis; non-erosive esophageal reflux disease (Symptomatic Gastroesophageal Reflux Disease (Symptomatic GERD)); NUD (Non Ulcer Dyspepsia); gastric cancer (including gastric cancer associated with promotion of interleukin-1β production by the gene polymorphism of interleukin-1); gastric MALT lymphoma; Zollinger-Ellison syndrome; hyperacidity (e.g., hyperacidity and ulcer due to postoperative stress); upper gastrointestinal bleeding due to peptic ulcer, acute stress ulcer, hemorrhagic gastritis or invasive stress (stress caused by major surgery in need of postoperative intensive care and cerebrovascular disorder in need of intensive treatment, head injury, multiple organ failure, extensive burn) , pre-anesthetic administration, eradication of Helicobacter pylori in mammals (e.g., human, swine, sheep, bovine, horse, dog, cat, rabbit, rat, mouse etc.).

The content of compound (I) or a salt thereof of the present invention in the pharmaceutical composition of the present invention is about 0.01 to 100 wt% of the whole composition. While the dose varies depending on the subject of administration, administration route, disease , for example, it is about 0.5 - about 1500 mg/day, preferably about 5 - about 150 mg/day, as the active ingredient for oral administration of an antiulcer agent to an adult (60 kg). Compound (I) or a salt thereof of the present invention may be administered once a day or in 2 or 3 portions a day.

Compound (I), or a salt thereof of the present invention has low toxicity, and can be safely administered orally or parenterally (e.g., local, rectal or intravenous administration, ) as it is or in the form of a pharmaceutical composition containing a pharmacologically acceptable carrier, such as tablet (including sugar-coated tablet, film-coated tablet), powder, granule, capsule (including soft capsule), orally disintegradable tablet, solution, injection, suppository, sustained release agent, adhesive patch , according to a method known *per se.* Particularly, it is preferably administered as an oral preparation such as tablet, granule, capsule.

As the pharmacologically acceptable carriers usable for the production of the pharmaceutical composition of the present invention, various organic and inorganic carrier substances conventionally used as materials for preparations and, for example, excipient, lubricant, binder, disintegrant, water-soluble polymer and basic inorganic salt for solid preparations; solvent, dissolution aids, suspending agent, isotonicity agent, buffer and soothing agent for liquid preparations, can be mentioned. Where necessary, general additives such as preservative, antioxidant, coloring agent, sweetening agent, souring agent, bubbling agent, flavoring can also be used.

As the "excipient", for example, lactose, sucrose, D-mannitol, glucose, corn starch, crystalline cellulose, light anhydrous silicic acid, titanium oxide can be mentioned.

As "the lubricant", for example, magnesium stearate, sucrose fatty acid ester, polyethylene glycol, talc, stearic acid can be mentioned.

As "the binding agent", for example, hydroxypropylcellulose, hydroxypropylmethylcellulose, crystalline cellulose, starch, polyvinyl pyrrolidone, gum Arabic, gelatin, pullulan, low-substituted hydroxypropylcellulose can be mentioned.

As "the disintegrating agent", (1) crospovidone, (2) disintegrants referred to as super disintegrants such as croscarmelose sodium (FMC-Asahi Kasei Corporation), carmellose calcium (Gotoku Yakuhin) , (3) carboxymethyl starch sodium (e.g., manufactured by Matsutani Chemical Industry Co., Ltd.), (4) low substituted hydroxypropylcellulose (e.g., manufactured by Shin-Etsu Chemical Co., Ltd.), (5) cornstarch can be mentioned. The "crospovidone" may be any crosslinked polymer having a chemical name of 1-ethenyl-2-pyrrolidinone homopolymer, also including those referred to as polyvinyl polypyrrolidone (PVPP) and 1-vinyl-2-pyrrolidinone homo- polymer. Specific examples include Kollidon CL (manufactured by BASF), Polyplasdone XL (manufactured by ISP), Polyplasdone XL-10 (manufactured by ISP), Polyplasdone INF-10 (manufactured by ISP) .

As "the water-soluble polymer", for example, ethanol-soluble water-soluble polymer [e.g., cellulose derivatives such as hydroxypropylcellulose (hereinafter sometimes described as HPC) , polyvinylpyrrolidone etc.], ethanol-insoluble water-soluble polymer [e.g., cellulose derivatives such as hydroxypropylmethylcellulose (hereinafter sometimes described as HPMC), methylcellulose, sodium carboxymethylcellulose , sodium polyacrylate, polyvinyl alcohol, sodium alginate, guar gum etc.] can be mentioned.

As "the basic inorganic salt", for example, basic inorganic salts of sodium, potassium, magnesium and/or calcium can be mentioned. Preferred is a basic inorganic salt of magnesium and/or calcium. More preferably, it is a basic inorganic salt of magnesium. As the basic inorganic salt of sodium, for example, sodium carbonate, sodium hydrogen carbonate, disodium hydrogenphosphate can be mentioned. As the basic inorganic salt of potassium, for example, potassium carbonate, potassium hydrogen carbonate can be mentioned. As the basic inorganic salt of magnesium, for example, heavy magnesium carbonate, magnesium carbonate, magnesium oxide, magnesium hydroxide, magnesium aluminometasilicate, magnesium silicate, magnesium aluminate, synthetic hydrotalcite [mg₆Al₂(OH)₁₆·CO₃·4H₂O] and aluminum hydroxide·magnesium, preferably, heavy magnesium carbonate, magnesium carbonate, magnesium oxide, magnesium hydroxide can be mentioned. As the basic inorganic salt of calcium, for example, precipitated calcium carbonate, calcium hydroxide can be mentioned.

As "the solvent", for example, water for injection, alcohol, propylene glycol, macrogol, sesame oil, corn oil, olive oil can be mentioned.

As "the dissolution aids", for example, polyethylene glycol, propylene glycol, D-mannitol, benzyl benzoate, ethanol, trisaminomethane, cholesterol, triethanolamine, sodium carbonate, sodium citrate can be mentioned.

As "the suspending agent", for example, surfactant such as stearyltriethanolamine, sodium lauryl sulfate, lauryl aminopropionate, lecithin, benzalkonium chloride, benzethonium chloride, glycerol monostearate ; hydrophilic polymer, for example, polyvinyl alcohol, polyvinylpyrrolidone, sodium carboxymethylcellulose, methylcellulose, hydroxymethylcellulose, hydroxyethylcellulose, hydroxypropylcellulose , can be mentioned.

As "the isotonizing agents", for example, glucose, D-sorbitol, sodium chloride, glycerin, D-mannitol can be mentioned.

As "the buffers", for example, buffers such as phosphate, acetate, carbonate, citrate can be mentioned.

As "the soothing agents", for example, benzyl alcohol can be mentioned.

As "the preservative", for example, p-oxybenzoates, chlorobutanol, benzyl alcohol, phenethyl alcohol, dehydroacetic acid, sorbic acid can be mentioned.

As "the antioxidant", for example, sulfite, ascorbic acid, α-tocopherol can be mentioned.

As "the coloring agent", for example, food colors such as Food Yellow No. 5, Food Red No. 2, Food Blue No. 2; food lake colors, diiron trioxide can be mentioned.

As "the sweetener", for example, saccharin sodium, dipotassium glycyrrhetinate, aspartame, stevia, thaumatin can be mentioned.

As "the souring agent", for example, citric acid (citric anhydride), tartaric acid, malic acid can be mentioned.

As "the bubbling agent", for example, sodium bicarbonate can be mentioned.

As "the flavor", any of synthetic substances and naturally occurring substances can be used and, for example, lemon, lime, orange, menthol, strawberry can be mentioned.

The compound of the present invention can be formed into a preparation for oral administration according to a method known *per se,* for example, by adding a carrier such as an excipient, a disintegrating agent, a binder, a lubricant, compression-molding the mixture, then if desirable, coating the product by a method known *per se* for the purpose of masking of taste, achieving the enteric property or durability. For producing an enteric-coated preparation, an intermediate layer can also be formed between the enteric-coated layer and the drug-containing layer by a method known *per se* for the purpose of separating the both layers.

Compound (I) or a salt thereof of the present invention can be produced, for example, as an orally disintegrating tablet by a method including coating a core containing crystalline cellulose and lactose with compound (I) or a salt thereof of the present invention and, where necessary, a basic inorganic salt, further coating the same with a water-soluble polymer-containing coating layer to give a composition, coating the obtained composition with a polyethylene glycol-containing enteric coating layer, then coating the same with a triethyl citrate-containing enteric coating layer, further coating the same with a polyethylene glycol-containing enteric coating layer, finally coating the same with mannitol to give fine granules, mixing the obtained fine granules and additive, and molding the mixture.

As the above-mentioned "enteric coating layer", for example, a layer made of a mixture of one or more kinds of aqueous enteric polymer base such as cellulose acetate phthalate (CAP), hydroxypropylmethylcellulose phthalate, hydroxymethylcellulose acetate succinate, methacrylic acid copolymer [e.g., Eudragit, L30D-55 (trade name; manufactured by Rohm), Kollicoat MAE30DP (trade name; manufactured by BASF), Polyquid PA30 (trade name; manufactured by Sanyo Chemical Industries, Ltd.) etc.], carboxymethylethylcellulose, shellac ; sustained-release base such as methacrylic acid copolymer [e.g., Eudragit NE30D (trade name), Eudragit RL30D (trade name), Eudragit RS30D (trade name) etc.] ; water-soluble polymer; plasticizer such as triethyl citrate, polyethylene glycol, acetylated monoglyceride, triacetine, castor oil, can be mentioned.

As the above-mentioned "additives", for example, water-soluble sugar alcohol (e.g., sorbitol, mannitol, maltitol, reduced starch saccharides, xylitol, reduced paratinose, erythritol etc.), crystalline cellulose (e.g., Ceolus KG 801, Avicel PH 101, Avicel PH 102, Avicel PH 301, Avicel PH 302, Avicel RC-591 (crystalline cellulose·carmellose sodium) etc.), low-substituted hydroxypropylcellulose (e.g., LH-22, LH-32, LH-23, LH-33 (Shin-Etsu Chemical Co., Ltd.) and a mixture of these etc.) can be mentioned, and further, binder, acidulant, bubbling agent, sweetening agent, flavoring, lubricant, coloring agent, stabilizer, excipient, disintegrant can also be used.

The compound of the present invention can also be used in combination with other 1 to 3 kinds of active ingredients.

As the "other active ingredients", for example, anti-Helicobacter pylori active substance, imidazole compound, bismuth salt, quinolone compound can be mentioned.

As the "anti-Helicobacter pylori active substance", for example, penicillin antibiotics (e.g., amoxicillin, benzylpenicillin, piperacillin, mecillinam etc.), cephem antibiotics (e.g., cefixime, cefaclor etc.), macrolide antibiotics (e.g., erythromycin, clarithromycin etc.), tetracycline antibiotics (e.g., tetracycline, minocycline, streptomycin etc.), aminoglycoside antibiotics (e.g., gentamicin, amikacin etc.), imipenem can be mentioned. Of these, penicillin antibiotics, macrolide antibiotics are preferable.

As the "imidazole compound", for example, metronidazole, miconazole can be mentioned.

As the "bismuth salt", for example, bismuth acetate, bismuth citrate can be mentioned.

As the "quinolone compound", for example, ofloxacin, ciploxacin can be mentioned.

For eradication of Helicobacter pylori, compound (I) or a salt thereof of the present invention and penicillin antibiotics (e.g., amoxicillin etc.) and erythromycin antibiotics (e.g., clarithromycin etc.) are preferably used. When the compound of the present invention is used for the eradication of Helicobacter pylori, the compound of the present invention itself has a selective antibacterial activity against Helicobacter pylori. However, when the compound is used in combination with other active ingredients, the antibacterial action of other antibiotics can be enhanced in addition to the antibacterial activity of the compound of the present invention, by the gastric pH-regulating action , thus providing an auxiliary action of the eradication effect based on the action of the antibiotics to be used in combination.

The "other active ingredients" and the compound (I) or a salt thereof of the present invention may be mixed according to a method known *per se* and used in combination as a single preparation of one pharmaceutical composition (e.g., tablet, powder, granule, capsule (including soft capsule), liquid, injection, suppository, sustained-release preparation etc.). Alternatively, they may be formed as separate preparations, and may be administered simultaneously or in a staggered manner to the same administration subject.

### Examples

The present invention is explained in more detail in the following by referring to Reference Examples, Examples, and Test Examples, which are not to be construed as limitative. It will be appreciated that the relevant Example compounds below are those falling within the scope of the claims. Those Example compounds not falling within the scope of the claims are provided for comparative purposes.

The "room temperature" in the following Reference Examples and Examples means normally about 10°C to about 35°C, which are not to be construed as limitative. The mixing ratio of liquid shows a volume ratio. The "%" indicates percentage by weight unless otherwise indicated. The yield shows mol/mol%. ¹H-NMR spectrum was measured using Varian Gemini-200 (200 MHz) and Mercury-300 (300 MHz) type spectrometers and tetramethylsilane as the internal standard. The liquid chromatograph mass analysis was performed using micromas ZQ2000 manufactured by Waters.
s: singlet, d: doublet, t: triplet, q: quartet, m: multiplet, br: broad, J: coupling constant, Hz: Hertz.

### Reference Example 1

### 7-chloro-2-methyl-1H-pyrrolo[2,3-c]pyridine

A solution (300 mL) of 2-chloro-3-nitropyridine (11.91 g) in tetrahydrofuran was cooled to -78°C, and a 0.5M isopropenylmagnesium bromide-tetrahydrofuran solution (300 mL) was added. The reaction mixture was stirred at -20°C for 18 hr, returned to room temperature, and concentrated under reduced pressure to a liquid amount of about 120 mL. A 20% aqueous ammonium chloride solution (300 mL) was added, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=6:1→1:1), and crystallized from diisopropyl ether to give the title compound as a pale-yellow solid (yield 3.50 g, yield 28%).
¹H-NMR (CDCl₃) δ: 2.52 (3H, s), 6.30 (1H, s), 7.34 (1H, d, J = 5.6 Hz), 7.98 (1H, d, J = 5.6 Hz), 8.46 (1H, br).

### Reference Example 2

### 7-chloro-2,3-dimethyl-1H-pyrrolo[2,3-c]pyridine

Using 2-chloro-3-nitropyridine (3.50 g) and 0.5M 1-methyl-1-propenylmagnesium bromide-tetrahydrofuran solution (100 mL), reactions similar to those of Reference Example 1 were carried out to give the title compound (580 mg).
¹H-NMR (CDCl₃) δ: 2.21 (3H, s), 2.44 (3H, s), 7.30 (1H, d, J = 5.7 Hz), 7.98 (1H, d, J = 5.7 Hz), 8.20 (1H, br).

### Reference Example 3

### 7-chloro-1-ethyl-2-methyl-1H-pyrrolo[2,3-c]pyridine

Sodium hydride (60% in oil, 115 mg) was suspended in N,N-dimethylformamide (5 mL), and a solution (5 mL) of 7-chloro-2-methyl-1H-pyrrolo[2,3-c]pyridine (333 mg) obtained in Reference Example 1 in N,N-dimethylformamide was added dropwise at 0°C. After stirring at the same temperature for 10 min, iodoethane (374 mg) was added dropwise at 0°C and the mixture was stirred at room temperature for 1 hr. N,N-dimethylformamide was evaporated under reduced pressure, 6% aqueous sodium hydrogen carbonate solution was added and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→3:1) to give the title compound as yellow crystals (yield 357 mg, yield 92%). ¹H-NMR (CDCl₃) δ: 1.39 (3H, t, J = 7.2 Hz), 2.45 (3H, s), 4.52 (2H, q, J = 7.2 Hz), 6.29-6.30 (1H, m), 7.32 (1H, d, J = 5.4 Hz), 7.92 (1H, d, J = 5.4 Hz).

### Reference Example 4

### 7-chloro-2-methyl-1-propyl-1H-pyrrolo[2,3-c]pyridine

Using 1-iodopropane (408 mg), methods similar to those of Reference Example 3 were carried out to give the title compound (402 mg) as a colorless oil.
¹H-NMR (CDCl₃) δ: 0.98 (3H, t, J = 7.2 Hz), 1.76-1.87 (2H, m), 2.45 (3H, s), 4.35-4.41 (2H, m), 6.28-6.29 (1H, m), 7.30 (1H, d, J = 5.1 Hz), 7.91 (1H, d, J = 5.1 Hz).

### Reference Example 5

### 1-butyl-7-chloro-2-methyl-1H-pyrrolo[2,3-c]pyridine

Using 1-iodobutane (442 mg), methods similar to those of Reference Example 3 were carried out to give the title compound (382 mg) as a pale-yellow oil.
¹H-NMR (CDCl₃) δ: 0.98 (3H, t, J = 6.9 Hz), 1.37-1.49 (2H, m), 1.70-1.81 (2H, m), 2.45 (3H, s), 4.39-4.48 (2H, m), 6.28 (1H, s), 7.31 (1H, d, J = 5.7 Hz), 7.91 (1H, d, J = 5.7 Hz).

### Reference Example 6

### 7-chloro-1-(cyclohexylmethyl)-2-methyl-1H-pyrrolo[2,3-c]pyridine

Using (bromomethyl)cyclohexane (425 mg), methods similar to those of Reference Example 3 were carried out to give the title compound (358 mg) as a pale-yellow oil. ¹H-NMR (CDCl₃) δ: 0.97-1.25 (5H, m), 1.51-1.80 (5H, m), 1.81-2.00 (1H, m), 2.45 (3H, s), 4.25 (2H, d, J = 7.4 Hz), 6.28-6.29 (1H, m), 7.32 (1H, d, J = 5.4 Hz), 7.92 (1H, d, J = 5.4 Hz).

### Reference Example 7

### 7-chloro-1-(cyclopropylmethyl)-2-methyl-1H-pyrrolo[2,3-c]pyridine

Using (bromomethyl)cyclopropane (324 mg), methods similar to those of Reference Example 3 were carried out to give the title compound (394 mg) as a pale-yellow powder.
¹H-NMR (CDCl₃) δ: 0.36-0.42 (2H, m), 0.50-0.57 (2H, m), 1.23-1.35 (1H, m), 2.47 (3H, s), 4.43 (2H, d, J = 6.6 Hz), 6.31-6.32 (1H, m), 7.32 (1H, d, J = 5.4 Hz), 7.92 (1H, d, J = 5.4 Hz).

### Reference Example 8

### 7-chloro-2-methyl-1-(pyridin-2-ylmethyl)-1H-pyrrolo[2,3-c]pyridine

Using 2-(bromomethyl)pyridine (607 mg), methods similar to those of Reference Example 3 were carried out to give the title compound (476 mg) as a pale-yellow oil. ¹H-NMR (CDCl₃) δ: 2.40 (3H, s), 5.88 (2H, s), 6.42 (1H, s), 6.53 (1H, d, J = 8.1 Hz), 7.13-7.18 (1H, m), 7.38 (1H, d, J = 5.4 Hz), 7.51-7.57 (1H, m), 7.96 (1H, d, J = 5.1 Hz), 8.57 (1H, d, J = 5.1 Hz).

### Reference Example 9

### 7-chloro-1-(4-methoxybenzyl)-2-methyl-1H-pyrrolo[2,3-c]pyridine

Using 4-(chloromethyl)phenyl methyl ether (376 mg), methods similar to those of Reference Example 3 were carried out to give the title compound (406 mg) as a pale-yellow oil.
¹H-NMR (CDCl₃) δ: 2.37 (3H, s), 3.75 (3H, s), 5.71 (2H, s), 6.37 (1H, s), 6.75-6.85 (4H, m), 7.36 (1H, d, J = 5.4 Hz), 7.95 (1H, d, J = 5.4 Hz).

### Reference Example 10

### 1-[2-(benzyloxy)ethyl]-7-chloro-2-methyl-1H-pyrrolo[2,3-c]pyridine

Using 7-chloro-2-methyl-1H-pyrrolo[2,3-c]pyridine (833 mg) obtained in Reference Example 1 and benzyl 2-chloroethyl ether (1.0 g), methods similar to those of Reference Example 3 were carried out to give the title compound (1.10 g) as a pale-yellow oil.
¹H-NMR (CDCl₃) δ: 2.50 (3H, s), 3.84 (2H, t, J = 5.7 Hz), 4.41 (2H, s), 4.69 (2H, t, J = 5.7 Hz), 6.30 (1H, s), 7.13-7.20 (3H, m), 7.24-7.30 (2H, m), 7.33 (1H, d, J = 5.4 Hz), 7.93 (1H, d, J = 5.4 Hz).

### Reference Example 11

### 1-benzyl-7-chloro-2-methyl-1H-pyrrolo[2,3-c]pyridine

Using benzyl bromide (0.342 mL), methods similar to those of Reference Example 3 were carried out to give the title compound (350 mg) as a pale-yellow oil. ¹H-NMR (CDCl₃) δ: 2.38 (3H, s), 5.80 (2H, s), 6.41 (1H, s), 6.85-6.95 (2H, m), 7.20-7.35 (3H, m), 7.38 (1H, d, J = 5.6 Hz), 7.96 (1H, d, J = 5.6 Hz).

### Reference Example 12

### 7-chloro-1,2-dimethyl-1H-pyrrolo[2,3-c]pyridine

Using iodomethane (0.130 mL), methods similar to those of Reference Example 3 were carried out to give the title compound (347 mg) as a yellow solid.
¹H-NMR (CDCl₃) δ: 2.45 (3H, s), 4.06 (3H, s), 6.30 (1H, s), 7.31 (1H, d, J = 5.4 Hz), 7.91 (1H, d, J = 5.4 Hz).

### Reference Example 13

### 7-chloro-1-(2-methoxyethyl)-2-methyl-1H-pyrrolo[2,3-c]pyridine

Using 2-bromoethyl methyl ether (0.197 mL), methods similar to those of Reference Example 3 were carried out to give the title compound (230 mg) as a colorless oil. ¹H-NMR (CDCl₃) δ: 2.50 (3H, s), 3.28 (3H, s), 3.75 (2H, t, J = 5.4 Hz), 4.65 (2H, t, J = 5.4 Hz), 6.30 (1H, s), 7.33 (1H, d, J = 5.4 Hz), 7.94 (1H, d, J = 5.4 Hz).

### Reference Example 14

### 2-chloro-3-nitro-6-(trifluoromethyl)pyridine

The compound was synthesized according to JP-A-63-48268.

### Reference Example 15

### 2,6-dibromo-3-nitropyridine

2,6-Dichloro-3-nitropyridine (5.0 g) was dissolved in 25% hydrogen bromide-acetic acid solution (50 mL), and the mixture was stirred at 80°C for 6 hr. The mixture was returned to room temperature, concentrated under reduced pressure to a liquid amount of about 20 mL, neutralized using a 12N aqueous sodium hydroxide solution at 0°C, and extracted with ethyl acetate. The extract was washed with saturated brine, dried over anhydrous sodium sulfate, and concentrated under reduced pressure. The yellow solid obtained as a residue was washed with a mixed solvent of diisopropyl ether and hexane to give the title compound as yellow crystals (yield 5.6 g, including impurity).
¹H-NMR (CDCl₃) δ: 7.65 (1H, d, J = 8.4 Hz), 8.03 (1H, d, J = 8.4 Hz).

### Reference Example 16

### N,N-dibenzyl-3-nitropyridine-2-amine

To a suspension (30 mL) of 2-chloro-3-nitropyridine (3.2 g) and sodium carbonate (2.2 g) in tetrahydrofuran was added dibenzylamine (7.9 g) at room temperature, and the mixture was heated under reflux overnight. The reaction mixture was returned to room temperature, water was added and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=99:1→9:1) to give the title compound as a yellow oil (yield 5.85 g, yield 92%).
¹H-NMR (CDCl₃) δ: 4.58 (4H, s), 6.74-6.78 (1H, m), 7.12-7.15 (4H, m), 7.22-7.30 (6H, m), 8.08-8.11 (1H, m), 8.35-8.37 (1H, m).

### Reference Example 17

### N,N-dibenzyl-6-chloro-3-nitropyridine-2-amine

Using 2,6-dichloro-3-nitropyridine (7.7 g), methods similar to those of Reference Example 16 were carried out to give the title compound (10.4 g) to give the title compound as yellow crystals.
¹H-NMR (CDCl₃) δ: 4.60 (4H, s), 6.72 (1H, d, J = 8.4 Hz), 7.11-7.16 (4H, m), 7.23-7.35 (6H, m), 8.04 (1H, d, J = 8.4 Hz).

### Reference Example 18

### N,N-dibenzyl-3-nitro-6-(trifluoromethyl)pyridine-2-amine

Using 2-chloro-3-nitro-6-(trifluoromethyl)pyridine (4.0 g) obtained in Reference Example 14, methods similar to those of Reference Example 16 were carried out to give the title compound (6.4 g) as a yellow oil. ¹H-NMR (CDCl₃) δ: 4.65 (4H, s), 7.06 (1H, d, J = 8.1 Hz), 7.14-7.17 (4H, m), 7.25-7.32 (6H, m), 8.15 (1H, d, J = 8.1 Hz).

### Reference Example 19

### N,N-dibenzyl-6-bromo-3-nitropyridine-2-amine

Using 2,6-dibromo-3-nitropyridine (5.6 g) obtained in Reference Example 15, methods similar to those of Reference Example 16 were carried out to give the title compound (7.0 g) as yellow crystals.
¹H-NMR (CDCl₃) δ: 4.59 (4H, s), 6.86 (1H, d, J = 8.1 Hz), 7.13-7.16 (4H, m), 7.26-7.30 (6H, m), 7.90 (1H, d, J = 8.1 Hz).

### Reference Example 20

### 6-(dibenzylamino)-5-nitropyridine-2-carbonitrile

To a solution (5 mL) of N,N-dibenzyl-6-chloro-3-nitropyridine-2-amine (354 mg) obtained in Reference Example 17 in N,N-dimethylformamide were added zinc cyanide (88 mg), tris(dibenzylideneacetone) dipalladium (0) (46 mg) and 1,1'-bis(diphenylphosphino)ferrocene (55 mg), and the mixture was stirred overnight at 120°C. The solvent was evaporated under reduced pressure, water was added and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow oil (yield 268 mg, yield 78%).
¹H-NMR (CDCl₃) δ: 4.63 (4H, s), 7.08-7.15 (5H, m), 7.26-7.31 (6H, m), 8.11 (1H, d, J = 7.8 Hz).

### Reference Example 21

### methyl 6-(dibenzylamino)-5-nitropyridine-2-carboxylate

To a solution (70 mL) of N,N-dibenzyl-6-bromo-3-nitropyridine-2-amine (7.8 g) obtained in Reference Example 19 in N,N-dimethylformamide were added palladium (II) acetate (444 mg), triphenylphosphine (519 mg), triethylamine (11 mL) and methanol (70 mL), and the mixture was stirred under a carbon monoxide atmosphere at 60°C for 20 hr. The reaction mixture was returned to room temperature, filtered through a hyflo super-cel (trade name: manufactured by Celite Co.), and washed with ethyl acetate. The filtrate was concentrated under reduced pressure. Water was added to the residue and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a yellow oil (yield 6.1 g, yield 81%).
¹H-NMR (CDCl₃) δ: 3.97 (3H, s), 4.66 (4H, s), 7.15-7.18 (4H, m), 7.25-7.28 (6H, m), 7.48 (1H, d, J = 8.1 Hz), 8.14 (1H, d, J = 8.1 Hz).

### Reference Example 22

### 6-(dibenzylamino)-5-nitropyridine-2-carboxylic acid

Methyl 6-(dibenzylamino)-5-nitropyridine-2-carboxylate (6.0 g) obtained in Reference Example 21 was dissolved in methanol (30 mL) and tetrahydrofuran (15 mL), a 8N aqueous sodium hydroxide solution (10 mL) was added, and the mixture was stirred at room temperature for 2 hr. The reaction mixture was cooled to 0°C, neutralized with 6N hydrochloric acid, and extracted with chloroform. The aqueous layer was extracted again with chloroform, and the extracts were combined, washed with saturated brine, dried over anhydrous magnesium sulfate, and concentrated under reduced pressure. The residue was purified by silica gel column chromatography (eluent: ethyl acetate→ethyl acetate-methanol=9:1) to give the title compound as a yellow oil (yield 5.8g, yield 98%).
¹H-NMR (CDCl₃) δ: 4.60 (4H, s), 7.16-7.20 (4H, m), 7.26-7.32 (6H, m), 7.61 (1H, d, J = 8.1 Hz), 8.24 (1H, d, J = 8.1 Hz).

### Reference Example 23

### N,N-dibenzyl-6-(morpholin-4-ylcarbonyl)-3-nitropyridine-2-amine

To a solution (20 mL) of 6-(dibenzylamino)-5-nitropyridine-2-carboxylic acid (3 g) obtained in Reference Example 22 in dichloromethane were added morpholine (0.99 g), 1-ethyl-3-(3'-dimethylaminopropyl)carbodiimide hydrochloride (2.2 g) and 4-dimethylaminopyridine (92 mg), and the mixture was stirred at room temperature for 5 hr. Water was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=2:1) to give the title compound as yellow crystals (yield 1.9 g, yield 58%).
¹H-NMR (CDCl₃) δ: 3.05 (2H, t, J = 4.8 Hz), 3.27 (2H, t, J = 4.8 Hz), 3.71 (4H, s), 4.60 (4H, s), 7.11 (1H, d, J = 8.1 Hz), 7.14-7.17 (4H, m), 7.24-7.32 (6H, m), 8.21 (1H, d, J = 8.1 Hz).

### Reference Example 24

### N-[2-(benzyloxy)ethyl]-6-(dibenzylamino)-5-nitropyridine-2-carboxamide

Using 6-(dibenzylamino)-5-nitropyridine-2-carboxylic acid (3.0 g) obtained in Reference Example 22 and 2-(benzyloxy)ethanamine (1.5 g), methods similar to those of Reference Example 23 were carried out to give the title compound (3.1 g) as a yellow oil.
¹H-NMR (CDCl₃) δ: 3.60-3.62 (4H, m), 4.51 (2H, s), 4.55 (4H, s), 7.14-7.32 (15H, m), 7.61 (1H, d, J = 8.1 Hz), 7.78 (1H, br), 8.21 (1H, d, J = 8.1 Hz).

### Reference Example 25

### 7-chloro-2,3,4-trimethyl-1H-pyrrolo[2,3-c]pyridine

Using 2-chloro-5-methyl-3-nitropyridine (2.0 g), methods similar to those of Reference Example 1 were carried out to give the title compound (687 mg) as a pale-yellow solid.
¹H-NMR (CDCl₃) δ: 2.37 (3H, s), 2.40 (3H, s), 2.59 (3H, s), 7.67 (1H, s), 8.17 (1H, br s).

### Reference Example 26

### N,N-dibenzyl-2-methyl-1H-pyrrolo[2,3-c]pyridine-7-amine

A solution (120 ml) of N,N-dibenzyl-3-nitropyridine-2-amine (5.3 g) obtained in Reference Example 16 in tetrahydrofuran was cooled to -78°C, a 0.5M isopropenylmagnesium bromide-tetrahydrofuran solution (100 ml) was added, and the mixture was stirred for 4 hr while gradually raising the temperature to -20°C. A saturated aqueous ammonium chloride solution was added to the reaction mixture. The mixture was returned to room temperature, and the organic layer was washed with saturated brine, dried over anhydrous sodium sulfate, and concentrated under reduced pressure. The residue was purified by silica gel column chromatography (eluent: hexane-ethyl acetate=9:1→4:1). The obtained yellow oil was left standing overnight at room temperature to allow crystallization. Recrystallization from diisopropyl ether, hexane and a small amount of ethanol gave the title compound as colorless crystals (255 mg, yield 4.7%).
¹H-NMR (CDCl₃) δ: 2.22 (3H, s), 4.77 (4H, s), 6.11-6.12 (1H, m), 6.94-6.96 (1H, m), 7.22-7.37 (10H, m), 7.66 (1H, br), 7.79 (1H, d, J = 5.7 Hz)

### Reference Example 27

### N,N-dibenzyl-5-chloro-2-methyl-1H-pyrrolo[2,3-c]pyridine-7-amine

Using N,N-dibenzyl-6-chloro-3-nitropyridine-2-amine (28.8 g) obtained in Reference Example 17, reactions under conditions similar to those of Reference Example 26 were carried out to give the title compound (10 g) as a red brown oil.
¹H-NMR (CDCl₃) δ: 2.17 (3H, s), 4.79 (4H, s), 6.03-6.04 (1H, m), 6.87 (1H, s), 7.21-7.39 (10H, m), 7.61 (1H, br).

### Reference Example 28

### N,N-dibenzyl-5-chloro-2,3-dimethyl-1H-pyrrolo[2,3-c]pyridine-7-amine

A solution (200 ml) of N,N-dibenzyl-6-chloro-3-nitropyridine-2-amine (10.4 g) obtained in Reference Example 17 in tetrahydrofuran was cooled to -78°C, and a 0.5M 1-methyl-1-propenylmagnesium bromide-tetrahydrofuran solution (200 ml) was added. The mixture was stirred for 4 hr while gradually raising the temperature to -20°C. A saturated aqueous ammonium chloride solution was added to the reaction mixture and the mixture was returned to room temperature. The organic layer was washed with saturated brine, dried over anhydrous sodium sulfate, and concentrated under reduced pressure. The residue was purified by silica gel column chromatography (eluent: hexane-ethyl acetate=12:1→4:1). The obtained yellow oil was left standing at room temperature for 2 days to allow crystallization. Recrystallization from a mixed solvent of diisopropyl ether and ethyl acetate gave the title compound as colorless crystals (1.1 g).
¹H-NMR (CDCl₃) δ: 2.07 (3H, s), 2.09 (3H, s), 4.80 (4H, s), 6.87 (1H, s), 7.24-7.45 (11H, m).

### Reference Example 29

### N,N-dibenzyl-2,3-dimethyl-5-(trifluoromethyl)-1H-pyrrolo[2,3-c]pyridine-7-amine

Using N,N-dibenzyl-3-nitro-6-(trifluoromethyl)pyridine-2-amine (6.4 g) obtained in Reference Example 18, reactions under conditions similar to those of Reference Example 28 were carried out to give the title compound (2.9 g) as a brown oil.
¹H-NMR (CDCl₃) δ: 2.11 (3H, s), 2.13 (3H, s), 4.86 (4H, s), 7.22-7.45 (11H, m), 7.62 (1H, br s).

### Reference Example 30

### 7-(dibenzylamino)-2,3-dimethyl-1H-pyrrolo[2,3-c]pyridine-5-carbonitrile

Using 6-(dibenzylamino)-5-nitropyridine-2-carbonitrile (4.6 g) obtained in Reference Example 20, reactions under conditions similar to those of Reference Example 28 were carried out, washed obtained brown solid with diisopropyl ether and gave the title compound (1.43 g) as a pale-brown powder.
¹H-NMR (CDCl₃) δ: 2.11-2.12 (6H, m), 4.84 (4H, s), 7.27-7.38 (11H, m), 7.72 (1H, br s).

### Reference Example 31

### N,N-dibenzyl-2,3-dimethyl-5-(morpholin-4-ylcarbonyl)-1H-pyrrolo[2,3-c]pyridine-7-amine

Using N,N-dibenzyl-6-(morpholin-4-ylcarbonyl)-3-nitropyridine-2-amine (1.9 g) obtained in Reference Example 23, reactions under conditions similar to those of Reference Example 28 were carried out to give the title compound (443 mg) as yellow crystals.
¹H-NMR (CDCl₃) δ: 2.15 (3H, s), 2.17 (3H, s), 3.19 (2H, br), 3.44 (2H, br), 3.70 (4H, br), 4.84 (4H, s), 7.24-7.33 (10H, m), 7.48 (1H, s), 7.65 (1H, br s).

### Reference Example 32

### N-[2-(benzyloxy)ethyl]-7-(dibenzylamino)-2,3-dimethyl-1H-pyrrolo[2,3-c]pyridine-5-carboxamide

Using N-[2-(benzyloxy)ethyl]-6-(dibenzylamino)-5-nitropyridine-2-carboxamide (3.1 g) obtained in Reference Example 24, reactions under conditions similar to those of Reference Example 28 were carried out to give the title compound (915 mg) as colorless crystals. ¹H-NMR (CDCl₃) δ: 2.14 (3H, s), 2.18 (3H, s), 3.54-3.62 (4H, m), 4.44 (2H, s), 4.82 (4H, s), 7.23-7.39 (15H, m), 7.65 (1H, br s), 7.89 (1H, s), 8.12 (1H, br).

### Reference Example 33

### 7-chloro-1,2,3-trimethyl-1H-pyrrolo[2,3-c]pyridine

Sodium hydride (60% in oil, 329 mg) was washed twice with hexane and suspended in N,N-dimethylformamide (15 mL). A solution (5 mL) of 7-chloro-2,3-dimethyl-1H-pyrrolo[2,3-c]pyridine (1.19 g) obtained in Reference Example 2 in N,N-dimethylformamide was added dropwise at 0°C. After stirring at the same temperature for 15 min, a solution (5 mL) of iodomethane (0.50 mL) in N,N-dimethylformamide was added dropwise at 0°C and the mixture was stirred at room temperature for 14 hr. A saturated aqueous sodium hydrogen carbonate solution was added to the reaction mixture and the mixture was extracted with ethyl acetate. The extract was washed with saturated aqueous sodium hydrogen carbonate solution, water and saturated brine, dried over anhydrous magnesium sulfate, and concentrated under reduced pressure. The residue was crystallized from diisopropyl ether to give the title compound as white crystals (yield 0.93 g, yield 72%).
¹H-NMR (CDCl₃) δ: 2.22 (3H, s), 2.37 (3H, s), 4.04 (3H, s), 7.28 (1H, d, J = 5.4 Hz), 7.91 (1H, d, J = 5.4 Hz).

### Reference Example 34

### 7-chloro-1-ethyl-2,3-dimethyl-1H-pyrrolo[2,3-c]pyridine

Using 7-chloro-2,3-dimethyl-1H-pyrrolo[2,3-c]pyridine (1.20 g) obtained in Reference Example 2 and iodoethane (0.64 mL), methods similar to those of Reference Example 33 were carried out to give the title compound (1.01 g) as white crystals.
¹H-NMR (CDCl₃) δ: 1.36 (3H, t, J = 7.2 Hz), 2.21 (3H, s), 2.37 (3H, s), 4.51 (2H, q, J = 7.2 Hz), 7.28 (1H, d, J = 5.4 Hz), 7.91 (1H, d, J = 5.4 Hz).

### Reference Example 35

### 7-chloro-2,3-dimethyl-1-propyl-1H-pyrrolo[2,3-c]pyridine

Using 7-chloro-2,3-dimethyl-1H-pyrrolo[2,3-c]pyridine (181 mg) obtained in Reference Example 2 and 1-iodopropane (204 mg), methods similar to those of Reference Example 33 were carried out to give the title compound (198 mg) as colorless crystals.
¹H-NMR (CDCl₃) δ: 0.97 (3H, t, J = 6.9 Hz), 1.71-1.83 (2H, m), 2.21 (3H, s), 2.37 (3H, s), 4.35-4.41 (2H, m), 7.27 (1H, d, J = 5.1 Hz), 7.91 (1H, d, J = 5.1 Hz)

### Reference Example 36

### 7-chloro-1-isobutyl-2,3-dimethyl-1H-pyrrolo[2,3-c]pyridine

Using 7-chloro-2,3-dimethyl-1H-pyrrolo[2,3-c]pyridine (1.48 g) obtained in Reference Example 2 and isobutyl bromide (1.20 mL), methods similar to those of Reference Example 33 were carried out to give the title compound (1.61 g) as white crystals.
¹H-NMR (CDCl₃) δ: 0.90 (6H, d, J = 6.6 Hz), 2.21-2.30 (m, 1H), 2.45 (3H, d, J = 0.9 Hz), 4.23 (2H, br d, J = 6.9 Hz), 6.29 (1H, d, J = 0.9 Hz), 7.31 (1H, d, J = 5.4 Hz), 7.92 (1H, d, J = 5.4 Hz).

### Reference Example 37

### 7-chloro-1-isobutyl-2-methyl-1H-pyrrolo[2,3-c]pyridine

Using 7-chloro-2-methyl-1H-pyrrolo[2,3-c]pyridine (1.48 g) obtained in Reference Example 1 and isobutyl bromide (1.2 mL), methods similar to those of Reference Example 3 were carried out to give the title compound (1.61 g) as an oil.
¹H-NMR (CDCl₃) δ: 0.90 (6H, d, J = 6.6 Hz), 2.21-2.30 (1H, m), 2.45 (3H, d, J = 0.9 Hz), 4.23 (2H, br d, J = 6.9 Hz), 6.29 (1H, d, J = 0.9 Hz), 7.31 (1H, d, J = 5.4 Hz), 7.92 (1H, d, J = 5.4 Hz).

### Reference Example 38

### N,N-dibenzyl-2-methyl-1-propyl-1H-pyrrolo[2,3-c]pyridine-7-amine

Sodium hydride (60% in oil, 58 mg) was suspended in N,N-dimethylformamide (5 mL), a solution (5 mL) of N,N-dibenzyl-2-methyl-1H-pyrrolo[2,3-c]pyridine-7-amine (327 mg) obtained in Reference Example 26 in N,N-dimethylformamide was added dropwise. After stirring at the same temperature for 20 min, 1-iodopropane (374 mg) was added dropwise at 0°C and the mixture was stirred at room temperature for 2 hr. A 6% aqueous sodium hydrogen carbonate solution was added to the reaction mixture, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=99:1→9:1) to give the title compound as an orange oil (yield 237 mg: including impurity).
¹H-NMR (CDCl₃) δ: 0.88 (3H, t, J = 7.5 Hz), 1.49-1.59 (2H, m), 2.43 (3H, s), 4.20-4.43 (6H, m), 6.26 (1H, s), 7.05-7.08 (4H, m), 7.16 (1H, d, J = 5.4 Hz), 7.18-7.30 (6H, m), 7.90 (1H, d, J = 5.7 Hz).

### Reference Example 39

### N,N-dibenzyl-5-chloro-2-methyl-1-propyl-1H-pyrrolo[2,3-c]pyridine-7-amine

Using N,N-dibenzyl-5-chloro-2-methyl-1H-pyrrolo[2,3-c]pyridine-7-amine (10 g) obtained in Reference Example 27, reactions under conditions similar to those of Reference Example 38 were carried out to give the title compound (7.7g: including impurity) as an orange oil.
¹H-NMR (CDCl₃) δ: 0.85 (3H, t, J = 7.5 Hz), 1.40-1.55 (2H, m), 2.39 (3H, s), 4.30-4.35 (6H, m), 6.19 (1H, s), 7.12-7.29 (11H, m).

### Reference Example 40

### N,N-dibenzyl-2,3-dimethyl-1-propyl-5-(trifluoromethyl)-1H-pyrrolo[2,3-c]pyridine-7-amine

Using N,N-dibenzyl-2,3-dimethyl-5-(trifluoromethyl)-1H-pyrrolo[2,3-c]pyridine-7-amine (1.0 g) obtained in Reference Example 29, reactions under conditions similar to those of Reference Example 38 were carried out to give the title compound (576 mg: including impurity) as an orange oil.
¹H-NMR (CDCl₃) δ: 0.86 (3H, t, J = 7.5 Hz), 1.39-1.50 (2H, m), 2.21 (3H, s), 2.34 (3H, s), 4.34 (4H, br), 4.38-4.44 (2H, m), 7.13-7.26 (10H, m), 7.50 (1H, s).

### Reference Example 41

### 1-[7-(dibenzylamino)-2-methyl-1H-pyrrolo[2,3-c]pyridin-3-yl]ethanone

N,N-Dibenzyl-2-methyl-1H-pyrrolo[2,3-c]pyridine-7-amine (327 mg) obtained in Reference Example 26 was dissolved in nitromethane (3 mL) and 1,2-dichloroethane (3 mL), and aluminum (III) chloride (133 mg) and acetyl chloride (79 mg) were added at 0°C. After stirring at the same temperature for 1 hr, the same amount of aluminum chloride and acetyl chloride was added again at 0°C. After further stirring for 1 hr, the mixture was weakly basified with a 8N aqueous sodium hydroxide solution, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→4:1) to give the title compound as a pale-yellow oil (yield 229 mg, yield 62%).
¹H-NMR (CDCl₃) δ: 2.44 (3H, s), 2.60 (3H, s), 4.80 (4H, s), 7.26-7.38 (11H, m), 8.03 (1H, d, J = 5.7 Hz), 8.10 (1H, br s).

### Reference Example 42

### 1-[5-chloro-7-(dibenzylamino)-2-methyl-1-propyl-1H-pyrrolo[2,3-c]pyridin-3-yl]propan-1-one

Using N,N-dibenzyl-5-chloro-2-methyl-1-propyl-1H-pyrrolo[2,3-c]pyridine-7-amine (1.0 g) obtained in Reference Example 39 and propionyl chloride (458 mg), reactions under conditions similar to those of Reference Example 41 were carried out to give the title compound (513 mg) as a yellow oil.
¹H-NMR (CDCl₃) δ: 0.85 (3H, t, J = 7.2 Hz), 1.25 (3H, t, J = 7.4 Hz), 1.41-1.57 (2H, m), 2.73 (3H, s), 2.96 (2H, q, J = 7.4 Hz), 4.29 (4H, br), 4.47-4.56 (2H, m), 7.07-7.13 (4H, m), 7.22-7.29 (6H, m), 7.60 (1H, s).

### Reference Example 43

### 1-[7-(dibenzylamino)-2-methyl-1H-pyrrolo[2,3-c]pyridin-3-yl]ethanol

A solution (5 mL) of 1-[7-(dibenzylamino)-2-methyl-1H-pyrrolo[2,3-c]pyridin-3-yl]ethanone (385 mg) obtained in Reference Example 41 in ethanol was cooled to 0°C, sodium borohydride (156 mg) was added. After stirring overnight at room temperature, the mixture was treated with acetic acid, weakly basified with a 6% aqueous sodium hydrogen carbonate solution, and extracted with ethyl acetate. The extract was washed with saturated brine, dried over anhydrous sodium sulfate, and concentrated under reduced pressure. The residue was purified by silica gel column chromatography (eluent: hexane-ethyl acetate=6:1→1:1) to give the title compound as a colorless oil (yield 217 mg, yield 56%).
¹H-NMR (CDCl₃) δ: 1.61 (2H, d, J = 6.6 Hz), 1.69 (1H, br), 2.21 (3H, s), 4.78 (4H, s), 5.13 (1H, q, J = 6.6 Hz), 7.19 (1H, d, J = 5.4 Hz), 7.25-7.37 (10H, m), 7.61 (1H, br s), 7.88 (1H, d, J = 5.4 Hz).

### Reference Example 44

### 1-[5-chloro-7-(dibenzylamino)-2-methyl-1-propyl-1H-pyrrolo[2,3-c]pyridin-3-yl]propan-1-ol

Using 1-[5-chloro-7-(dibenzylamino)-2-methyl-1-propyl-1H-pyrrolo[2,3-c]pyridin-3-yl]propan-1-one (513 mg) obtained in Reference Example 42, reactions under conditions similar to those of Reference Example 43 were carried out to give the title compound (415 mg) as a yellow oil.
¹H-NMR (CDCl₃) δ: 0.81-0.88 (6H, m), 1.38-1.47 (2H, m), 1.80-2.06 (2H, m), 2.37 (1H, s), 4.20-4.38 (6H, m), 4.81-4.86 (1H, m), 7.12-7.29 (10H, m), 7.40 (1H, s).

### Reference Example 45

### N,N-dibenzyl-3-ethyl-2-methyl-1H-pyrrolo[2,3-c]pyridine-7-amine

A solution (1 mL) of 1-[7-(dibenzylamino)-2-methyl-1H-pyrrolo[2,3-c]pyridin-3-yl]ethanol (217 mg) obtained in Reference Example 43 in trifluoroacetic acid was cooled to 0°C, and triethylsilane (135 mg) was added dropwise. After stirring at the same temperature for 10 min, the mixture was treated with a 8N aqueous sodium hydroxide solution, and extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as a pale-yellow solid (yield 135 mg, yield 65%).
¹H-NMR (CDCl₃) δ: 1.18 (3H, t, J = 7.5 Hz), 2.15 (3H, s), 2.62 (2H, q, J = 7.5 Hz), 4.79 (4H, s), 6.97 (1H, d, J = 8.4 Hz), 7.23-7.37 (10H, m), 7.51 (1H, br s), 7.87 (1H, d, J = 8.4 Hz).

### Reference Example 46

### N,N-dibenzyl-5-chloro-2-methyl-1,3-dipropyl-1H-pyrrolo[2,3-c]pyridine-7-amine

Using 1-[5-chloro-7-(dibenzylamino)-2-methyl-1-propyl-1H-pyrrolo[2,3-c]pyridin-3-yl]propan-1-ol (415 mg) obtained in Reference Example 44, reactions under conditions similar to those of Reference Example 45 were carried out to give the title compound (404 mg) as a colorless oil.
¹H-NMR (CDCl₃) δ: 0.78-0.94 (6H, m), 1.37-1.60 (4H, m), 2.31 (3H, s), 2.58 (2H, t, J = 7.2 Hz), 4.28-4.36 (6H, m), 7.10-7.31 (11H, m).

### Reference Example 47

### 7-(dibenzylamino)-2,3-dimethyl-1H-pyrrolo[2,3-c]pyridine-5-carboxamide

A mixed solution of a 30% hydrogen peroxide solution (1 mL) and a 3N aqueous sodium hydroxide solution (3 mL) was cooled to 0°C, and a solution (3 mL) of 7-(dibenzylamino)-2,3-dimethyl-1H-pyrrolo[2,3-c]pyridine-5-carbonitrile (366 mg) obtained in Reference Example 30 in dimethylsulfoxide was added dropwise. After stirring at room temperature for 30 min, the mixture was extracted with ethyl acetate. The extract was washed twice with water and saturated brine, dried over anhydrous sodium sulfate, and concentrated under reduced pressure. The residue was purified by silica gel column chromatography (eluent: hexane-ethyl acetate=9:1→1:1). The obtained pale-yellow solid was washed with a mixed solvent of ethyl acetate and hexane to give the title compound as a pale-yellow powder (yield 262 mg, yield 68%).
¹H-NMR (DMSO-d₆) δ: 2.15 (3H, s), 2.36 (3H, s), 4.76 (4H, s), 7.15-7.29 (11H, m), 7.47 (1H, br), 7.64 (1H, s), 11.06 (1H, br s).

### Reference Example 48

### 7-(dibenzylamino)-2-methyl-1-propyl-1H-pyrrolo[2,3-c]pyridine-3-carbaldehyde

N,N-Dibenzyl-2-methyl-1-propyl-1H-pyrrolo[2,3-c]pyridine-7-amine (237 mg) obtained in Reference Example 38 was dissolved in nitromethane (1 mL) and 1,2-dichloroethane (1 mL), and aluminum (III) chloride (85 mg) and dichloromethyl methyl ether (74 mg) were added at 0°C. After stirring at the same temperature for 30 min, the same amount of aluminum (III) chloride and dichloromethyl methyl ether was added, and the mixture was stirred for 16 hr. The reaction mixture was weakly basified with a 8N aqueous sodium hydroxide solution, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→2:1) to give the title compound as a yellow oil (yield 64 mg, yield 25%).
¹H-NMR (CDCl₃) δ: 0.90 (3H, t, J = 7.5 Hz), 1.51-1.61 (2H, m), 2.69 (3H, s), 4.29 (4H, br), 4.47-4.53 (2H, m), 7.03-7.09 (4H, m), 7.20-7.27 (6H, m), 7.90 (1H, d, J = 5.1 Hz), 8.14 (1H, d, J = 5.1 Hz), 10.18 (1H, s).

### Reference Example 49

### 7-(dibenzylamino)-2-methyl-1-propyl-1H-pyrrolo[2,3-c]pyridine-3-carbonitrile

To a solution (2 mL) of 7-(dibenzylamino)-2-methyl-1-propyl-1H-pyrrolo[2,3-c]pyridine-3-carbaldehyde (398 mg) obtained in Reference Example 48 in formic acid was added hydroxylamine hydrochloride (90 mg) at room temperature, and the mixture was heated under reflux for 1 hr. The reaction mixture was returned to room temperature, diluted with water, neutralized with a 8N aqueous sodium hydroxide solution at 0°C, and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) to give the title compound as colorless crystals (yield 134 mg, yield 34%). ¹H-NMR (CDCl₃) δ: 0.89 (3H, t, J = 7.2 Hz), 1.49-1.59 (2H, m), 2.58 (3H, m), 4.30 (4H, br), 4.43-4.48 (2H, m), 7.02-7.06 (4H, m), 7.22-7.26 (6H, m), 7.33 (1H, d, J = 5.4 Hz), 8.09 (1H, d, J = 5.1 Hz).

### Reference Example 50

### 2,3-dimethyl-1-propyl-5-(trifluoromethyl)-1H-pyrrolo[2,3-c]pyridine-7-amine

N,N-Dibenzyl-2,3-dimethyl-1-propyl-5-(trifluoromethyl)-1H-pyrrolο[2,3-c]pyridine-7-amine (576 mg) obtained in Reference Example 40 was dissolved in methanol (3 mL), and the mixture was adjusted to pH 2-3 with a 1M hydrogen chloride-diethyl ether solution. To this solution was added a 10% palladium carbon 50% water-containing product (300 mg), and the mixture was stirred at under a hydrogen atmosphere for 1.5 hr. The reaction mixture was filtered through hyflo super-cel (trade name: manufactured by Celite Co.), washed with methanol, and the filtrate was concentrated under reduced pressure. To the residue was added a 6% aqueous sodium hydrogen carbonate solution and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=3:1→1:1). The obtained colorless powder was washed with a mixed solvent of ethyl acetate and hexane to give the title compound as a colorless powder (yield 239 mg, yield 81%). ¹H-NMR (CDCl₃) δ: 0.98 (3H, t, J = 7.5 Hz), 1.76-1.89 (2H, m), 2.19 (3H, s), 2.34 (3H, s), 4.14-4.19 (2H, m), 4.67 (2H, br s), 7.29 (1H, s).

### Reference Example 51

### 2,3-dimethyl-5-(trifluoromethyl)-1H-pyrrolo[2,3-c]pyridine-7-amine

Using N,N-dibenzyl-2,3-dimethyl-5-(trifluoromethyl)-1H-pyrrolo[2,3-c]pyridine-7-amine (1.7 g) obtained in Reference Example 29, methods similar to those of Reference Example 50 were carried out to give the title compound (657 mg) as colorless crystals.
¹H-NMR (CDCl₃) δ: 2.20 (3H, s), 2.38 (3H, s), 5.00 (2H, br s), 7.33 (1H, s), 10.66 (1H, br s).

### Reference Example 52

### 2-methyl-1,3-dipropyl-1H-pyrrolo[2,3-c]pyridine-7-amine

Using N,N-dibenzyl-5-chloro-2-methyl-1,3-dipropyl-1H-pyrrolo[2,3-c]pyridine-7-amine (404 mg) obtained in Reference Example 46, methods similar to those of Reference Example 50 were carried out to give the title compound (145 mg) as colorless crystals.
¹H-NMR (CDCl₃) δ: 0.88-0.99 (6H, m), 1.50-1.63 (2H, m), 1.73-1.85 (2H, m), 2.33 (3H, s), 2.60 (2H, t, J = 7.5 Hz), 4.13-4.19 (2H, m), 4.45 (2H, br s), 6.91 (1H, d, J = 5.7 Hz), 7.65 (1H, d, J = 5.7 Hz).

### Reference Example 53

### 2,3-dimethyl-5-(morpholin-4-ylcarbonyl)-1H-pyrrolo[2,3-c]pyridine-7-amine

Using N,N-dibenzyl-2,3-dimethyl-5-(morpholin-4-ylcarbonyl)-1H-pyrrolo[2,3-c]pyridine-7-amine (443 mg) obtained in Reference Example 31, methods similar to those of Reference Example 50 were carried out to give the title compound (180 mg) as colorless crystals. ¹H-NMR (CDCl₃) δ: 2.10 (3H, s), 2.27 (3H, s), 3.72 (8H, br), 4.98 (2H, br), 7.00 (1H, s), 10.40 (1H, s).

### Reference Example 54

### 7-amino-N-(2-hydroxyethyl)-2,3-dimethyl-1H-pyrrolo[2,3-c]pyridine-5-carboxamide

Using N-[2-(benzyloxy)ethyl]-7-(dibenzylamino)-2,3-dimethyl-1H-pyrrolo[2,3-c]pyridine-5-carboxamide (915 mg) obtained in Reference Example 32, methods similar to those of Reference Example 50 were carried out to give the title compound (380 mg) as colorless crystals. ¹H-NMR (CDCl₃) δ: 2.13 (3H, s), 2.34 (3H, s), 3.34-3.41 (2H, m), 3.51 (2H, q, J = 5.7 Hz), 4.83 (1H, t, J = 5.1 Hz), 5.92 (2H, br), 7.47 (1H, s), 8.27 (1H, t, J = 5.7 Hz), 10.90 (1H, br s).

### Reference Example 55

### 2-methyl-1-propyl-1H-pyrrolo[2,3-c]pyridine-7-amine

To a solution (50 mL) of N-benzyl-2-methyl-1-propyl-1H-pyrrolo[2,3-c]pyridine-7-amine (1.8 g) obtained in Example 2 in methanol were added a 10% palladium carbon 50% water-containing product (3.6 g) and ammonium formate (2.0 g), and the mixture was heated under reflux for 3 hr. The reaction mixture was returned to room temperature and filtered. After washing with methanol, the filtrate was concentrated under reduced pressure. The residue was purified by basic silica gel column chromatography (eluent: hexane-ethyl acetate=2:1→1:2). The obtained colorless powder was washed with a mixed solvent of diisopropyl ether and hexane to give the title compound as a colorless powder (yield 602 mg, yield 50%).
¹H-NMR (CDCl₃) δ: 0.98 (3H, t, J = 7.5 Hz), 1.80-1.89 (2H, m), 2.41 (3H, s), 4.14-4.19 (2H, m), 4.44 (2H, m), 6.17 (1H, s), 6.92 (1H, d, J = 5.4 Hz), 7.66 (1H, d, J = 5.4 Hz).

### Reference Example 56

### 4-fluoro-2-methylbenzaldehyde

To a mixture of magnesium (3.36 g) and tetrahydrofuran (10 mL) was slowly added dropwise a solution of 1-bromo-4-fluoro-2-methylbenzene (24.8 g) in tetrahydrofuran (200 mL). After completion of the dropwise addition, the reaction mixture was stirred at room temperature for 30 min. To the obtained solution was added a solution (10 mL) of N,N-dimethylformamide (15.0 mL) in tetrahydrofuran at 0°C. The reaction mixture was stirred at room temperature for 1 hr. 1N Hydrochloric acid and water were added to the reaction mixture, and the mixture was extracted with ethyl acetate. The extract was washed with 1N hydrochloric acid, water and saturated brine, dried over anhydrous magnesium sulfate, and concentrated under reduced pressure. The residue was distilled under reduced pressure (66-68°C/6 mmHg) to give the title compound as a colorless oil (yield 12.1 g, yield 66%).
¹H-NMR (CDCl₃) δ: 2.68 (3H, s), 6.93-7.06 (2H, m), 7.79-7.84 (1H, m), 10.18 (1H, s).

### Reference Example 57

### 2,6-diethylbenzaldehyde

Using 2-bromo-1,3-diethylbenzene (1.43 g), methods similar to those of Reference Example 56 were carried out to give the title compound (0.62 g) as a yellow oil. ¹H-NMR (CDCl₃) δ: 1.25 (6H, t, J = 7.8 Hz), 2.97 (4H, q, J = 7.8 Hz), 7.11-7.14 (2H, m), 7.36-7.41 (1H, m), 10.60 (1H, s).

### Reference Example 58

### (2,4-dimethylphenyl)methanol

To a solution (100 mL) of 2,4-dimethylbenzaldehyde (10.4 g) in methanol was added sodium borohydride (0.83 g) at 0°C. The reaction mixture was stirred at 0°C for 1 hr, and the reaction mixture was concentrated under reduced pressure. Water was added to the residue, and the mixture was extracted with ethyl acetate. The extract was washed with a saturated aqueous sodium hydrogen carbonate solution, water and saturated brine, dried over anhydrous magnesium sulfate, and concentrated under reduced pressure to give the title compound as a colorless oil (yield 10.8 g, yield quantitative).
¹H-NMR (CDCl₃) δ: 1.55 (1H, t, J = 5.4 Hz), 2.31 (3H, s), 2.33 (3H, s), 4.64 (2H, d, J = 5.4 Hz), 6.98-7.00 (2H, m), 7.19-7.22 (1H, m).

### Reference Example 59

### (4-fluoro-2-methylphenyl)methanol

Using 4-fluoro-2-methylbenzaldehyde (10.0 g) obtained in Reference Example 56 and sodium borohydride (0.80 g), methods similar to those of Reference Example 58 were carried out to give the title compound (10.9 g) as a colorless oil.
¹H-NMR (CDCl₃) δ: 1.60 (1H, t, J = 5.4 Hz), 2.35 (3H, s), 4.65 (2H, d, J = 5.4 Hz), 6.83-6.89 (2H, m), 7.26-7.31 (1H, m).

### Reference Example 60

### 1-(bromomethyl)-2,4-dimethylbenzene

To a solution (100 mL) of (2,4-dimethylphenyl)methanol (10.77 g) obtained in Reference Example 58 in diisopropyl ether was added phosphorus tribromide (4.90 mL) at 0°C, and the reaction mixture was stirred at room temperature for 1 hr. Water was added to the reaction mixture and the mixture was extracted with ethyl acetate. The extract was washed twice with water, a saturated aqueous sodium hydrogen carbonate solution and saturated brine, dried over anhydrous magnesium sulfate, and concentrated under reduced pressure to give the title compound as a colorless oil (yield 15.5g, yield quantitative).
¹H-NMR (CDCl₃) δ: 2.30 (3H, s), 2.37 (3H, s), 4.50 (2H, s), 6.96-6.99 (2H, m), 7.17-7.19 (1H, m).

### Reference Example 61

### 1-(bromomethyl)-4-fluoro-2-methylbenzene

Using (4-fluoro-2-methylphenyl)methanol (9.0 g) obtained in Reference Example 59 and phosphorus tribromide (4.1 mL), methods similar to those of Reference Example 60 were carried out to give the title compound (11.6 g) as a colorless oil.
¹H-NMR (CDCl₃) δ: 2.40 (3H, s), 4.48 (2H, s), 6.82-6.91 (2H, m), 7.24-7.28 (1H, m).

### Reference Example 62

### 2-[(2,4-dimethylphenyl)methyl]-1H-isoindole-1,3 (2H)-dione

To a solution (150 mL) of potassium phthalimide (16.0 g) in N,N-dimethylformamide was added a solution (50 mL) of 1-(bromomethyl)-2,4-dimethylbenzene (15.5 g) obtained in Reference Example 60 in N,N-dimethylformamide at 0°C. The reaction mixture was stirred at room temperature for 12 hr. The insoluble material was filtered off. Water was added to the filtrate, and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was crystallized from ethyl acetate-hexane to give the title compound as white crystals (yield 15.9 g, yield 77%).
¹H-NMR (CDCl₃) δ: 2.27 (3H, s), 2.45 (3H, s), 4.82 (2H, s), 6.93 (1H, d, J = 7.5 Hz), 6.97 (1H, s), 7.18 (1H, d, J = 7.5 Hz), 7.67-7.73 (2H, m), 7.80-7.86 (2H, m).

### Reference Example 63

### 2-[(4-fluoro-2-methylphenyl)methyl]-1H-isoindole-1,3 (2H)-dione

Using 1-(bromomethyl)-4-fluoro-2-methylbenzene (11.5 g) obtained in Reference Example 61 and potassium phthalimide (11.5 g), methods similar to those of Reference Example 62 were carried out to give the title compound (13.9 g) as white crystals.
¹H-NMR (CDCl₃) δ: 2.48 (3H, s), 4.41 (2H, s), 6.78-6.88 (2H, m), 7.27-7.30 (1H, m), 7.70-7.74 (2H, m), 7.81-7.87 (2H, m).

### Reference Example 64

### 2,4-dimethylbenzylamine

To a solution (150 mL) of 2-[(2,4-dimethylphenyl)methyl]-1H-isoindole-1,3 (2H)-dione (15.0 g) obtained in Reference Example 62 in ethanol was added hydrazine monohydrate (3.0 g) at 0°C. The reaction mixture was stirred at 80°C for 1 hr. After cooling to room temperature, water was added to the reaction mixture and the mixture was extracted with ethyl acetate. 1N Hydrochloric acid was added to the obtained organic layer, and the aqueous layer was washed with ethyl acetate. A 8N aqueous sodium hydroxide solution was added to basify the aqueous layer, and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was distilled under reduced pressure (98-101°C/9-10 mmHg) to give the title compound as a colorless oil (yield 2.2 g, yield 28%). ¹H-NMR (CDCl₃) δ: 1.32 (2H, br s), 2.30 (6H, s), 3.81 (2H, s), 6.98-7.00 (2H, m), 7.16-7.18 (1H, m).

### Reference Example 65

### 4-fluoro-2-methylbenzylamine

Using 2-[(4-fluoro-2-methylphenyl)methyl]-1H-isoindole-1,3(2H)-dione (12.8 g) obtained in Reference Example 63 and hydrazine monohydrate (2.40 g), methods similar to those of Reference Example 45 were carried out to give the title compound (2.29 g) as a yellow oil. ¹H-NMR (CDCl₃) δ: 1.33 (2H, br s), 2.33 (3H, s), 3.81 (2H, s), 6.83-6.88 (2H, m), 7.21-7.26 (1H, m).

### Reference Example 66

### tert-butyl (4-methyl-3-nitropyridin-2-yl)carbamate

To a solution of 2-amino-4-methyl-3-nitropyridine (5.00 g) in tetrahydrofuran (180 mL) was added sodium hydride (60% in oil, 3.28 g) at 0°C. The reaction mixture was stirred at room temperature for 90 min. To the reaction mixture was added a solution of di-tert-butyl dicarbonate (7.14 g) in tetrahydrofuran (20 mL) at 0°C. The reaction mixture was stirred at room temperature for 5 hr. The reaction mixture was poured into ice water, and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was crystallized from ethyl acetate to give the title compound as a white solid (yield 6.33 g, yield 82%). ¹H-NMR (CDCl₃) δ: 1.51 (9H, s), 2.48 (3H, d, J = 0.6 Hz), 7.00 (1H, dd, J = 0.6, 4.8 Hz), 8.03 (1H, br s), 8.38 (1H, d, J = 4.8 Hz).

### Reference Example 67

### tert-butyl benzyl(4-methyl-3-nitropyridin-2-yl)carbamate

Sodium hydride (60% in oil, 201 mg) was washed twice with hexane, and suspended in N,N-dimethylformamide (15 mL). A solution (15 mL) of tert-butyl (4-methyl-3-nitropyridin-2-yl)carbamate (1.01 g) obtained in Reference Example 66 in N,N-dimethylformamide was added dropwise at 0°C. After stirring at the same temperature for 1 hr, a solution (1 mL) of benzyl bromide (0.55 mL) in N,N-dimethylformamide was added dropwise at 0°C and the mixture was stirred at room temperature for 14 hr. A saturated aqueous sodium hydrogen carbonate solution was added to the reaction mixture and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=5:1) and crystallized from diisopropyl ether to give the title compound as white crystals (yield 0.82 g, yield 60%).
¹H-NMR (CDCl₃) δ: 1.35 (9H, s), 2.42 (3H, s), 5.05 (2H, s), 7.07 (1H, d, J = 4.8 Hz), 7.22-7.31 (3H, m), 7.38-7.41 (2H, m), 8.37 (1H, d, J = 4.8 Hz).

### Reference Example 68

### tert-butyl (4-fluoro-2-methylbenzyl)(4-methyl-3-nitropyridin-2-yl)carbamate

Using tert-butyl (4-methyl-3-nitropyridin-2-yl)carbamate (2.67 g) obtained in Reference Example 66 and 1-(bromomethyl)-4-fluoro-2-methylbenzene (2.28 g) obtained in Reference Example 61, methods similar to those of Reference Example 48 were carried out to give the title compound (3.37 g) as a yellow solid.
¹H-NMR (CDCl₃) δ: 1.37 (9H, s), 2.33 (3H, s), 2.40 (3H, s), 5.02 (2H, s), 6.72-6.84 (2H, m), 7.07-7.09 (1H, m), 7.27-7.30 (1H, m), 8.35-8.37 (1H, m).

### Reference Example 69

### methyl 7-[benzyl(tert-butoxycarbonyl)amino]-1H-pyrrolo[2,3-c]pyridine-2-carboxylate

i) potassium (1-{2-[benzyl(tert-butoxycarbonyl)amino]-3-nitropyridin-4-yl}-3-ethoxy-3-oxoprop-1-en-2-olate
   To a suspension of potassium ethoxide (684 mg) in diethyl ether (10 mL) was slowly added diethyl oxalate (1.05 mL) at 0°C. The reaction mixture was stirred at room temperature for 10 min. To the reaction mixture was added a solution of tert-butyl benzyl (4-methyl-3-nitropyridin-2-yl)carbamate (2.41 g) obtained in Reference Example 67 in diethyl ether (20 mL) at 0°C and the mixture was stirred for 1 hr, and further stirred at room temperature for 1 hr. The precipitated solid was collected by filtration to give the title compound as a red solid (2.68 g, yield 79%), which was directly used for the next reaction without purification.
ii) To a solution of potassium (1-{2-[benzyl(tert-butoxycarbonyl)amino]-3-nitropyridin-4-yl}-3-ethoxy-3-oxoprop-1-en-2-olate (2.68 g) obtained in i) in methanol (50 mL) was added a 10% palladium carbon 50% water-containing product (262 mg) and the mixture was stirred under a hydrogen atmosphere for 14 hr. The catalyst was filtered off, and the filtrate was concentrated under reduced pressure. Water was added to the residue, and the mixture was extracted with ethyl acetate. The extract was washed with a saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=4:1). Crystallization from ethyl acetate-hexane gave the title compound as white crystals (yield 602 mg, yield 28%).
   ¹H-NMR (CDCl₃) δ: 1.42 (9H, s), 3.96 (3H, s), 5.26 (2H, s), 7.16-7.30 (6H, m), 7.43 (1H, d, J = 5.4 Hz), 8.10 (1H, d, J = 5.4 Hz), 9.41 (1H, br s).

### Reference Example 70

### methyl 7-[(tert-butoxycarbonyl)(4-fluoro-2-methylbenzyl)amino]-1H-pyrrolo[2,3-c]pyridine-2-carboxylate

i) potassium (1-{2-[(tert-butoxycarbonyl)(4-fluoro-2-methylbenzyl)amino]-3-nitropyridin-4-yl}-3-ethoxy-3-oxoprop-1-en-2-olate
   Using tert-butyl (4-fluoro-2-methylbenzyl)(4-methyl-3-nitropyridin-2-yl)carbamate (2.97 g) obtained in Reference Example 68, reactions under conditions similar to those of Reference Example 69i) were carried out to give the title compound (3.29 g) as a red solid.
ii) Using potassium (1-{2-[(tert-butoxycarbonyl)(4-fluoro-2-methylbenzyl)amino]-3-nitropyridin-4-yl}-3-ethoxy-3-oxoprop-1-en-2-olate (3.29 g) obtained in i), reactions under conditions similar to those of Reference Example 69ii) were carried out to give the title compound (1.13 g) as a white solid.
   ¹H-NMR (CDCl₃) δ: 1.42 (9H, s), 2.24 (3H, s), 3.97 (3H, s), 5.19 (2H, s), 6.73-6.81 (2H, m), 7.15-7.22 (2H, m), 7.40-7.42 (1H, m), 8.06 (1H, d, J = 5.4 Hz), 9.41 (1H, br s).

### Reference Example 71

### ethyl 7-[(tert-butoxycarbonyl)(4-fluoro-2-methylbenzyl)amino]-3-methyl-1H-pyrrolo[2,3-c]pyridine-2-carboxylate

i) ethyl 3-{2-[(tert-butoxycarbonyl)(4-fluoro-2-methylbenzyl)amino]-3-nitropyridin-4-yl}-2-oxobutanoate
   To a solution of potassium (1-{2-[(tert-butoxycarbonyl)(4-fluoro-2-methylbenzyl)amino]-3-nitropyridin-4-yl}-3-ethoxy-3-oxoprop-1-en-2-olate (3.71 g) obtained in Reference Example 70i) in N,N-dimethylformamide (40 mL) was added iodomethane (0.50 mL). The reaction mixture was stirred at room temperature for 3 hr. A saturated aqueous sodium hydrogen carbonate solution was added to the reaction mixture and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as an oil (yield 2.48 g, yield 70%).
ii) To a solution of ethyl 3-{2-[(tert-butoxycarbonyl)(4-fluoro-2-methylbenzyl)amino]-3-nitropyridin-4-yl}-2-oxobutanoate (2.48 g) obtained in i) in ethanol (25 mL) was added a 10% palladium carbon 50% water-containing product (249 mg), and the mixture was stirred under a hydrogen atmosphere for 12 hr. The catalyst was filtered off, and the filtrate was concentrated under reduced pressure. Water was added to the residue, and the mixture was extracted with ethyl acetate. The extract was washed with a saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=5:1) and crystallized from diisopropyl ether to give the title compound as white crystals (yield 0.98 g, yield 44%).
   ¹H-NMR (CDCl₃) δ: 1.42 (9H, s), 1.43 (3H, t, J = 7.2 Hz), 2.23 (3H, s), 2.57 (3H, s), 4.43 (2H, q, J = 7.2 Hz), 5.18 (2H, s), 6.71-6.80 (2H, m), 7.17-7.22 (1H, m), 7.38 (1H, d, J = 5.4 Hz), 8.05 (1H, d, J = 5.4 Hz), 9.09 (1H, br s) .

### Reference Example 72

### methyl 7-[benzyl(tert-butoxycarbonyl)amino]-1-propyl-1H-pyrrolo[2,3-c]pyridine-2-carboxylate

Using methyl 7-[benzyl(tert-butoxycarbonyl)amino]-1H-pyrrolo[2,3-c]pyridine-2-carboxylate (502 mg) obtained in Reference Example 69 and 1-iodopropane (0.15 mL), reactions under conditions similar to those of Reference Example 38 were carried out to give the title compound (500 mg) as a white solid.
¹H-NMR (CDCl₃) δ: 0.65 (3H, br s), 1.33 (11H, br s), 3.90 (3H, s), 4.19-4.29 (1H, m), 4.53-4.56 (1H, m), 5.04-5.15 (2H, m), 7.21-7.25 (4H, m), 7.35 (2H, br s), 7.46 (1H, d, J = 5.4 Hz), 8.14 (1H, d, J = 5.4 Hz).

### Reference Example 73

### ethyl 7-[(tert-butoxycarbonyl)(4-fluoro-2-methylbenzyl)amino]-3-methyl-1-propyl-1H-pyrrolo[2,3-c]pyridine-2-carboxylate

Using ethyl 7-[(tert-butoxycarbonyl)(4-fluoro-2-methylbenzyl)amino]-3-methyl-1H-pyrrolo[2,3-c]pyridine-2-carboxylate (922 mg) obtained in Reference Example 71 and 1-iodopropane (0.25 mL), reactions under conditions similar to those of Reference Example 38 were carried out to give the title compound (954 mg) as an oil. ¹H-NMR (CDCl₃) δ: 0.58 (3H, br s), 1.26-1.46 (14H, m), 2.20 (3H, s), 2.50 (3H, s), 4.11-4.21 (1H, m), 4.30-4.47 (3H, m), 5.00-5.10 (1H, m), 5.15-5.20 (1H, m), 6.67-6.78 (2H, m), 7.20-7.30 (1H, m), 7.43 (1H, d, J = 5.4 Hz), 8.12 (1H, d, J = 5.4 Hz).

### Reference Example 74

### methyl 3-chloro-7-[benzyl(tert-butoxycarbonyl)amino]-1-propyl-1H-pyrrolo[2,3-c]pyridine-2-carboxylate

Using methyl 7-[benzyl(tert-butoxycarbonyl)amino]-1-propyl-1H-pyrrolo[2,3-c]pyridine-2-carboxylate (376 mg) obtained in Reference Example 72 and sulfuryl chloride (0.08 mL), reactions under conditions similar to those of Example 70 were carried out to give the title compound (341 mg) as an oil.
¹H-NMR (CDCl₃) δ: 0.57 (3H, br s), 1.34 (11H, br s), 3.97 (3H, s), 4.10-4.24 (1H, m), 4.41-4.51 (1H, m), 5.08-5.16 (2H, m), 7.18-7.25 (3H, m), 7.32 (2H, br s), 7.51 (1H, d, J = 5.4 Hz), 8.21 (1H, d, J = 5.4 Hz)

### Reference Example 75

### tert-butyl (4-fluoro-2-methylbenzyl)[2-(hydroxymethyl)-3-methyl-1-propyl-1H-pyrrolo[2,3-c]pyridin-7-yl]carbamate

To a suspension of lithium aluminum hydride (331 mg) in tetrahydrofuran (15 mL) was added a solution of ethyl 7-[(tert-butoxycarbonyl)(4-fluoro-2-methylbenzyl)amino]-3-methyl-1-propyl-1H-pyrrolo[2,3-c]pyridine-2-carboxylate (1.63 g) obtained in Reference Example 73 in tetrahydrofuran (10 mL) at 0°C. The reaction mixture was stirred at the same temperature for 2 hr. The reaction mixture was strongly basified by adding a 8N aqueous sodium hydroxide solution, and the mixture was extracted with ethyl acetate. The extract was washed with water and saturated brine, dried over anhydrous sodium sulfate, and concentrated under reduced pressure. The residue was purified by silica gel column chromatography (eluent: hexane-ethyl acetate=2:1). Crystallization from ethyl acetate-hexane gave the title compound as white crystals (yield 1.06 g, yield 71%). ¹H-NMR (CDCl₃) δ: 0.75 (3H, t, J = 7.2 Hz), 1.35 (11H, br s), 1.52 (1H, br t, J = 5.7 Hz), 2.17 (3H, s), 2.29 (3H, s), 3.87-4.09 (2H, m), 4.67-4.80 (2H, m), 4.98-5.17 (2H, m), 6.71-6.78 (2H, m), 7.25-7.35 (2H, m), 8.08 (1H, d, J = 5.4 Hz).

### Example 1

### N-benzyl-1-ethyl-2-methyl-1H-pyrrolo[2,3-c]pyridine-7-amine

7-Chloro-1-ethyl-2-methyl-1H-pyrrolo[2,3-c]pyridine (350 mg) obtained in Reference Example 3 was dissolved in benzylamine (1 mL) and heated at 180°C for 30 min using a microwave focused chemical synthetic reaction apparatus manufactured by CEM. The reaction mixture was cooled to room temperature, water was added and the mixture was extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→4:1) and the obtained yellow oil was crystallized from a mixed solvent of diisopropyl ether and hexane to give the title compound as colorless crystals (yield 108 mg, yield 23%).
¹H-NMR (CDCl₃) δ: 1.37 (3H, t, J = 7.2 Hz), 2.38 (3H, s), 4.21 (2H, q, J = 7.2 Hz), 4.58 (1H, br), 4.77 (2H, d, J = 5.7 Hz), 6.16 (1H, s), 6.87 (1H, d, J = 5.4 Hz), 7.24-7.45 (5H, m), 7.76 (1H, d, J = 5.4 Hz).
LC/MS: 266 [M+H]⁺.

### Example 2

### N-benzyl-2-methyl-1-propyl-1H-pyrrolo[2,3-c]pyridine-7-amine

Using 7-chloro-2-methyl-1-propyl-1H-pyrrolo[2,3-c]pyridine (402 mg) obtained in Reference Example 4 and benzylamine (1.0 mL), reactions under conditions similar to those of Example 1 were carried out to give the title compound (173 mg) as a colorless solid.
¹H-NMR (CDCl₃) δ: 0.84 (3H, t, J = 7.5 Hz), 1.72-1.83 (2H, m), 2.37 (3H, s), 4.05-4.10 (2H, m), 4.51 (1H, br), 4.74 (2H, d, J = 5.1 Hz), 6.15 (1H, s), 6.87 (1H, d, J = 5.4 Hz), 7.24-7.45 (5H, m), 7.76 (1H, d, J = 5.4 Hz).
LC/MS: 280 [M+H]⁺.

### Example 3

### N-benzyl-1-butyl-2-methyl-1H-pyrrolo[2,3-c]pyridine-7-amine

Using 1-butyl-7-chloro-2-methyl-1H-pyrrolo[2,3-c]pyridine (442 mg) obtained in Reference Example 5 and benzylamine (1.0 mL), reactions under conditions similar to those of Example 1 were carried out to give the title compound (192 mg) as a colorless solid.
¹H-NMR (CDCl₃) δ: 0.83 (3H, t, J = 7.2 Hz), 1.15-1.31 (2H, m), 1.61-1.80 (2H, m), 2.37 (3H, s), 4.06-4.14 (2H, m), 4.52 (1H, br), 4.75 (2H, d, J = 7.5 Hz), 6.15 (1H, s), 6.87 (1H, d, J = 5.4 Hz), 7.25-7.46 (5H, m), 7.77 (1H, d, J = 5.4 Hz).
LC/MS: 294 [M+H]⁺.

### Example 4

### N-benzyl-1-(cyclohexylmethyl)-2-methyl-1H-pyrrolo[2,3-c]pyridine-7-amine

Using 7-chloro-1-(cyclohexylmethyl)-2-methyl-1H-pyrrolo[2,3-c]pyridine (358 mg) obtained in Reference Example 6 and benzylamine (1.0 mL), reactions under conditions similar to those of Example 1 were carried out to give the title compound (182 mg) as a colorless solid.
¹H-NMR (CDCl₃) δ: 0.80-0.97 (2H, m), 0.99-1.20 (3H, m), 1.35-1.44 (2H, m), 1.66-1.72 (3H, m), 1.74-1.90 (1H, m), 2.35 (3H, s), 3.87 (2H, d, J = 6.9 Hz), 4.49 (1H, br), 4.73 (2H, d, J = 5.1 Hz), 6.13 (1H, s), 6.88 (1H, d, J = 5.4 Hz), 7.25-7.45 (5H, m), 7.78 (1H, d, J = 5.4 Hz). LC/MS: 334 [M+H]⁺.

### Example 5

### N-benzyl-1-(cyclopropylmethyl)-2-methyl-1H-pyrrolo[2,3-c]pyridine-7-amine

Using 7-chloro-1-(cyclopropylmethyl)-2-methyl-1H-pyrrolo[2,3-c]pyridine (394 mg) obtained in Reference Example 7 and benzylamine (1.0 mL), reactions under conditions similar to those of Example 1 were carried out to give the title compound (185 mg) as a colorless solid.
¹H-NMR (CDCl₃) δ: 0.20-0.26 (2H, m), 0.45-0.52 (2H, m), 1.09-1.20 (1H, m), 2.36 (3H, s), 4.22 (2H, d, J = 5.4 Hz), 4.73-4.82 (3H, m), 6.15 (1H, s), 6.87 (1H, d, J = 5.7 Hz), 7.24-7.46 (5H, m), 7.76 (1H, d, J = 5.7 Hz). LC/MS: 292 [M+H]⁺.

### Example 6

### N-benzyl-2-methyl-1-(pyridin-2-ylmethyl)-1H-pyrrolo[2,3-c]pyridine-7-amine

Using 7-chloro-2-methyl-1-(pyridin-2-ylmethyl)-1H-pyrrolo[2,3-c]pyridine (476mg) obtained in Reference Example 8 and benzylamine (1.0 mL), reactions under conditions similar to those of Example 1 were carried out to give the title compound (178 mg) as a colorless solid.
¹H-NMR (CDCl₃) δ: 2.44 (3H, m), 4.69 (2H, d, J = 5.4 Hz), 5.52 (2H, s), 6.19 (1H, s), 6.77 (1H, br), 6.84 (1H, d, J = 5.7 Hz), 7.37 (1H, d, J = 7.8 Hz), 7.12-7.33 (6H, m), 7.59-7.65 (1H, m), 7.76 (1H, d, J = 5.7 Hz), 8.27-8.30 (1H, m).
LC/MS: 329 [M+H]⁺.

### Example 7

### N-benzyl-1-(4-methoxybenzyl)-2-methyl-1H-pyrrolo[2,3-c]pyridine-7-amine

Using 7-chloro-1-(4-methoxybenzyl)-2-methyl-1H-pyrrolo[2,3-c]pyridine (406 mg) obtained in Reference Example 9 and benzylamine (1.0 mL), reactions under conditions similar to those of Example 1 were carried out to give the title compound (48 mg) as a colorless solid.
¹H-NMR (CDCl₃) δ: 2.39 (3H, s), 3.76 (3H, s), 4.40-4.48 (3H, m), 5.38 (2H, s), 6.24 (1H, s), 6.69-6.77 (4H, m), 6.89 (1H, d, J = 5.4 Hz), 7.01-7.05 (2H, m), 7.20-7.25 (3H, m), 7.74 (1H, d, J = 5.4 Hz).
LC/MS: 358 [M+H]⁺.

### Example 8

### N-benzyl-1-[2-(benzyloxy)ethyl]-2-methyl-1H-pyrrolo[2,3-c]pyridine-7-amine

Using 1-[2-(benzyloxy)ethyl]-7-chloro-2-methyl-1H-pyrrolo[2,3-c]pyridine (1.10 g) obtained in Reference Example 10 and benzylamine (1.5 mL), reactions under conditions similar to those of Example 1 were carried out to give the title compound (282 mg) as a colorless solid.
¹H-NMR (CDCl₃) δ: 2.33 (3H, s), 3.69 (2H, t, J = 4.5 Hz), 4.30 (2H, s), 4.49 (2H, t, J = 4.5 Hz), 4.57 (2H, d, J = 5.1 Hz), 6.20-6.23 (2H, m), 6.87-6.94 (3H, m), 7.18-7.36 (8H, m), 7.78 (1H, d, J = 5.4 Hz).
LC/MS: 372 [M+H]⁺.

### Example 9

### N,1-dibenzyl-2-methyl-1H-pyrrolo[2,3-c]pyridine-7-amine

Using 1-benzyl-7-chloro-2-methyl-1H-pyrrolo[2,3-c]pyridine (350 mg) obtained in Reference Example 11 and benzylamine (1.0 mL), reactions under conditions similar to those of Example 1 were carried out to give the title compound (122 mg) as a colorless solid.
¹H-NMR (CDCl₃) δ: 2.39 (3H, s), 4.34 (1H, d, J = 5.2 Hz), 4.45 (2H, d, J = 5.2 Hz), 5.46 (2H, s), 6.27 (1H, s), 6.80-7.05 (5H, m), 7.15-7.27 (6H, m), 7.76 (1H, d, J = 5.4 Hz).
LC/MS: 328 [M+H]⁺.

### Example 10

### N-benzyl-1,2-dimethyl-1H-pyrrolo[2,3-c]pyridine-7-amine

Using 7-chloro-1,2-dimethyl-1H-pyrrolo[2,3-c]pyridine (347 mg) obtained in Reference Example 12 and benzylamine (1.0 mL), reactions under conditions similar to those of Example 1 were carried out to give the title compound (50 mg) as a colorless solid.
¹H-NMR (CDCl₃) δ: 2.37 (3H, s), 3.92 (3H, s), 4.60-4.80 (1H, br), 4.74 (2H, s), 6.15 (1H, s), 6.86 (1H, d, J = 5.8 Hz), 7.20-7.45 (5H, m), 7.74 (1H, d, J = 5.8 Hz). LC/MS: 252 [M+H]⁺.

### Example 11

### N-benzyl-1-(2-methoxyethyl)-2-methyl-1H-pyrrolo[2,3-c]pyridine-7-amine

Using 7-chloro-1-(2-methoxyethyl)-2-methyl-1H-pyrrolo[2,3-c]pyridine (230 mg) obtained in Reference Example 13 and benzylamine (1.0 mL), reactions under conditions similar to those of Example 1 were carried out to give the title compound (170 mg) as a colorless solid.
¹H-NMR (CDCl₃) δ: 2.35 (3H, s), 3.11 (3H, s), 3.65 (2H, t, J = 4.4 Hz), 4.47 (2H, t, J = 4.4 Hz), 4.70 (2H, d, J = 5.0 Hz), 6.18 (1H, s), 6.30 (1H, br), 6.85 (1H, d, J = 5.8 Hz), 7.20-7.50 (5H, m), 7.76 (1H, d, J = 5.8 Hz). LC/MS: 296 [M+H]⁺.

### Example 12

### N-benzyl-2,3-dimethyl-1H-pyrrolo[2,3-c]pyridine-7-amine

Using 7-chloro-2,3-dimethyl-1H-pyrrolo[2,3-c]pyridine (200 mg) obtained in Reference Example 2 and benzylamine (1.0 mL), reactions under conditions similar to those of Example 1 were carried out to give the title compound (154 mg) as a colorless solid.
¹H-NMR (CDCl₃) δ: 2.17 (3H, s), 2.29 (3H, s), 4.51 (1H, br), 4.67 (2H, d, J = 5.2 Hz), 6.85 (1H, d, J = 5.2 Hz), 7.20-7.40 (5H, m), 7.78 (1H, d, J = 5.2 Hz), 8.30 (1H, br).
LC/MS: 252 [M+H]⁺.

### Example 13

### 2,3-dimethyl-N-(2-methylbenzyl)-1H-pyrrolo[2,3-c]pyridine-7-amine

Using 7-chloro-2,3-dimethyl-1H-pyrrolo[2,3-c]pyridine (262 mg) obtained in Reference Example 2 and 2-methylbenzylamine (0.45 mL), reactions under conditions similar to those of Example 1 were carried out to give the title compound (281 mg) as a colorless solid.
¹H-NMR (CDCl₃) δ: 2.16 (3H, s), 2.24 (3H, s), 2.28 (3H, s), 4.37 (1H, br), 4.60 (2H, d, J = 4.8 Hz), 6.82 (1H, d, J = 5.7 Hz), 7.01-7.14 (3H, m), 7.20-7.25 (1H, m), 7.75 (1H, d, J = 5.7 Hz), 8.68 (1H, br).
LC/MS: 266 [M+H]⁺.

### Example 14

### N-(2,6-dimethylbenzyl)-2,3-dimethyl-1H-pyrrolo[2,3-c]pyridine-7-amine

A mixture of 7-chloro-2,3-dimethyl-1H-pyrrolo[2,3-c]pyridine (246 mg) obtained in Reference Example 2 and 2,6-dimethylbenzylamine (467 mg) was stirred at 150°C for 1 hr using a microwave focused chemical synthetic reaction apparatus manufactured by CEM. A saturated aqueous sodium hydrogen carbonate solution was added to the reaction mixture, and the mixture was extracted with ethyl acetate. The extract was washed successively with a saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=1:1) to give the title compound as a colorless solid (yield 231 mg, yield 84%).
¹H-NMR (CDCl₃) δ: 2.17 (3H, s), 2.32 (3H, s), 2.34 (6H, s), 3.91 (1H, br), 4.66 (2H, d, J = 4.2 Hz), 6.83 (1H, d, J = 5.7 Hz), 6.98-7.01 (2H, m), 7.05-7.10 (1H, m), 7.80 (1H, d, J = 5.7 Hz), 8.19 (1H, br).
LC/MS: 280 [M+H]⁺.

### Example 15

### 2-[7-(benzylamino)-2-methyl-1H-pyrrolo[2,3-c]pyridin-1-yl]ethanol

N-Benzyl-1-[2-(benzyloxy)ethyl]-2-methyl-1H-pyrrolo[2,3-c]pyridine-7-amine (421 mg) obtained in Example 8 was dissolved in methanol (2 mL), and the mixture was adjusted to pH 2-3 with a 4N hydrogen chloride-ethyl acetate solution. A 10% palladium carbon 50% water-containing product (200 mg) was added and the mixture was stirred overnight under a hydrogen atmosphere. The reaction mixture was filtered through hyflo super-cel (trade name: manufactured by Celite Co.), and the filtrate was concentrated under reduced pressure. A 6% aqueous sodium hydrogen carbonate solution was added to the residue and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=1:1) to give the title compound as a colorless solid (yield 86 mg, yield 27%). ¹H-NMR (CDCl₃) δ: 1.65 (1H, br), 2.35 (3H, s), 3.92 (2H, t, J = 4.5 Hz), 4.43 (2H, t, J = 4.5 Hz), 4.65 (2H, d, J = 4.5 Hz), 6.12 (1H, br), 6.17 (1H, s), 6.81 (1H, d, J = 5.4 Hz), 7.21-7.41 (5H, m), 7.66 (1H, d, J = 5.4 Hz). LC/MS: 282 [M+H]⁺.

### Example 16

### N-(2,3-dihydro-1H-inden-1-yl)-2,3-dimethyl-1H-pyrrolo[2,3-c]pyridine-7-amine

Using 7-chloro-2,3-dimethyl-1H-pyrrolo[2,3-c]pyridine (217 mg) obtained in Reference Example 2 and 2,3-dihydro-1H-indene-1-amine (0.5 mL), reactions under conditions similar to those of Example 1 were carried out to give the title compound (153 mg) as a colorless solid.
¹H-NMR (CDCl₃) δ: 1.62-1.92 (1H, m), 2.17 (3H, s), 2.32 (3H, s), 2.58-2.69 (1H, m), 2.78-2.98 (2H, m), 4.44 (1H, br), 5.72-5.79 (1H, m), 6.89 (1H, d, J = 5.9 Hz), 7.06-7.11 (1H, m), 7.15-7.28 (3H, m), 7.78 (1H, d, J = 5.9 Hz), 8.30 (1H, br).
LC/MS: 278 [M+H]⁺.

### Example 17

### 2,3-dimethyl-N-(1,2,3,4-tetrahydronaphthalen-1-yl)-1H-pyrrolo[2,3-c]pyridine-7-amine

Using 7-chloro-2,3-dimethyl-1H-pyrrolo[2,3-c]pyridine (254 mg) obtained in Reference Example 2 and 1,2,3,4-tetrahydronaphthalene-1-amine (0.7 mL), reactions under conditions similar to those of Example 1 were carried out to give the title compound (139 mg) as a colorless solid.
¹H-NMR (CDCl₃) δ: 1.76-1.86 (2H, m), 1.95-2.06 (2H, m), 2.17 (3H, s), 2.32 (3H, s), 2.72-2.76 (2H, m), 4.35 (1H, br), 5.43-5.49 (1H, m), 6.81 (1H, d, J = 5.9 Hz), 7.03-7.16 (3H, m), 7.34-7.37 (1H, m), 7.77 (1H, d, J = 5.9 Hz), 8.18 (1H, br).
LC/MS: 292 [M+H]⁺.

### Example 18

### 2,3-dimethyl-N-[2-(trifluoromethyl)benzyl]-1H-pyrrolo[2,3-c]pyridine-7-amine

Using 7-chloro-2,3-dimethyl-1H-pyrrolo[2,3-c]pyridine (267 mg) obtained in Reference Example 2 and 2-(trifluoromethyl)benzylamine (0.52 mL), reactions under conditions similar to those of Example 1 were carried out to give the title compound (402 mg) as a colorless solid.
¹H-NMR (CDCl₃) δ: 2.15 (3H, s), 2.30 (3H, s), 4.52 (1H, br t, J = 5.1 Hz), 4.90 (2H, d, J = 5.1 Hz), 6.83 (1H, d, J = 5.7 Hz), 7.26-7.37 (1H, m), 7.58-7.60 (2H, m), 7.75 (1H, d, J = 5.7 Hz), 8.26 (1H, br s).

### Example 19

### N-(2,4-dimethylbenzyl)-2,3-dimethyl-1H-pyrrolo[2,3-c]pyridine-7-amine

Using 7-chloro-2,3-dimethyl-1H-pyrrolo[2,3-c]pyridine (268 mg) obtained in Reference Example 2 and 2,4-dimethylbenzylamine (509 mg) obtained in Reference Example 64, reactions under conditions similar to those of Example 1 were carried out to give the title compound (273 mg) as a colorless solid.
¹H-NMR (CDCl₃) δ: 2.17 (3H, s), 2.21 (3H, s), 2.25 (3H, s), 2.28 (3H, s), 4.31 (1H, br t, J = 5.1 Hz), 4.56 (2H, d, J = 5.1 Hz), 6.81-6.85 (2H, m), 6.89 (1H, s), 7.09 (1H, d, J = 7.5 Hz), 7.75 (1H, d, J = 5.7 Hz), 8.68 (1H, br s).

### Example 20

### N-(4-fluoro-2-methylbenzyl)-2,3-dimethyl-1H-pyrrolo[2,3-c]pyridine-7-amine

Using 7-chloro-2,3-dimethyl-1H-pyrrolo[2,3-c]pyridine (305 mg) obtained in Reference Example 2 and 4-fluoro-2-methylbenzylamine (564 mg) obtained in Reference Example 65, reactions under conditions similar to those of Example 1 were carried out to give the title compound (229 mg) as a colorless solid.
¹H-NMR (CDCl₃) δ: 2.17 (3H, s), 2.18 (3H, s), 2.29 (3H, s), 4.45 (1H, br s), 4.52-5.54 (2H, m), 6.62-6.75 (2H, m), 6.83 (1H, d, J = 5.7 Hz), 7.08-7.12 (1H, m), 7.72 (1H, d, J = 5.7 Hz), 9.01 (1H, br s).

### Example 21

### N-benzyl-N,2,3-trimethyl-1H-pyrrolo[2,3-c]pyridine-7-amine

Using 7-chloro-2,3-dimethyl-1H-pyrrolo[2,3-c]pyridine (282 mg) obtained in Reference Example 2 and N-methylbenzylamine (0.51 mL), reactions under conditions similar to those of Example 1 were carried out to give the title compound (325 mg) as a colorless solid.
¹H-NMR (CDCl₃) δ: 2.16 (3H, s), 2.24 (3H, s), 3.12 (3H, s), 4.69 (2H, s), 6.93-6.95 (1H, m), 7.26-7.46 (5H, m), 7.64 (1H, br s), 7.88 (1H, d, J = 5.4 Hz).

### Example 22

### N-benzyl-1,2,3-trimethyl-1H-pyrrolo[2,3-c]pyridine-7-amine

Using 7-chloro-1,2,3-trimethyl-1H-pyrrolo[2,3-c]pyridine (262 mg) obtained in Reference Example 33 and benzylamine (0.44 mL), reactions under conditions similar to those of Example 1 were carried out to give the title compound (198 mg) as a colorless solid.
¹H-NMR (CDCl₃) δ: 2.17 (3H, s), 2.29 (3H, s), 3.90 (3H, s), 4.67 (1H, br d, J = 5.4 Hz), 4.74 (2H, d, J = 5.4 Hz), 6.84 (1H, d, J = 5.7 Hz), 7.26-7.37 (3H, m), 7.42-7.57 (2H, m), 7.75 (1H, d, J = 5.7 Hz).

### Example 23

### N-benzyl-1-ethyl-2,3-dimethyl-1H-pyrrolo[2,3-c]pyridine-7-amine hydrochloride

Using 7-chloro-1-ethyl-2,3-dimethyl-1H-pyrrolo[2,3-c]pyridine (262 m) obtained in Reference Example 34 and benzylamine (0.44 mL), reactions under conditions similar to those of Example 1 were carried out to give a free base of the title compound (181 mg) as an oil. The obtained free base was dissolved in methanol (5 mL), 10% hydrogen chloride methanol solution (2 mL) was added, and the mixture was concentrated under reduced pressure. The residue was crystallized from ethanol to give the title compound as a white solid (109 mg).
¹H-NMR (DMSO-d₆) δ: 1.24 (3H, t, J = 7.4 Hz), 2.17 (3H, s), 2.42 (3H, s), 4.53 (2H, q, J = 7.4 Hz), 4.89 (2H, d, J = 6.2 Hz), 7.10 (1H, d, J = 7.0 Hz), 7.28-7.46 (5H, m), 8.00-8.20 (1H, m), 12.62 (1H, br s), 1H not detected.

### Example 24

### 1-ethyl-N-(4-fluoro-2-methylbenzyl)-2,3-dimethyl-1H-pyrrolo[2,3-c]pyridine-7-amine

Using 7-chloro-1-ethyl-2,3-dimethyl-1H-pyrrolo[2,3-c]pyridine (234 mg) obtained in Reference Example 34 and 4-fluoro-2-methylbenzylamine (549 mg) obtained in Reference Example 65, reactions under conditions similar to those of Example 1 were carried out to give the title compound (262 mg) as a colorless solid.
¹H-NMR (CDCl₃) δ: 1.29 (3H, t, J = 7.2 Hz), 2.18 (3H, s), 2.30 (3H, s), 2.41 (3H, s), 4.16 (2H, q, J = 7.2 Hz), 4.35 (1H, br t, J = 5.1 Hz), 4.68 (2H, d, J = 5.1 Hz), 6.82-6.90 (3H, m), 7.29-7.34 (1H, m), 7.78 (1H, d, J = 5.4 Hz).

### Example 25

### N-benzyl-2,3-dimethyl-1-propyl-1H-pyrrolo[2,3-c]pyridine-7-amine

Using 7-chloro-2,3-dimethyl-1-propyl-1H-pyrrolo[2,3-c]pyridine (198 mg) obtained in Reference Example 35 and benzylamine (1.0 mL), reactions under conditions similar to those of Example 1 were carried out to give the title compound (87 mg) as a pale-yellow crystal.
¹H-NMR (CDCl₃) δ: 0.83 (3H, t, J = 7.5 Hz), 1.67-1.80 (2H, m), 2.17 (3H, s), 2.29 (3H, s), 4.04-4.09 (2H, m), 4.50 (1H, br), 4.74 (2H, d, J = 5.4 Hz), 6.85 (1H, d, J = 5.7 Hz), 7.26-7.37 (3H, m), 7.41-7.45 (2H, m), 7.77 (1H, d, J = 5.7 Hz).

### Example 26

### N-(4-fluoro-2-methylbenzyl)-2,3-dimethyl-1-propyl-1H-pyrrolo[2,3-c]pyridine-7-amine

Using 7-chloro-2,3-dimethyl-1-propyl-1H-pyrrolo[2,3-c]pyridine (223 mg) obtained in Reference Example 35 and 4-fluoro-2-methylbenzylamine (309 mg) obtained in Reference Example 65, reactions under conditions similar to those of Example 1 were carried out to give the title compound (110 mg) as a colorless solid.
¹H-NMR (CDCl₃) δ: 0.78 (3H, t, J = 7.8 Hz), 1.66-1.74 (2H, m), 2.17 (3H, s), 2.29 (3H, s), 2.41 (3H, s), 3.99-4.04 (2H, m), 4.28 (1H, br t, J = 5.1 Hz), 4.65 (2H, d, J = 5.1 Hz), 6.85-6.94 (3H, m), 7.29-7.34 (1H, m), 7.78 (1H, d, J = 5.4 Hz).

### Example 27

### N-(4-fluoro-2-methylbenzyl)-1-isobutyl-2,3-dimethyl-1H-pyrrolo[2,3-c]pyridine-7-amine

Using 7-chloro-1-isobutyl-2,3-dimethyl-1H-pyrrolo[2,3-c]pyridine (435 mg) obtained in Reference Example 36 and 4-fluoro-2-methylbenzylamine (627 mg) obtained in Reference Example 65, reactions under conditions similar to those of Example 1 were carried out to give the title compound (404 mg) as a colorless solid.
¹H-NMR (CDCl₃) δ: 0.74 (6H, d, J = 6.9 Hz), 2.01-2.10 (1H, m), 2.17 (3H, s), 2.27 (3H, s), 2.41 (3H, s), 3.83 (2H, d, J = 7.5 Hz), 4.24 (1H, br t, J = 4.5 Hz), 4.64 (2H, d, J = 4.5 Hz), 6.83-6.94 (3H, m), 7.29-7.34 (1H, m), 7.78 (1H, d, J = 5.7 Hz).

### Example 28

### 2-methyl-N-(2-methylbenzyl)-1-propyl-1H-pyrrolo[2,3-c]pyridine-7-amine

Using 7-chloro-2-methyl-1-propyl-1H-pyrrolo[2,3-c]pyridine (502 mg) obtained in Reference Example 4 and 2-methylbenzylamine (0.75 mL), reactions under conditions similar to those of Example 1 were carried out to give the title compound (455 mg) as a colorless solid.
¹H-NMR (CDCl₃) δ: 0.78 (3H, t, J = 7.5 Hz), 1.68-1.80 (2H, m), 2.36 (3H, d, J = 0.9 Hz), 2.43 (3H, s), 4.00-4.05 (2H, m), 4.33 (1H, br t, J = 4.8 Hz), 4.70 (2H, d, J = 4.8 Hz), 6.14 (1H, d, J = 0.9 Hz), 6.87 (1H, d, J = 5.4 Hz), 7.17-7.23 (3H, m), 7.35-7.38 (1H, m), 7.77 (1H, d, J = 5.4 Hz).

### Example 29

### N-(2,6-dimethylbenzyl)-2-methyl-1-propyl-1H-pyrrolo[2,3-c]pyridine-7-amine

Using 7-chloro-2-methyl-1-propyl-1H-pyrrolo[2,3-c]pyridine (471 mg) obtained in Reference Example 4 and 2,6-dimethylbenzylamine (737 mg), reactions under conditions similar to those of Example 1 were carried out to give the title compound (232 mg) as a colorless solid.
¹H-NMR (CDCl₃) δ: 0.67 (3H, t, J = 7.5 Hz), 1.62-1.73 (2H, m), 2.34 (3H, d, J = 0.9 Hz), 2.43 (6H, s), 3.90-3.95 (2H, m), 4.08 (1H, br t, J = 4.5 Hz), 4.68 (2H, d, J = 4.5 Hz), 6.13 (1H, d, J = 0.9 Hz), 6.86 (1H, d, J = 5.4 Hz), 7.05-7.15 (3H, m), 7.80 (1H, d, J = 5.4 Hz).

### Example 30

### N-(4-fluoro-2-methylbenzyl)-2-methyl-1-propyl-1H-pyrrolo[2,3-c]pyridine-7-amine

Using 7-chloro-2-methyl-1-propyl-1H-pyrrolo[2,3-c]pyridine (496 mg) obtained in Reference Example 4 and 4-fluoro-2-methylbenzylamine (862 mg) obtained in Reference Example 65, reactions under conditions similar to those of Example 1 were carried out to give the title compound (228 mg) as a white solid.
¹H-NMR (CDCl₃) δ: 0.79 (3H, t, J = 7.5 Hz), 1.67-1.80 (2H, m), 2.37 (3H, s), 2.42 (3H, s), 4.01-4.06 (2H, m) 4.29 (1H, br t, J = 5.1 Hz), 4.66 (2H, d, J = 5.1 Hz), 6.15 (1H, s), 6.84-6.95 (3H, m), 7.31-7.35 (1H, m), 7.77 (1H, d, J = 5.4 Hz).

### Example 31

### N-(4-fluoro-2-methylbenzyl)-1-isobutyl-2-methyl-1H-pyrrolo[2,3-c]pyridine-7-amine

Using 7-chloro-1-isobutyl-2-methyl-1H-pyrrolo[2,3-c]pyridine (600 mg) obtained in Reference Example 37 and 4-fluoro-2-methylbenzylamine (915 mg) obtained in Reference Example 65, reactions under conditions similar to those of Example 1 were carried out to give the title compound (770 mg) as a colorless solid.
¹H-NMR (CDCl₃) δ: 0.76 (6H, d, J = 6.9 Hz), 2.04-2.18 (1H, m), 2.36 (3H, d, J = 0.9 Hz), 2.42 (3H, s), 3.84 (2H, d, J = 7.5 Hz), 4.25 (1H, br t, J = 4.8 Hz), 4.65 (2H, d, J = 4.8 Hz), 6.16 (1H, d, J = 0.9 Hz), 6.85-6.96 (3H, m), 7.31-7.35 (1H, m), 7.78 (1H, d, J = 5.4 Hz).

### Example 32

### 2-methyl-N-[(1R)-1-phenylethyl]-1-propyl-1H-pyrrolo[2,3-c]pyridine-7-amine

Using 7-chloro-2-methyl-1-propyl-1H-pyrrolo[2,3-c]pyridine (507 mg) obtained in Reference Example 4 and (R)-1-phenylethylamine (0.78 mL), reactions under conditions similar to those of Example 1 were carried out to give the title compound (464 mg) as a colorless solid.
¹H-NMR (CDCl₃) δ: 0.90 (3H, t, J = 7.2 Hz), 1.64 (3H, d, J = 6.6 Hz), 1.72-1.86 (2H, m), 2.37 (3H, d, J = 0.9 Hz), 4.09-4.17 (2H, m), 4.57 (1H, br d, J = 5.7 Hz), 5.46 (1H, dq, J = 5.7, 6.6 Hz), 6.12 (1H, d, J = 0.9 Hz), 6.81 (1H, d, J = 5.4 Hz), 7.21-7.26 (1H, m), 7.30-7.36 (2H, m), 7.43-7.47 (2H, m), 7.70 (1H, d, J = 5.4 Hz).

### Example 33

### 2-methyl-N-[(1S)-1-phenylethyl]-1-propyl-1H-pyrrolo[2,3-c]pyridine-7-amine

Using 7-chloro-2-methyl-1-propyl-1H-pyrrolo[2,3-c]pyridine (518 mg) obtained in Reference Example 4 and (S)-1-phenylethylamine (0.80 mL), reactions under conditions similar to those of Example 1 were carried out to give the title compound (402 mg) as a colorless solid.
¹H-NMR (CDCl₃) δ: 0.91 (3H, t, J = 7.5 Hz), 1.64 (3H, d, J = 6.6 Hz), 1.73-1.86 (2H, m), 2.37 (3H, d, J = 0.9 Hz), 4.10-4.15 (2H, m), 4.58 (1H, br d, J = 6.0 Hz), 5.47 (1H, dq, J = 6.0, 6.6 Hz), 6.13 (1H, d, J = 0.9 Hz), 6.82 (1H, d, J = 5.4 Hz), 7.22-7.28 (1H, m), 7.31-7.37 (2H, m), 7.44-7.48 (2H, m), 7.72 (1H, d, J = 5.4 Hz).

### Example 34

### N-benzyl-2,3,4-trimethyl-1H-pyrrolo[2,3-c]pyridine-7-amine

7-Chloro-2,3,4-trimethyl-1H-pyrrolo[2,3-c]pyridine (389 g) obtained in Reference Example 25 was dissolved in benzylamine (1 mL), and the mixture was heated at 180°C for 75 min using a microwave focused chemical synthetic reaction apparatus manufactured by CEM. The reaction mixture was cooled to room temperature, water was added and the mixture was extracted with tetrahydrofuran. The extract was washed with saturated brine, dried over anhydrous sodium sulfate, and concentrated under reduced pressure. The residue was purified by basic silica gel column chromatography (eluent: hexane-ethyl acetate=1:1→1:2). The obtained a yellow oil was crystallized from a mixed solvent of diisopropyl ether and ethyl acetate to give the title compound as colorless crystals (yield 272 mg, yield 51%). ¹H-NMR (DMSO-d₆) δ: 2.26-2.27 (6H, m), 2.38 (3H, s), 4.61 (2H, d, J = 5.7 Hz), 6.15 (1H, t, J = 7.2 Hz), 7.17-7.36 (5H, m), 10.56 (1H, br s).

### Example 35

### 7-(benzylamino)-2-methyl-1-propyl-1H-pyrrolo[2,3-c]pyridine-3-carbonitrile

Using 7-(dibenzylamino)-2-methyl-1-propyl-1H-pyrrolo[2,3-c]pyridine-3-carbonitrile (134 mg) obtained in Reference Example 49, reactions under conditions similar to those of Reference Example 48 were carried out, recrystallized with ethyl acetate and hexane and gave the title compound (24 mg) as colorless crystals. ¹H-NMR (CDCl₃) δ: 0.87 (3H, t, J = 7.5 Hz), 1.76-1.89 (2H, m), 2.55 (3H, s), 4.08-4.14 (2H, m), 4.55 (1H, br), 4.74 (2H, d, J = 4.8 Hz), 7.01 (1H, d, J = 5.7 Hz), 7.26-7.44 (5H, m), 7.92 (1H, d, J = 5.7 Hz).

### Example 36

### N-benzyl-3-ethyl-2-methyl-1H-pyrrolo[2,3-c]pyridine-7-amine

To a solution (2 mL) of N,N-dibenzyl-3-ethyl-2-methyl-1H-pyrrolo[2,3-c]pyridine-7-amine (162 mg) obtained in Reference Example 45 in methanol was added a 10% palladium carbon 50% water-containing product (150 mg), and the mixture was stirred overnight under a hydrogen atmosphere. The reaction mixture was filtered through hyflo super-cel (trade name: manufactured by Celite Co.), and the filtrate was concentrated under reduced pressure. The residue was purified by basic silica gel column chromatography (eluent: hexane-ethyl acetate=9:1→3:1). Recrystallization of the obtained pale-yellow powder from a mixed solvent of diisopropyl ether and ethyl acetate gave the title compound as colorless crystals (yield 42 mg, yield 34%).
¹H-NMR (CDCl₃) δ: 1.20 (3H, t, J = 7.6 Hz), 2.30 (3H, s), 2.64 (2H, q, J = 7.6 Hz), 4.52 (1H, br), 4.68 (2H, d, J = 7.8 Hz), 6.89 (1H, d, J = 5.6 Hz), 7.20-7.31 (5H, m), 7.77 (1H, d, J = 5.6 Hz), 8.44 (1H, br s).

### Example 37

### 1-[7-(benzylamino)-2-methyl-1H-pyrrolo[2,3-c]pyridin-3-yl]ethanone

To a solution (2 mL) of 1-[7-(dibenzylamino)-2-methyl-1H-pyrrolo[2,3-c]pyridin-3-yl]ethanone (229 mg) obtained in Reference Example 41 in methanol was added a 10% palladium carbon 50% water-containing product (150 mg), the mixture was stirred overnight under a hydrogen atmosphere. The reaction mixture was filtered through hyflo super-cel (trade name: manufactured by Celite Co.), and the filtrate was concentrated under reduced pressure. The residue was purified by silica gel column chromatography (eluent: hexane-ethyl acetate=1:1→1:4). The obtained colorless oil was crystallized from a mixed solvent of ethyl acetate and hexane, and recrystallized from ethyl acetate to give the title compound as colorless crystals (yield 59 mg, yield 34%).
¹H-NMR (DMSO-d₆) δ: 2.49-2.51 (3H, m), 2.65 (3H, s), 4.68 (2H, d, J = 5.4 Hz), 6.55 (1H, t, J = 5.4 Hz), 7.14 (1H, d, J = 5.6 Hz), 7.25-7.41 (5H, m), 7.68 (1H, d, J = 5.6 Hz), 11.69 (1H, br s).

### Example 38

### N-benzyl-5-chloro-2,3-dimethyl-1H-pyrrolo[2,3-c]pyridine-7-amine

N,N-dibenzyl-5-chloro-2,3-dimethyl-1H-pyrrolo[2,3-c]pyridine-7-amine (368 mg) obtained in Reference Example 28 was dissolved in nitromethane (1 mL) and 1,2-dichloroethane (1 mL), and aluminum (III) chloride (131 mg) and dichloromethyl methyl ether (113 mg) were added at 0°C. The addition was repeated twice in the same manner at 30 min intervals. Furthermore, the mixture was stirred at the same temperature for 30 min, weakly basified with a 8N aqueous sodium hydroxide solution, and extracted with ethyl acetate. The extract was washed with saturated brine, dried over anhydrous sodium sulfate, and concentrated under reduced pressure. The residue was purified by silica gel column chromatography (eluent: hexane-ethyl acetate=9:1→3:1). The obtained yellow oil was crystallized from a mixed solvent of diisopropyl ether and hexane to give the title compound as a pale-yellow powder (yield 97 mg, yield 35%).
¹H-NMR (CDCl₃) δ: 2.11 (3H, s), 2.28 (3H, s), 4.45 (1H, br), 4.70 (2H, d, J = 3.9 Hz), 6.82 (1H, s), 7.26-7.40 (5H, m), 7.79 (1H, br s).

### Example 39

### 7-(benzylamino)-2,3-dimethyl-1H-pyrrolo[2,3-c]pyridine-5-carbonitrile

7-(Dibenzylamino)-2,3-dimethyl-1H-pyrrolo[2,3-c]pyridine-5-carbonitrile (183 mg) obtained in Reference Example 30 was dissolved in nitromethane (1 mL) and 1,2-dichloroethane (1 mL), and aluminum (III) chloride (67 mg) and dichloromethyl methyl ether (57 mg) were added at 0°C. After stirring at the same temperature for 1 hr, the same amount of aluminum (III) chloride and dichloromethyl methyl ether was added, and the mixture was further stirred for 1 hr. The mixture was weakly basified with a saturated aqueous sodium hydrogen carbonate solution, and the mixture was extracted with ethyl acetate. The extract was washed with saturated brine, dried over anhydrous sodium sulfate, and concentrated under reduced pressure. The residue was purified by basic silica gel column chromatography (eluent: hexane-ethyl acetate=9:1→2:1). The obtained yellow powder was washed with a mixed solvent of ethyl acetate and hexane to give the title compound as a colorless powder (yield 66 mg, yield 48%).
¹H-NMR (DMSO-d₆) δ: 2.12 (3H, s), 2.33 (3H, s), 4.64 (2H, d, J = 5.7 Hz), 6.84 (1H, t, J = 5.7 Hz), 7.24-7.41 (6H, m), 11.15 (1H, br s).

### Example 40

### 7-(benzylamino)-2,3-dimethyl-1H-pyrrolo[2,3-c]pyridine-5-carboxamide

7-(Dibenzylamino)-2,3-dimethyl-1H-pyrrolo[2,3-c]pyridine-5-carboxamide (262 mg) obtained in Reference Example 47 was dissolved in tetrahydrofuran (3 mL) and methanol (1 mL). The mixture was adjusted to pH 2-3 using a 1M hydrogen chloride-diethyl ether solution, a 10% palladium carbon 50% water-containing product (200 mg) was added, and the mixture was stirred under a hydrogen atmosphere for 1.5 hr. The reaction mixture was filtered through hyflo super-cel (trade name: manufactured by Celite Co.), washed with methanol, and the filtrate was concentrated under reduced pressure. A 6% aqueous sodium hydrogen carbonate solution was added to the residue and the mixture was extracted with tetrahydrofuran. The extract was washed with saturated brine, dried over anhydrous sodium sulfate, and concentrated under reduced pressure. The residue was purified by basic silica gel column chromatography (eluent: ethyl acetate). The obtained colorless powder was washed with diisopropyl ether to give the title compound as a colorless powder (yield 33 mg, yield 16%). ¹H-NMR (DMSO-d₆) δ: 2.13 (3H, s), 2.32 (3H, s), 4.74 (2H, d, J = 5.7 Hz), 6.62 (1H, t, J = 5.7 Hz), 7.12 (1H, br), 7.21-7.43 (5H, m), 7.48 (1H, s), 7.72 (1H, br), 10.87 (1H, br s).

### Example 41

### 7-(benzylamino)-2-methyl-1H-pyrrolo[2,3-c]pyridine-3-carbaldehyde

N,N-Dibenzyl-2-methyl-1H-pyrrolo[2,3-c]pyridine-7-amine (100 mg) obtained in Reference Example 26 was dissolved in nitromethane (2 mL) and 1,2-dichloroethane (2 mL), dichloromethyl methyl ether (178 mg) was added at 0°C, and aluminum (III) chloride (145 mg) was added by small portions. The mixture was stirred at 0°C for 2 hr, and the reaction mixture was added by small portions to a 6% aqueous sodium hydrogen carbonate solution cooled to 0°C. The mixture was extracted with ethyl acetate, and the extract was washed with saturated brine, dried over anhydrous sodium sulfate, and concentrated under reduced pressure. The residue was purified by silica gel column chromatography (eluent: hexane-ethyl acetate=2:3→1:4) to give the title compound as a yellow powder (46 mg, yield 56%).
¹H-NMR (CDCl₃) δ: 2.68 (3H, s), 4.67 (2H, d, J = 5.2 Hz), 6.58 (1H, t, J = 5.2 Hz), 7.19-7.42 (6H, m), 7.71 (1H, d, J = 5.8 Hz), 10.0 (1H, s), 11.8 (1H, br s).

### Example 42

### N-(4-fluoro-2-methylbenzyl)-2,3-dimethyl-1-propyl-5-(trifluoromethyl)-1H-pyrrolo[2,3-c]pyridine-7-amine

To a solution (5 mL) of 2,3-dimethyl-1-propyl-5-(trifluoromethyl)-1H-pyrrolo[2,3-c]pyridine-7-amine (239 mg) obtained in Reference Example 50 in toluene were added 4-fluoro-2-methylbenzaldehyde (144 mg) obtained in Reference Example 56 and anhydrous magnesium sulfate (376 mg), and the mixture was heated under reflux for 2 hr. The reaction mixture was returned to room temperature, toluene was evaporated under reduced pressure, and the residue was suspended in tetrahydrofuran (2 mL) and methanol (2 mL). Sodium borohydride (78 mg) was added at 0°C and the mixture was stirred at room temperature for 4 hr. The reaction mixture was treated with acetic acid, a 6% aqueous sodium hydrogen carbonate solution was added and the mixture was extracted with ethyl acetate. The extract was washed with saturated brine, dried over anhydrous sodium sulfate, and concentrated under reduced pressure. Recrystallization of the residue from hexane gave the title compound as colorless crystals (yield 68 mg, yield 17%).
¹H-NMR (CDCl₃) δ: 0.82 (3H, t, J = 7.5 Hz), 1.63-1.78 (2H, m), 2.18 (3H, s), 2.30 (3H, s), 2.42 (3H, s), 4.02-4.07 (2H, m), 4.49 (1H, br), 4.70 (2H, d, J = 4.8 Hz), 6.82-6.93 (2H, m), 7.24 (1H, s), 7.34-7.39 (1H, m).

### Example 43

### N-(4-fluoro-2-methylbenzyl)-2,3-dimethyl-5-(trifluoromethyl)-1H-pyrrolo[2,3-c]pyridine-7-amine

Using 2,3-dimethyl-5-(trifluoromethyl)-1H-pyrrolo[2,3-c]pyridine-7-amine (657 mg) obtained in Reference Example 51, reactions under conditions similar to those of Example 42 were carried out to give the title compound (56 mg) as colorless crystals.
¹H-NMR (CDCl₃) δ: 2.18 (3H, s), 2.33 (3H, s), 2.34 (3H, s), 4.30 (1H, br), 4.66 (2H, d, J = 4.8 Hz), 6.77-6.88 (2H, m), 7.24-7.32 (2H, m), 7.95 (1H, br s).

### Example 44

### N-(4-fluoro-2-methylbenzyl)-2-methyl-1,3-dipropyl-1H-pyrrolo[2,3-c]pyridine-7-amine

Using 2-methyl-1,3-dipropyl-1H-pyrrolo[2,3-c]pyridine-7-amine (145 mg) obtained in Reference Example 52, reactions under conditions similar to those of Example 42 were carried out to give the title compound (84 mg) as a colorless oil.
¹H-NMR (CDCl₃) δ: 0.78 (3H, t, J = 7.5 Hz), 0.91 (3H, t, J = 7.2 Hz), 1.51-1.75 (4H, m), 2.29 (3H, s), 2.42 (3H, s), 2.60 (2H, t, J = 7.2 Hz), 4.00-4.06 (2H, m), 4.28 (1H, br), 4.65 (2H, d, J = 4.8 Hz), 6.83-6.95 (3H, m), 7.30-7.35 (1H, m), 7.77 (1H, d, J = 5.7 Hz).

### Example 45

### N-(4-fluoro-2-methylbenzyl)-2,3-dimethyl-5-(morpholin-4-ylcarbonyl)-1H-pyrrolo[2,3-c]pyridine-7-amine

Using 2,3-dimethyl-5-(morpholin-4-ylcarbonyl)-1H-pyrrolo[2,3-c]pyridine-7-amine (180 mg) obtained in Reference Example 53, reactions under conditions similar to those of Example 42 were carried out to give the title compound (34 mg) as colorless crystals.
¹H-NMR (CDCl₃) δ: 2.11 (3H, s), 2.33 (3H, s), 2.35 (3H, s), 3.20-3.40 (2H, m), 3.40-3.60 (6H, m), 4.57 (2H, d, J = 5.4 Hz), 6.55 (1H, br), 6.91-6.97 (1H, m), 7.05-7.09 (2H, m), 7.22-7.26 (1H, m), 10.87 (1H, s).

### Example 46

### N-(4-fluorobenzyl)-2-methyl-1-propyl-1H-pyrrolo[2,3-c]pyridine-7-amine

Using 2-methyl-1-propyl-1H-pyrrolo[2,3-c]pyridine-7-amine (189 mg) obtained in Reference Example 55 and 4-fluorobenzaldehyde (149 mg), reactions under conditions similar to those of Example 42 were carried out to give the title compound (254 mg) as colorless crystals.
¹H-NMR (CDCl₃) δ: 0.84 (3H, t, J = 7.5 Hz), 1.64-1.84 (2H, m), 2.38 (3H, s), 4.05-4.11 (2H, m), 4.48 (1H, br), 4.71 (2H, d, J = 5.4 Hz), 6.16 (1H, s), 6.89 (1H, d, J = 5.4 Hz), 7.01-7.08 (2H, m), 7.38-7.43 (2H, m), 7.76 (1H, d, J = 5.4 Hz).

### Example 47

### N-(2,6-diethylbenzyl)-2-methyl-1-propyl-1H-pyrrolo[2,3-c]pyridine-7-amine

Using 2-methyl-1-propyl-1H-pyrrolo[2,3-c]pyridine-7-amine (189 mg) obtained in Reference Example 55, and 2,6-diethylbenzaldehyde (195 mg) obtained in Reference Example 57, reactions under conditions similar to those of Example 42 were carried out to give the title compound (155 mg) as colorless crystals.
¹H-NMR (CDCl₃) δ: 0.64 (3H, t, J = 7.5 Hz), 1.21-1.28 (6H, m), 1.60-1.70 (2H, m), 2.34 (3H, s), 2.77 (4H, q, J = 7.2 Hz), 3.88-3.94 (2H, m), 4.09-4.16 (1H, m), 4.69 (2H, d, J = 4.2 Hz), 6.14 (1H, s), 6.87 (1H, d, J = 5.7 Hz), 7.11-7.13 (2H, m), 7.21-7.26 (1H, m), 7.82 (1H, d, J = 5.7 Hz).

### Example 48

### 2-methyl-N-[(3-methyl-2-thienyl)methyl]-1-propyl-1H-pyrrolo[2,3-c]pyridine-7-amine

Using 2-methyl-1-propyl-1H-pyrrolo[2,3-c]pyridine-7-amine (189 mg) obtained in Reference Example 55 and 3-methylthiophene-2-carbaldehyde (151 mg), reactions under conditions similar to those of Example 42 were carried out to give the title compound (155 mg) as colorless crystals.
¹H-NMR (CDCl₃) δ: 0.88 (3H, t, J = 7.5 Hz), 1.75-1.85 (2H, m), 2.29 (3H, s), 2.38 (3H, s), 4.08-4.16 (2H, m), 4.45 (1H, br), 4.83 (2H, d, J = 5.1 Hz), 6.16 (1H, s), 6.85-6.91 (2H, m), 7.14 (1H, d, J = 5.4 Hz), 7.78 (1H, d, J = 5.4 Hz).

### Example 49

### 7-[(4-fluoro-2-methylbenzyl)amino]-N-(2-hydroxyethyl)-2,3-dimethyl-1H-pyrrolo[2,3-c]pyridine-5-carboxamide

To a solution (20 mL) of 7-amino-N-(2-hydroxyethyl)-2,3-dimethyl-1H-pyrrolo[2,3-c]pyridine-5-carboxamide (260 mg) obtained in Reference Example 54 in methanol were added 4-fluoro-2-methylbenzaldehyde (218 mg), anhydrous magnesium sulfate (380 mg) and sodium borocyanotrihydride (147 mg), and the mixture was heated under reflux for 8 hr. The reaction mixture was returned to room temperature, a 6% aqueous sodium hydrogen carbonate solution was added and the mixture was extracted with ethyl acetate. The extract was washed with saturated brine, dried over anhydrous sodium sulfate, and concentrated under reduced pressure. The residue was purified by silica gel column chromatography (eluent: ethyl acetate-methanol=19:1). The obtained yellow oil was crystallized from a mixed solvent of diisopropyl ether and ethyl acetate, and recrystallized from ethyl acetate and ethanol to give the title compound as colorless crystals (yield 158 mg, yield 41%). ¹H-NMR (CDCl₃) δ: 2.13 (3H, s), 2.31 (3H, s), 2.38 (3H, s), 3.34-3.39 (2H, m), 3.52 (2H, q, J = 5.7 Hz), 4.69 (2H, d, J = 5.1 Hz), 4.83 (1H, t, J = 5.1 Hz), 6.48 (1H, t, J = 5.4 Hz), 6.95-7.09 (2H, m), 7.41-7.47 (2H, m), 8.40 (1H, t, J = 5.4 Hz), 10.89 (1H, br s).

### Example 50

### 2-methyl-7-[(2-methylbenzyl)amino]-1-propyl-1H-pyrrolo[2,3-c]pyridine-3-carbaldehyde

Using 2-methyl-N-(2-methylbenzyl)-1-propyl-1H-pyrrolo[2,3-c]pyridine-7-amine (319 mg) obtained in Example 28, reactions under conditions similar to those of Reference Example 48 were carried out to give the title compound (221 mg) as a colorless solid.
¹H-NMR (CDCl₃) δ: 0.82 (3H, t, J = 7.5 Hz), 1.73-1.86 (2H, m), 2.42 (3H, s), 2.66 (3H, s), 4.06-4.11 (2H, m), 4.34 (1H, br t, J = 4.8 Hz), 4.70 (2H, d, J = 4.8 Hz), 7.16-7.24 (3H, m), 7.33-7.36 (1H, m), 7.54 (1H, d, J = 5.4 Hz), 7.97 (1H, d, J = 5.4 Hz), 10.15 (1H, s).

### Example 51

### 7-[(2,6-dimethylbenzyl)amino]-2-methyl-1-propyl-1H-pyrrolo[2,3-c]pyridine-3-carbaldehyde

Using N-(2,6-dimethylbenzyl)-2-methyl-1-propyl-1H-pyrrolo[2,3-c]pyridine-7-amine (139 mg) obtained in Example 29, reactions under conditions similar to those of Reference Example 48 were carried out to give the title compound (73 mg) as a colorless solid.
¹H-NMR (CDCl₃) δ: 0.71 (3H, t, J = 7.5 Hz), 1.69-1.77 (2H, m), 2.43 (6H, s), 2.65 (6H, s), 3.98-4.03 (2H, m), 4.10 (1H, br t, J = 4.2 Hz), 4.70 (2H, d, J = 4.2 Hz), 7.07-7.16 (3H, m), 7.55 (1H, d, J = 5.4 Hz), 8.02 (1H, d, J = 5.4 Hz), 10.18 (1H, s).

### Example 52

### 7-[(4-fluoro-2-methylbenzyl)amino]-2-methyl-1-propyl-1H-pyrrolo[2,3-c]pyridine-3-carbaldehyde

Using N-(4-fluoro-2-methylbenzyl)-2-methyl-1-propyl-1H-pyrrolo[2,3-c]pyridine-7-amine (514 mg) obtained in Example 30, reactions under conditions similar to those of Reference Example 48 were carried out to give the title compound (322 mg) as a pale-yellow solid.
¹H-NMR (CDCl₃) δ: 0.83 (3H, t, J = 7.5 Hz), 1.70-1.83 (2H, m), 2.41 (3H, s), 2.66 (3H, s), 4.06-4.12 (2H, m), 4.30 (1H, br), 4.66 (2H, d, J = 5.1 Hz), 6.85-6.96 (2H, m), 7.29-7.34 (1H, m), 7.56 (1H, d, J = 5.4 Hz), 7.97 (1H, d, J = 5.4 Hz), 10.16 (1H, s).

### Example 53

### 7-[(4-fluorobenzyl)amino]-2-methyl-1-propyl-1H-pyrrolo[2,3-c]pyridine-3-carbaldehyde

Using N-(4-fluorobenzyl)-2-methyl-1-propyl-1H-pyrrolo[2,3-c]pyridine-7-amine (254 mg) obtained in Example 46, reactions under conditions similar to those of Reference Example 48 were carried out to give the title compound (170 mg) as colorless crystals.
¹H-NMR (CDCl₃) δ: 0.89 (3H, t, J = 7.5 Hz), 1.79-1.88 (2H, m), 2.67 (3H, s), 4.11-4.17 (2H, m), 4.49 (1H, br), 4.71 (2H, d, J = 5.1 Hz), 7.02-7.08 (2H, m), 7.37-7.42 (2H, m), 7.57 (1H, d, J = 5.4 Hz), 7.96 (1H, d, J = 5.4 Hz), 10.17 (1H, s).

### Example 54

### 7-[(2,6-diethylbenzyl)amino]-2-methyl-1-propyl-1H-pyrrolo[2,3-c]pyridine-3-carbaldehyde

Using N-(2,6-diethylbenzyl)-2-methyl-1-propyl-1H-pyrrolo[2,3-c]pyridine-7-amine (155 mg) obtained in Example 47, reactions under conditions similar to those of Reference Example 48 were carried out to give the title compound (83 mg) as colorless crystals.
¹H-NMR (CDCl₃) δ: 0.67 (3H, t, J = 7.5 Hz), 1.20-1.27 (6H, m), 1.60-1.80 (2H, m), 2.64 (3H, s), 2.76 (4H, q, J = 7.5 Hz), 3.95-4.00 (2H, m), 4.12 (1H, br), 4.78 (2H, d, J = 4.2 Hz), 7.11-7.14 (2H, m), 7.22-7.26 (1H, m), 7.56 (1H, d, J = 5.4 Hz), 8.02 (1H, d, J = 5.4 Hz), 10.17 (1H, s).

### Example 55

### 7-[(4-fluoro-2-methylbenzyl)amino]-1-isobutyl-2-methyl-1H-pyrrolo[2,3-c]pyridine-3-carbaldehyde

Using N-(4-fluoro-2-methylbenzyl)-1-isobutyl-2-methyl-1H-pyrrolo[2,3-c]pyridine-7-amine (339 mg) obtained in Example 31, reactions under conditions similar to those of Reference Example 48 were carried out to give the title compound (259 mg) as a colorless solid.
¹H-NMR (CDCl₃) δ: 0.81 (6H, d, J = 6.6 Hz), 2.11-2.20 (1H, m), 2.41 (3H, s), 2.66 (3H, s), 3.90 (2H, d, J = 6.9 Hz), 4.26 (1H, br t, J = 4.5 Hz), 4.65 (2H, d, J = 4.5 Hz), 6.86-6.97 (2H, m), 7.30-7.34 (1H, m), 7.57 (1H, d, J = 5.7 Hz), 7.98 (1H, d, J = 5.7 Hz), 10.18 (1H, s).

### Example 56

### 2-methyl-7-{[(1R)-1-phenylethyl]amino}-1-propyl-1H-pyrrolo[2,3-c]pyridine-3-carbaldehyde

Using 2-methyl-N-[(1R)-1-phenylethyl]-1-propyl-1H-pyrrolo[2,3-c]pyridine-7-amine (328 mg) obtained in Example 32, reactions under conditions similar to those of Reference Example 48 were carried out to give the title compound (95 mg) as a colorless solid.
¹H-NMR (CDCl₃) δ: 0.96 (3H, t, J = 7.2 Hz), 1.65 (3H, d, J = 6.6 Hz), 1.78-1.93 (2H, m), 2.68 (3H, s), 4.15-4.21 (2H, m), 4.57 (1H, br d, J = 5.4 Hz), 5.45 (1H, dq, J = 5.4, 6.6 Hz), 7.23-7.28 (1H, m), 7.32-7.37 (2H, m), 7.42-7.45 (2H, m), 7.49 (1H, d, J = 5.4 Hz), 7.91 (1H, d, J = 5.4 Hz), 10.15 (1H, s).

### Example 57

### 2-methyl-7-{[(1S)-1-phenylethyl]amino}-1-propyl-1H-pyrrolo[2,3-c]pyridine-3-carbaldehyde

Using 2-methyl-N-[(1S)-1-phenylethyl]-1-propyl-1H-pyrrolo[2,3-c]pyridine-7-amine (542 mg) obtained in Example 33, reactions under conditions similar to those of Reference Example 48 were carried out to give the title compound (196 mg) as a colorless solid.
¹H-NMR (CDCl₃) δ: 0.96 (3H, t, J = 7.5 Hz), 1.65 (3H, d, J = 6.9 Hz), 1.78-1.93 (2H, m), 2.67 (3H, s), 4.15-4.21 (2H, m), 4.58 (1H, br d, J = 5.7 Hz), 5.45 (1H, dq, J = 5.7, 6.9 Hz), 7.24-7.29 (1H, m), 7.32-7.38 (2H, m), 7.43-7.46 (2H, m), 7.50 (1H, d, J = 5.4 Hz), 7.92 (1H, d, J = 5.4 Hz), 10.15 (1H, s).

### Example 58

### 1-{7-[(4-fluoro-2-methylbenzyl)amino]-2-methyl-1-propyl-1H-pyrrolo[2,3-c]pyridin-3-yl}ethanone

Using N-(4-fluoro-2-methylbenzyl)-2-methyl-1-propyl-1H-pyrrolo[2,3-c]pyridine-7-amine (789 mg) obtained in Example 30, methods similar to those of Reference Example 41 were carried out to give the title compound (380 mg) as colorless crystals.
¹H-NMR (CDCl₃) δ: 0.81 (3H, t, J = 7.5 Hz), 1.72-1.81 (2H, m), 2.42 (3H, s), 2.65 (3H, s), 2.73 (3H, s), 4.10-4.15 (2H, m), 4.33 (1H, br), 4.66 (2H, d, J = 5.4 Hz), 6.85-6.97 (2H, m), 7.25-7.35 (2H, m), 7.94 (1H, d, J = 5.4 Hz).

### Example 59

### [7-(benzylamino)-2-methyl-1H-pyrrolo[2,3-c]pyridin-3-yl]methanol

7-(Benzylamino)-2-methyl-1H-pyrrolo[2,3-c]pyridine-3-carbaldehyde (45 mg) obtained in Example 41 was suspended in ethanol (2 ml), and sodium borohydride (7 mg) was added at 0°C. After stirring at room temperature for 2 hr, several drops of acetic acid were added for a treatment, and the solvent was evaporated under reduced pressure. A 6% aqueous sodium hydrogen carbonate solution was added to the residue and the mixture was extracted with ethyl acetate. The extract was washed with saturated brine, dried over anhydrous sodium sulfate, and concentrated under reduced pressure. The residue was purified by silica gel column chromatography (eluent: ethyl acetate-methanol=9:1) to give the title compound as a yellow powder (26 mg, yield 57%).
¹H-NMR (DMSO-d₆) δ: 2.36 (3H, s), 4.48-4.55 (3H, m), 4.66 (2H, d, J = 5.7 Hz), 6.40 (1H, t, J = 5.7 Hz), 6.76 (1H, d, J = 5.7 Hz), 7.20-7.37 (5H, m), 7.49 (1H, d, J = 5.7 Hz), 10.73 (1H, br s).

### Example 60

### {2-methyl-7-[(2-methylbenzyl)amino]-1-propyl-1H-pyrrolo[2,3-c]pyridin-3-yl}methanol

Using 2-methyl-7-[(2-methylbenzyl)amino]-1-propyl-1H-pyrrolo[2,3-c]pyridine-3-carbaldehyde (183 mg) obtained in Example 50, reactions under conditions similar to those of Example 59 were carried out to give the title compound (121 mg) as a colorless solid.
¹H-NMR (CDCl₃) δ: 0.78 (3H, t, J = 7.5 Hz), 1.34 (1H, br t, J = 4.5 Hz), 1.68-1.80 (2H, m), 2.40 (3H, s), 2.42 (3H, s), 4.02-4.08 (2H, m), 4.36 (1H, br t, J = 4.8 Hz), 4.71 (2H, d, J = 4.8 Hz), 4.76 (2H, d, J = 4.5 Hz), 6.99 (1H, d, J = 5.4 Hz), 7.18-7.24 (3H, m), 7.35-7.38 (1H, m), 7.84 (1H, d, J = 5.4 Hz).

### Example 61

### {7-[(2,6-dimethylbenzyl)amino]-2-methyl-1-propyl-1H-pyrrolo[2,3-c]pyridin-3-yl}methanol

Using 7-[(2,6-dimethylbenzyl)amino]-2-methyl-1-propyl-1H-pyrrolo[2,3-c]pyridine-3-carbaldehyde (52 mg) obtained in Example 51, reactions under conditions similar to those of Example 59 were carried out to give the title compound (30 mg) as a colorless solid.
¹H-NMR (CDCl₃) δ: 0.68 (3H, t, J = 7.5 Hz), 1.24 (1H, t, J = 5.4 Hz), 1.63-1.70 (2H, m), 2.38 (3H, s), 2.43 (6H, s), 3.93-3.98 (2H, m), 4.11 (1H, br t, J = 4.5 Hz), 4.69 (2H, d, J = 4.5 Hz), 4.76 (2H, d, J = 5.4 Hz), 7.00 (1H, d, J = 5.7 Hz), 7.06-7.17 (3H, m), 7.87 (1H, d, J = 5.7 Hz).

### Example 62

### {7-[(4-fluoro-2-methylbenzyl)amino]-2-methyl-1-propyl-1H-pyrrolo[2,3-c]pyridin-3-yl}methanol

Using 7-[(4-fluoro-2-methylbenzyl)amino]-2-methyl-1-propyl-1H-pyrrolo[2,3-c]pyridine-3-carbaldehyde (322 mg) obtained in Example 52, methods similar to those of Example 59 were carried out to give the title compound (171 mg) as colorless crystals.
¹H-NMR (CDCl₃) δ: 0.80 (3H, t, J = 7.5 Hz), 1.35 (1H, br), 1.69-1.80 (2H, m), 2.40-2.41 (6H, m), 4.02-4.08 (2H, m), 4.31 (1H, br), 4.66 (2H, d, J = 4.8 Hz), 4.76 (2H, s), 6.84-6.94 (2H, m), 7.00 (1H, d, J = 5.7 Hz), 7.27-7.34 (1H, m), 7.82 (1H, d, J = 5.7 Hz).

### Example 63

### {7-[(4-fluorobenzyl)amino]-2-methyl-1-propyl-1H-pyrrolo[2,3-c]pyridin-3-yl}methanol

Using 7-[(4-fluorobenzyl)amino]-2-methyl-1-propyl-1H-pyrrolo[2,3-c]pyridine-3-carbaldehyde (170 mg) obtained in Example 53, methods similar to those of Example 59 were carried out to give the title compound (yield 80 mg, yield 47%) as colorless crystals.
¹H-NMR (CDCl₃) δ: 0.86 (3H, t, J = 7.5 Hz), 1.33 (1H, br), 1.70-1.81 (2H, m), 2.41 (3H, s), 4.07-4.12 (2H, m), 4.50 (1H, br), 4.71 (2H, d, J = 5.4 Hz), 4.77 (2H, s), 7.00-7.08 (3H, m), 7.37-7.42 (2H, m), 7.81 (1H, d, J = 5.4 Hz).

### Example 64

### {7-[(2,6-diethylbenzyl)amino]-2-methyl-1-propyl-1H-pyrrolo[2,3-c]pyridin-3-yl}methanol

Using 7-[(2,6-diethylbenzyl)amino]-2-methyl-1-propyl-1H-pyrrolo[2,3-c]pyridine-3-carbaldehyde (82 mg) obtained in Example 54, methods similar to those of Example 59 were carried out to give the title compound (29 mg) as colorless crystals.
¹H-NMR (CDCl₃) δ: 0.64 (3H, t, J = 7.5 Hz), 1.21-1.30 (7H, m), 1.60-1.70 (2H, m), 2.37 (3H, s), 2.76 (4H, q, J = 7.5 Hz), 3.90-3.96 (2H, m), 4.14 (1H, br), 4.69 (2H, d, J = 4.2 Hz), 4.76 (2H, s), 6.99 (1H, d, J = 5.4 Hz), 7.12 (2H, d, J = 7.2 Hz), 7.22-7.26 (1H, m), 7.87 (1H, d, J = 5.4 Hz).

### Example 65

### {7-[(4-fluoro-2-methylbenzyl)amino]-1-isobutyl-2-methyl-1H-pyrrolo[2,3-c]pyridin-3-yl}methanol

Using 7-[(4-fluoro-2-methylbenzyl)amino]-1-isobutyl-2-methyl-1H-pyrrolo[2,3-c]pyridine-3-carbaldehyde (216 mg) obtained in Example 55, reactions under conditions similar to those of Example 59 were carried out to give the title compound (145 mg) as a colorless solid.
¹H-NMR (CDCl₃) δ: 0.76 (6H, d, J = 6.3 Hz), 1.25 (1H, t, J = 5.1 Hz), 2.03-2.14 (1H, m), 2.39 (3H, s), 2.41 (3H, s), 3.86 (2H, d, J = 6.9 Hz), 4.28 (1H, br t, J = 4.8 Hz), 4.65 (2H, d, J = 4.8 Hz), 4.77 (2H, d, J = 5.1 Hz), 6.85-6.96 (2H, m), 7.01 (1H, d, J = 5.4 Hz), 7.30-7.35 (1H, m), 7.84 (1H, d, J = 5.4 Hz).

### Example 66

### (2-methyl-7-{[(1R)-1-phenylethyl]amino}-1-propyl-1H-pyrrolo[2,3-c]pyridin-3-yl)methanol

Using 2-methyl-7-{[(1R)-1-phenylethyl]amino}-1-propyl-1H-pyrrolo[2,3-c]pyridine-3-carbaldehyde (62 mg) obtained in Example 56, reactions under conditions similar to those of Example 59 were carried out to give the title compound (29 mg) as a colorless solid.
¹H-NMR (CDCl₃) δ: 0.92 (3H, t, J = 7.2 Hz), 1.24 (1H, t, J = 5.1 Hz), 1.64 (3H, d, J = 6.6 Hz), 1.73-1.87 (2H, m), 2.40 (3H, s), 4.14 (2H, t, J = 5.1 Hz), 4.59 (1H, br d, J = 6.0 Hz), 4.74 (2H, d, J = 5.1 Hz), 5.42-5.50 (1H, m), 6.93 (1H, d, J = 5.4 Hz), 7.21-7.26 (1H, m), 7.30-7.35 (2H, m), 7.43-7.45 (2H, m), 7.76 (1H, d, J = 5.4 Hz).

### Example 67

### (2-methyl-7-{[(1S)-1-phenylethyl]amino}-1-propyl-1H-pyrrolo[2,3-c]pyridin-3-yl)methanol

Using 2-methyl-7-{[(1S)-1-phenylethyl]amino}-1-propyl-1H-pyrrolo[2,3-c]pyridine-3-carbaldehyde (149 mg) obtained in Example 57, reactions under conditions similar to those of Example 59 were carried out to give the title compound (81 mg) as a colorless solid.
¹H-NMR (CDCl₃) δ: 0.92 (3H, t, J = 7.5 Hz), 1.25 (1H, t, J = 5.1 Hz), 1.64 (3H, d, J = 6.9 Hz), 1.73-1.87 (2H, m), 2.41 (3H, s), 4.12-4.17 (2H, m), 4.60 (1H, br d, J = 6.0 Hz), 4.75 (2H, d, J = 5.1 Hz), 5.47 (1H, dq, J = 6.0, 6.9 Hz), 6.94 (1H, d, J = 5.4 Hz), 7.22-7.28 (1H, m), 7.32-7.37 (2H, m), 7.44-7.47 (2H, m), 7.77 (1H, d, J = 5.4 Hz).

### Example 68

### 1-{7-[(4-fluoro-2-methylbenzyl)amino]-2-methyl-1-propyl-1H-pyrrolo[2,3-c]pyridin-3-yl}ethanol

Using 1-{7-[(4-fluoro-2-methylbenzyl)amino]-2-methyl-1-propyl-1H-pyrrolo[2,3-c]pyridin-3-yl}ethanone (127 mg) obtained in Example 58, reactions under conditions similar to those of Reference Example 43 were carried out to give the title compound (49 mg) as colorless crystals.
¹H-NMR (CDCl₃) δ: 0.79 (3H, t, J = 7.5 Hz), 1.59-1.76 (6H, m), 2.38 (3H, s), 2.41 (3H, s), 4.00-4.06 (2H, m), 4.29 (1H, br), 4.65 (2H, d, J = 4.8 Hz), 5.19 (1H, q, J = 6.6 Hz), 6.84-6.95 (2H, m), 7.14 (1H, d, J = 5.7 Hz), 7.28-7.34 (1H, m), 7.79 (1H, d, J = 5.4 Hz).

### Example 69

### 3-ethyl-N-(4-fluoro-2-methylbenzyl)-2-methyl-1-propyl-1H-pyrrolo[2,3-c]pyridine-7-amine

Using 1-{7-[(4-fluoro-2-methylbenzyl)amino]-2-methyl-1-propyl-1H-pyrrolo[2,3-c]pyridin-3-yl}ethanol (169 mg) obtained in Example 68, reactions under conditions similar to those of Reference Example 45 were carried out to give the title compound (81 mg) as colorless crystals.
¹H-NMR (CDCl₃) δ: 0.78 (3H, t, J = 7.5 Hz), 1.16 (3H, t, J = 7.2 Hz), 1.63-1.80 (2H, m), 2.30 (3H, s), 2.41 (3H, s), 2.64 (2H, q, J = 7.2 Hz), 4.00-4.05 (2H, m), 4.28 (1H, br), 4.65 (2H, d, J = 4.8 Hz), 6.84-6.95 (3H, m), 7.30-7.35 (1H, m), 7.78 (1H, d, J = 5.4 Hz).

### Example 70

### 3-chloro-N-(4-fluoro-2-methylbenzyl)-2-methyl-1-propyl-1H-pyrrolo[2,3-c]pyridine-7-amine

To a solution of N-(4-fluoro-2-methylbenzyl)-2-methyl-1-propyl-1H-pyrrolo[2,3-c]pyridine-7-amine (77 mg) obtained in Example 30 in diethyl ether (5 mL) was added sulfuryl chloride (0.02 mL). The reaction mixture was stirred at room temperature for 1 hr. A saturated aqueous sodium hydrogen carbonate solution was added to the reaction mixture and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=4:1) to give the title compound as an oil (yield 72 mg, yield 83%).
¹H-NMR (CDCl₃) δ: 0.79 (3H, t, J = 7.2 Hz), 1.68-1.78 (2H, m), 2.37 (3H, s), 2.41 (3H, s), 4.01-4.06 (2H, m), 4.30 (1H, br t, J = 4.8 Hz), 4.65 (2H, d, J = 4.8 Hz), 6.83-6.94 (3H, m), 7.28-7.33 (1H, m), 7.82 (1H, d, J = 5.4 Hz).

### Example 71

### 3-bromo-N-(4-fluoro-2-methylbenzyl)-2-methyl-1-propyl-1H-pyrrolo[2,3-c]pyridine-7-amine

To a solution of N-(4-fluoro-2-methylbenzyl)-2-methyl-1-propyl-1H-pyrrolo[2,3-c]pyridine-7-amine (403 mg) obtained in Example 30 in acetic acid (5 mL) was added bromine (0.08 mL). The reaction mixture was stirred at room temperature for 15 min, and concentrated under reduced pressure. A saturated aqueous sodium hydrogen carbonate solution was added to the residue, and the mixture was extracted with ethyl acetate. The extract was washed with a saturated aqueous sodium hydrogen carbonate solution, water and saturated brine, dried over anhydrous sodium sulfate, and concentrated under reduced pressure. The residue was purified by silica gel column chromatography (eluent: hexane-ethyl acetate=5:1) and crystallized from diisopropyl ether to give the title compound as white crystals (yield 259 mg, yield 51%).
¹H-NMR (CDCl₃) δ: 0.80 (3H, t, J = 7.2 Hz), 1.66-1.79 (2H, m), 2.39 (3H, s), 2.41 (3H, s), 4.04-4.09 (2H, m), 4.30 (1H, br t, J = 4.8 Hz), 4.65 (2H, d, J = 4.8 Hz), 6.83-6.94 (3H, m), 7.28-7.33 (1H, m), 7.83 (1H, d, J = 5.4 Hz).

### Example 72

### methyl 3-chloro-7-(benzylamino)-1-propyl-1H-pyrrolo[2,3-c]pyridine-2-carboxylate

To a solution of methyl 3-chloro-7-[benzyl(tert-butoxycarbonyl)amino]-1-propyl-1H-pyrrolo[2,3-c]pyridine-2-carboxylate (338 mg) obtained in Reference Example 74 in methanol (5 mL) was added 6N hydrochloric acid (3 mL), and the reaction mixture was stirred at 90°C for 1 hr. The reaction mixture was cooled to room temperature, and concentrated under reduced pressure. A saturated aqueous sodium hydrogen carbonate solution was added to the residue, and the mixture was extracted with ethyl acetate. The extract was washed with a saturated aqueous sodium hydrogen carbonate solution, water and saturated brine, dried over anhydrous sodium sulfate, and concentrated under reduced pressure. The residue was crystallized from diisopropyl ether to give the title compound as white crystals (yield 180 mg, yield 68%).
¹H-NMR (CDCl₃) δ: 0.79 (3H, t, J = 7.5 Hz), 1.80-1.88 (2H, m), 3.97 (3H, s), 4.52-4.57 (2H, m), 4.73-4.75 (2H, m), 4.79-4.81 (1H, m), 6.98 (1H, d, J = 5.7 Hz), 7.30-7.44 (5H, m), 7.86 (1H, d, J = 5.7 Hz).

### Example 73

### {7-[(4-fluoro-2-methylbenzyl)amino]-3-methyl-1-propyl-1H-pyrrolo[2,3-c]pyridin-2-yl}methanol

To a solution of ethyl 7-[(tert-butoxycarbonyl)(4-fluoro-2-methylbenzyl)amino]-3-methyl-1-propyl-1H-pyrrolo[2,3-c]pyridine-2-carboxylate (944 mg) obtained in Reference Example 73 in diethyl ether (10 mL) was added lithium aluminum hydride (150 mg) at 0°C. The reaction mixture was stirred at the same temperature for 2 hr, and further at room temperature for 1 hr. A 8N aqueous sodium hydroxide solution was added to the reaction mixture, and the mixture was extracted with ethyl acetate. The extract was washed with water and saturated brine, dried over anhydrous sodium sulfate, and concentrated under reduced pressure. The residue was dissolved in methanol (10 mL), and 6N hydrochloric acid (3 mL) was added. The reaction mixture was stirred at 80°C for 15 min. The reaction mixture was cooled to room temperature, and concentrated under reduced pressure. A saturated aqueous sodium hydrogen carbonate solution was added to the residue, and the mixture was extracted with ethyl acetate. The extract was washed with a saturated aqueous sodium hydrogen carbonate solution, water and saturated brine, dried over anhydrous sodium sulfate, and concentrated under reduced pressure. The residue was crystallized from ethyl acetate to give the title compound as white crystals (yield 498 mg, yield 74%).
¹H-NMR (CDCl₃) δ: 0.77 (3H, t, J = 7.5 Hz), 1.70-1.81 (3H, m), 2.24 (3H, s), 2.40 (3H, s), 4.13-4.19 (2H, m), 4.39 (1H, br t, J = 4.5 Hz), 4.64 (2H, d, J = 4.5 Hz), 4.75 (2H, s), 6.82-6.98 (3H, m), 7.27-7.31 (1H, m), 7.78 (1H, d, J = 5.7 Hz).

### Example 74

### N-[7-(2,3-dimethyl-1H-pyrrolo[2,3-c]pyridyl)]benzamide

To a solution of 7-chloro-2,3-dimethyl-1H-pyrrolo[2,3-c]pyridine (306 mg) obtained in Reference Example 2 in toluene (20 mL) were added tris(dibenzylideneacetone)dipalladium (15.8 mg), 9,9-dimethyl-4,5-bis(diphenylphosphino)xanthine (30.1 mg), cesium carbonate (775 mg) and benzamide (291 mg), and the reaction mixture was stirred under an argon atmosphere at 120°C for 14 hr. After cooling to room temperature, a saturated aqueous sodium hydrogen carbonate solution was added to the reaction mixture and the mixture was extracted with ethyl acetate. The extract was washed with a saturated aqueous sodium hydrogen carbonate solution, water and saturated brine, dried over anhydrous magnesium sulfate, and concentrated under reduced pressure. The residue was purified by silica gel column chromatography (eluent: hexane-ethyl acetate=3:1) to give the title compound as white crystals (yield 117 g, yield 26%).
¹H-NMR (CDCl₃) δ: 2.23 (3H, s), 2.43 (3H, s), 7.20 (1H, d, J = 5.7 Hz), 7.47-7.61 (3H, m), 7.79 (1H, d, J = 5.7 Hz), 7.98-8.01 (2H, m), 8.84 (1H, br s), 1H not detected.

The structures of the compounds obtained in Reference Examples 1-13, 25 and 33-37 are shown in Table 1, the structures of the compounds obtained in Reference Examples 26-32 and 38-49 are shown in Table 2, the structures of the compounds obtained in Reference Examples 50-55 are shown in Table 3, and the structures of the compounds obtained in Reference Examples 69-75 are shown in Table 4.

**Table 1**

| | | | | | |
|---|---|---|---|---|---|
| | 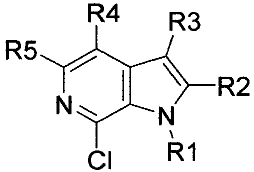 | | | | |

| Ref. Ex. No. | R1 | R2 | R3 | R4 | R5 |
|---|---|---|---|---|---|
| 1 | H | CH₃ | H | H | H |
| 2 | H | CH₃ | CH₃ | H | H |
| 3 | CH₂CH₃ | CH₃ | H | H | H |
| 4 | CH₂CH₂CH₃ | CH₃ | H | H | H |
| 5 | CH₂CH₂CH₂CH₃ | CH₃ | H | H | H |
| 6 | cyclohexylmethyl | CH₃ | H | H | H |
| 7 | cyclopropylmethyl | CH₃ | H | H | H |
| 8 | pyridin-2-ylmethyl | CH₃ | H | H | H |
| 9 | 4-methoxybenzyl | CH₃ | H | H | H |
| 10 | 2-(benzyloxy)ethyl | CH₃ | H | H | H |
| 11 | benzyl | CH₃ | H | H | H |
| 12 | CH₃ | CH₃ | H | H | H |
| 13 | 2-methoxyethyl | CH₃ | H | H | H |
| 25 | H | CH₃ | CH₃ | CH₃ | H |
| 33 | CH₃ | CH₃ | CH₃ | H | H |
| 34 | CH₂CH₃ | CH₃ | CH₃ | H | H |
| 35 | CH₂CH₂CH₃ | CH₃ | CH₃ | H | H |
| 36 | CH₂CH(CH₃)₂ | CH₃ | CH₃ | H | H |
| 37 | CH₂CH(CH₃)₂ | CH₃ | H | H | H |

**Table 2**

| | | | | | |
|---|---|---|---|---|---|
| | 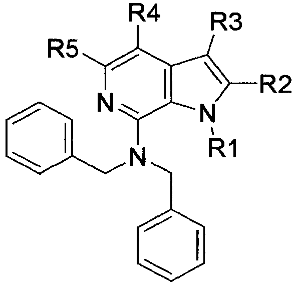 | | | | |

| Ref. Ex. No. | R1 | R2 | R3 | R4 | R5 |
|---|---|---|---|---|---|
| 26 | H | CH₃ | H | H | H |
| 27 | H | CH₃ | H | H | Cl |
| 28 | H | CH₃ | CH₃ | H | Cl |
| 29 | H | CH₃ | CH₃ | H | CF₃ |
| 30 | H | CH₃ | CH₃ | H | CN |
| 31 | H | CH₃ | CH₃ | H | 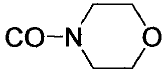 |
| 32 | H | CH₃ | CH₃ | H | CONHCH₂CH₂-OCH₂Ph |
| 38 | CH₂CH₂CH₃ | CH₃ | H | H | H |
| 39 | CH₂CH₂CH₃ | CH₃ | H | H | Cl |
| 40 | CH₂CH₂CH₃ | CH₃ | CH₃ | H | CF₃ |
| 41 | H | CH₃ | COCH₃ | H | H |
| 42 | CH₂CH₂CH₃ | CH₃ | COCH₂CH₃ | H | Cl |
| 43 | H | CH₃ | CH(OH)CH₃ | H | H |
| 44 | CH₂CH₂CH₃ | CH₃ | CH(OH)CH₂CH₃ | H | Cl |
| 45 | H | CH₃ | CH₂CH₃ | H | H |
| 46 | CH₂CH₂CH₃ | CH₃ | CH₂CH₂CH₃ | H | Cl |
| 47 | H | CH₃ | CH₃ | H | CONH₂ |
| 48 | CH₂CH₂CH₃ | CH₃ | CHO | H | H |
| 49 | CH₂CH₂CH₃ | CH₃ | CN | H | H |

**Table 3**

| | | | | | |
|---|---|---|---|---|---|
| | 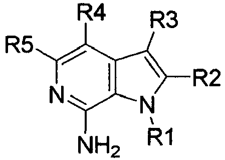 | | | | |

| Ref. Ex. No. | R1 | R2 | R3 | R4 | R5 |
|---|---|---|---|---|---|
| 50 | CH₂CH₂CH₃ | CH₃ | CH₃ | H | CF₃ |
| 51 | H | CH₃ | CH₃ | H | CF₃ |
| 52 | CH₂CH₂CH₃ | CH₃ | CH₂CH₂CH₃ | H | H |
| 53 | H | CH₃ | CH₃ | H | CO-morpholino |
| 54 | H | CH₃ | CH₃ | H | CONHCH₂CH₂OH |
| 55 | CH₂CH₂CH₃ | CH₃ | H | H | H |

**Table 4**

| | | | | | | | | | |
|---|---|---|---|---|---|---|---|---|---|
| | 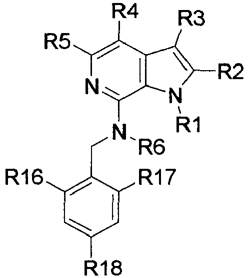 | | | | | | | | |

| Ref. Ex. No. | R1 | R2 | R3 | R4 | R5 | R6 | R16 | R17 | R18 |
|---|---|---|---|---|---|---|---|---|---|
| 69 | H | CO₂CH₃ | H | H | H | Boc | H | H | H |
| 70 | H | CO₂CH₃ | H | H | H | Boc | CH₃ | H | F |
| 71 | H | CO₂CH₂CH₃ | CH₃ | H | H | Boc | CH₃ | H | F |
| 72 | CH₂CH₂CH₃ | CO₂CH₃ | H | H | H | Boc | H | H | H |
| 73 | CH₂CH₂CH₃ | CO₂CH₂CH₃ | CH₃ | H | H | Boc | CH₃ | H | F |
| 74 | CH₂CH₂CH₃ | CO₂CH₃ | Cl | H | H | Boc | H | H | H |
| 75 | CH₂CH₂CH₃ | CH₂OH | CH₃ | H | H | Boc | CH₃ | H | F |

The structures of the compounds obtained in Examples 1-74 are shown in Tables 5-8.

**Table 5**

| | | | | | | | | | |
|---|---|---|---|---|---|---|---|---|---|
| | 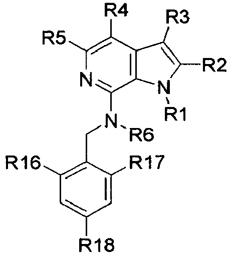 | | | | | | | | |

| Ex. No. | R1 | R2 | R3 | R4 | R5 | R6 | R16 | R17 | R18 |
|---|---|---|---|---|---|---|---|---|---|
| 1 | CH₂CH₃ | CH₃ | H | H | H | H | H | H | H |
| 2 | CH₂CH₂CH₃ | CH₃ | H | H | H | H | H | H | H |
| 3 | CH₂CH₂CH₂CH₃ | CH₃ | H | H | H | H | H | H | H |
| 4 | cyclohexylmethyl | CH₃ | H | H | H | H | H | H | H |
| 5 | cyclopropylmethyl | CH₃ | H | H | H | H | H | H | H |
| 6 | pyridin-2-ylmethyl | CH₃ | H | H | H | H | H | H | H |
| 7 | 4-methoxybenzyl | CH₃ | H | H | H | H | H | H | H |
| 8 | 2-(benzyloxy)ethyl | CH₃ | H | H | H | H | H | H | H |
| 9 | benzyl | CH₃ | H | H | H | H | H | H | H |
| 10 | CH₃ | CH₃ | H | H | H | H | H | H | H |
| 11 | 2-methoxyethyl | CH₃ | H | H | H | H | H | H | H |
| 12 | H | CH₃ | CH₃ | H | H | H | H | H | H |
| 13 | H | CH₃ | CH₃ | H | H | H | CH₃ | H | H |
| 14 | H | CH₃ | CH₃ | H | H | H | CH₃ | CH₃ | H |
| 15 | 2-hydroxyethyl | CH₃ | H | H | H | H | H | H | H |
| 18 | H | CH₃ | CH₃ | H | H | H | CF₃ | H | H |
| 19 | H | CH₃ | CH₃ | H | H | H | CH₃ | H | CH₃ |
| 20 | H | CH₃ | CH₃ | H | H | H | CH₃ | H | F |
| 21 | H | CH₃ | CH₃ | H | H | CH₃ | H | H | H |
| 22 | CH₃ | CH₃ | CH₃ | H | H | H | H | H | H |
| 23 | CH₂CH₃ | CH₃ | CH₃ | H | H | H | H | H | H |
| 24 | CH₂CH₃ | CH₃ | CH₃ | H | H | H | CH₃ | H | F |
| 25 | CH₂CH₂CH₃ | CH₃ | CH₃ | H | H | H | H | H | H |
| 26 | CH₂CH₂CH₃ | CH₃ | CH₃ | H | H | H | CH₃ | H | F |
| 27 | CH₂CH(CH₃)₂ | CH₃ | CH₃ | H | H | H | CH₃ | H | F |
| 28 | CH₂CH₂CH₃ | CH₃ | H | H | H | H | CH₃ | H | H |
| 29 | CH₂CH₂CH₃ | CH₃ | H | H | H | H | CH₃ | CH₃ | H |
| 30 | CH₂CH₂CH₃ | CH₃ | H | H | H | H | CH₃ | H | F |

**Table 6**

| | | | | | | | | | |
|---|---|---|---|---|---|---|---|---|---|
| | 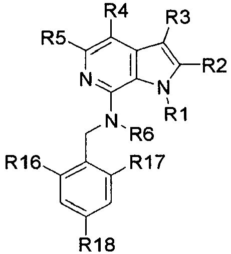 | | | | | | | | |

| Ex. No. | R1 | R2 | R3 | R4 | R5 | R6 | R16 | R17 | R18 |
|---|---|---|---|---|---|---|---|---|---|
| 31 | CH₂CH(CH₃)₂ | CH₃ | H | H | H | H | CH₃ | H | F |
| 34 | H | CH₃ | CH₃ | CH₃ | H | H | H | H | H |
| 35 | CH₂CH₂CH₃ | CH₃ | CN | H | H | H | H | H | H |
| 36 | H | CH₃ | CH₂CH₃ | H | H | H | H | H | H |
| 37 | H | CH₃ | COCH₃ | H | H | H | H | H | H |
| 38 | H | CH₃ | CH₃ | H | Cl | H | H | H | H |
| 39 | H | CH₃ | CH₃ | H | CN | H | H | H | H |
| 40 | H | CH₃ | CH₃ | H | CONH₂ | H | H | H | H |
| 41 | H | CH₃ | CHO | H | H | H | H | H | H |
| 42 | CH₂CH₂CH₃ | CH₃ | CH₃ | H | CF₃ | H | CH₃ | H | F |
| 43 | H | CH₃ | CH₃ | H | CF₃ | H | CH₃ | H | F |
| 44 | CH₂CH₂CH₃ | CH₃ | CH₂CH₂CH₃ | H | H | H | CH₃ | H | F |
| 45 | H | CH₃ | CH₃ | H | 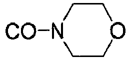 | H | CH₃ | H | F |
| 46 | CH₂CH₂CH₃ | CH₃ | H | H | H | H | H | H | F |
| 47 | CH₂CH₂CH₃ | CH₃ | H | H | H | H | CH₂CH₃ | CH₂CH₃ | H |
| 49 | H | CH₃ | CH₃ | H | CONHCH₂CH₂OH | H | CH₃ | H | F |
| 50 | CH₂CH₂CH₃ | CH₃ | CHO | H | H | H | CH₃ | H | H |
| 51 | CH₂CH₂CH₃ | CH₃ | CHO | H | H | H | CH₃ | CH₃ | H |
| 52 | CH₂CH₂CH₃ | CH₃ | CHO | H | H | H | CH₃ | H | F |
| 53 | CH₂CH₂CH₃ | CH₃ | CHO | H | H | H | H | H | F |
| 54 | CH₂CH₂CH₃ | CH₃ | CHO | H | H | H | CH₂CH₃ | CH₂CH₃ | H |
| 55 | CH₂CH(CH₃)₂ | CH₃ | CHO | H | H | H | CH₃ | H | F |
| 58 | CH₂CH₂CH₃ | CH₃ | COCH₃ | H | H | H | CH₃ | H | F |
| 59 | H | CH₃ | CH₂OH | H | H | H | H | H | H |
| 60 | CH₂CH₂CH₃ | CH₃ | CH₂OH | H | H | H | CH₃ | H | H |

**Table 7**

| | | | | | | | | | |
|---|---|---|---|---|---|---|---|---|---|
| | 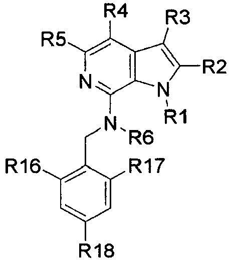 | | | | | | | | |

| Ex. No. | R1 | R2 | R3 | R4 | R5 | R6 | R16 | R17 | R18 |
|---|---|---|---|---|---|---|---|---|---|
| 61 | CH₂CH₂CH₃ | CH₃ | CH₂OH | H | H | H | CH₃ | CH₃ | H |
| 62 | CH₂CH₂CH₃ | CH₃ | CH₂OH | H | H | H | CH₃ | H | F |
| 63 | CH₂CH₂CH₃ | CH₃ | CH₂OH | H | H | H | H | H | F |
| 64 | CH₂CH₂CH₃ | CH₃ | CH₂OH | H | H | H | CH₂CH₃ | CH₂CH₃ | H |
| 65 | CH₂CH(CH₃)₂ | CH₃ | CH₂OH | H | H | H | CH₃ | H | F |
| 68 | CH₂CH₂CH₃ | CH₃ | CH(OH)CH₃ | H | H | H | CH₃ | H | F |
| 69 | CH₂CH₂CH₃ | CH₃ | CH₂CH₃ | H | H | H | CH₃ | H | F |
| 70 | CH₂CH₂CH₃ | CH₃ | Cl | H | H | H | CH₃ | H | F |
| 71 | CH₂CH₂CH₃ | CH₃ | Br | H | H | H | CH₃ | H | F |
| 72 | CH₂CH₂CH₃ | CO₂CH₃ | Cl | H | H | H | H | H | H |
| 73 | CH₂CH₂CH₃ | CH₂OH | CH₃ | H | H | H | CH₃ | H | F |

**Table 8**

| | | | |
|---|---|---|---|
| 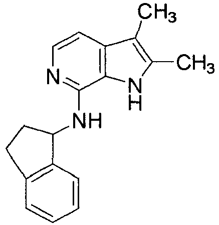 | 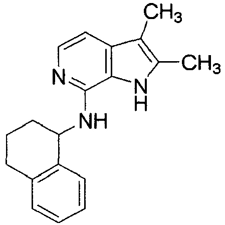 | 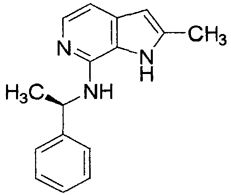 | 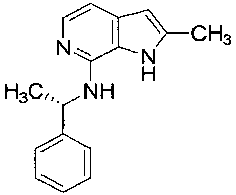 |
| Example 16 | Example 17 | Example 32 | Example 33 |
| 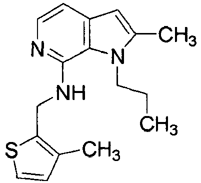 | 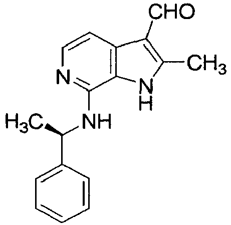 | 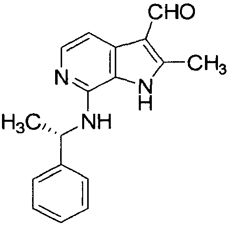 | 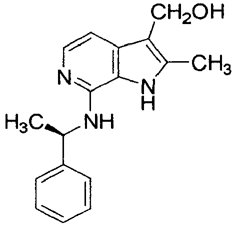 |
| Example 48 | Example 56 | Example 57 | Example 66 |
| 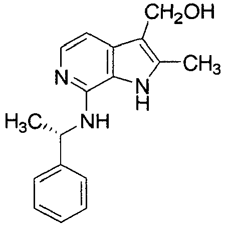 | 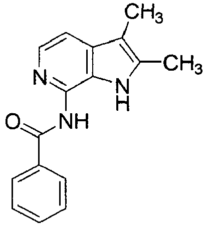 | | |
| Example 67 | Example 74 | | |

### Experimental Example

### proton, potassium-adenosine triphosphatase (H⁺/K⁺-ATPase) inhibitory activity test

According to the method of Wallmark et al. [Biochem. Biophys. Acta., 728, 31 (1983)], a stomach mucous membrane microsome fraction was prepared from the stomach of a pig. First, the corpus ventriculi was isolated, washed with tap water, immersed in 3M brine, and the surface of the mucous membrane was wiped with a paper towel. The stomach mucous membrane was separated, chopped, and homogenized using polytron (Kinematica) in 0.25 M saccharose solution (pH 6.8) containing 1 mM EDTA and 10 mM tris hydrochloric acid. The obtained homogenate was centrifuged at 20,000×g for 30 min, and the supernatant was centrifuged at 100,000×g for 90 min. After suspending the precipitated part in a 0.25 M saccharose solution, layered on a 0.25 M saccharose solution containing 7.5% Ficoll, and centrifuged at 100,000×g for 5 hr. The fraction of the interface between the both layers was collected and washed by centrifugation with a 0.25 M saccharose solution.

The obtained microsome fraction was used as the H⁺/K⁺-ATPase standard product.

A test compound (5 µL) dissolved in a 10% aqueous dimethyl sulfoxide solution was added to 40 µL of a 50 mM Hepes-tris buffer (5 mM magnesium chloride, 10 mM potassium chloride, 10 µM valinomycin, pH=6.5) containing 2.5 µg/mL (based on the protein concentration) of an enzyme standard product, and the mixture was incubated at 37°C for 30 min. The enzyme reaction was started by the addition of 5 µL of a 2 mM adenosine triphosphate tris salt solution (50 mM Hepes-tris buffer (5 mM magnesium chloride, pH 6.5)). The enzyme reaction was carried out at 37°C for 20 min, and quenched by adding 15 µL of malachite green solution (a mixture of 0.12% malachite green sulfuric acid (5N) solution, 7.5% ammonium molybdate and 11% Tween 20 at a ratio of 100:25:2). The mixture was stood at room temperature for 15 min, and the resulting reaction product of inorganic phosphorus and malachite green was colorimetrically determined at a wavelength of 610 nm. The amount of inorganic phosphoric acid in a reaction solution free of potassium chloride was measured in the same manner. The H⁺/K⁺-ATPase activity was measured by subtracting the obtained amount from the amount of inorganic phosphoric acid in the presence of potassium chloride. The inhibitory rate (%) was determined from the control activity value and the activity value at each concentration of the test compound, and 50% inhibitory concentration (IC₅₀) of proton and potassium-adenosine triphosphatase was determined. The results are shown in Table 9. Example compounds 2, 5 and 65 do not fall within the scope of the claims and are provided for comparative purposes.

**Table 9**

| Example compound | IC₅₀ (µM) |
|---|---|
| 2 | 0.98 |
| 5 | 1.2 |
| 12 | 0.027 |
| 14 | 0.014 |
| 16 | 0.0098 |
| 26 | 0.024 |
| 65 | 0.0032 |
| 74 | 1.8 |

From the results of Table 9, it is clear that the compound (I) of the present invention has a superior H⁺/K⁺-ATPase inhibitory activity.

### Industrial Applicability

Since compound (II) including compound (I) shows a superior proton pump inhibitory action, it is useful as a clinically beneficial agent for the prophylaxis or treatment of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress and the like; Helicobacter pylori eradication agent; or a suppressant of upper gastrointestinal hemorrhage due to peptic ulcer, acute stress ulcer, hemorrhagic gastritis or invasive stress.

## Claims

1. A compound represented by the formula (I): 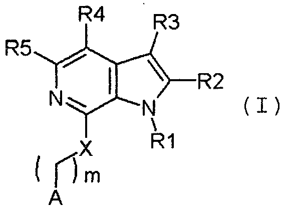
wherein R1 is (i) a hydrogen atom, or (ii) a hydrocarbon group selected from a C₁₋₆ alkyl group, a C₂₋₆ alkenyl group, a C₂₋₆ alkynyl group, a C₃₋₇ cycloalkyl group, a C₆₋₁₄ aryl group, a C₇₋₁₆ aralkyl group, and a group represented by the formula: 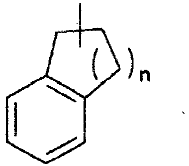 wherein n is 1 or 2, each of which is optionally substituted by substituent(s) selected from (1) halogen atom, (2) nitro, (3) cyano, (4) hydroxy, (5) C₁₋₆ alkoxy optionally having 1 to 3 halogen atoms,(6) C₆₋₁₄ aryloxy, (7) C₇₋₁₆ aralkyloxy, (8) mercapto, (9) C₁₋₆ alkylthio optionally having 1 to 3 halogen atoms, (10) C₆₋₁₄ arylthio, (11) C₇₋₁₆ aralkylthio, (12) amino, (13) mono-C₁₋₆ alkylamino, (14) mono-C₆₋₁₄ arylamino, (15) mono-C₇₋₁₆ aralkylamino, (16) di-C₁₋₆ alkylamino, (17) di-C₆₋₁₄ arylamino, (18) di-C₇₋₁₆ aralkylamino, (19) formyl, (20) C₁₋₆ alkyl-carbonyl, (21) C₆₋₁₄ aryl-carbonyl, (22) carboxyl, (23) C₁₋₆ alkoxy-carbonyl, (24) C₆₋₁₄ aryloxy-carbonyl, (25) carbamoyl, (26) thiocarbamoyl, (27) mono-C₁₋₆ alkyl-carbamoyl, (28) di-C₁₋₆ alkyl-carbamoyl, (29) C₆₋₁₄ aryl-carbamoyl, (30) C₁₋₆ alkylsulfonyl, (31) C₆₋₁₄ arylsulfonyl, (32) C₁₋₆ alkylsulfinyl, (33) C₆₋₁₄ arylsulfinyl, (34) formylamino, (35) C₁₋₆ alkyl-carbonylamino, (36) C₆₋₁₄ aryl-carbonylamino, (37) C₁₋₆ alkoxy-carbonylamino, (38) C₁₋₆ alkylsulfonylamino, (39) C₆₋₁₄ arylsulfonylamino, (40) C₁₋₆ alkyl-carbonyloxy, (41) C₆₋₁₄ aryl-carbonyloxy, (42) C₁₋₆ alkoxy-carbonyloxy, (43) mono-C₁₋₆ alkyl-carbamoyloxy, (44) di-C₁₋₆ alkyl-carbamoyloxy, (45) C₆₋₁₄ aryl-carbamoyloxy, (46) a 5- to 10-membered heterocyclic group containing, besides carbon atom, one or two kinds of 1 to 4 hetero atoms selected from nitrogen atom, sulfur atom and oxygen atom, (47) C₁₋₃ alkylenedioxy, (48) C₃₋₇ cycloalkyl,
R2 is a C₁₋₄ alkyl group,
R3 is a C₁₋₄ alkyl group,
R4 and R5 are the same or different and each is a hydrogen atom, or a C₁₋₆ alkyl group,
X is a bond, O, or 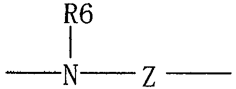 [wherein R6 is a hydrogen atom or a C₁₋₆ alkyl group, and Z is a bond or -CO-],
m is 0 or 1,
A is a) a hydrocarbon group selected from i) a C₆₋₁₀ aryl group optionally substituted by 1 to 4 substituents selected from C₁₋₆ alkyl optionally substituted by 1 to 5 halogens, C₁₋₆ alkoxy, cyano and halogen atom, ii) C₇₋₁₆ aralkyl group optionally substituted by 1 to 4 substituents selected from C₁₋₆ alkyl optionally substituted by 1 to 5 halogens, C₁₋₆ alkoxy, cyano and halogen atom, and iii) a group represented by the formula: 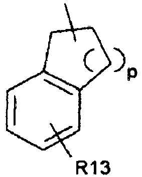 wherein p is 1 or 2, and R13 is (i) a hydrogen atom or (ii) a C₁₋₆ alkyl group optionally substituted by 1 to 4 substituents selected from hydroxy, cyano and C₁₋₆ alkoxy, or b) a 5- to 14-membered monocyclic or bicyclic heterocyclic group containing, besides carbon atom, one or two kinds of 1 to 4 hetero atoms selected from nitrogen atom, oxygen atom and sulfur atom, optionally substituted by 1 to 3 substituent(s) selected from (1) halogen atom, (2) nitro, (3) cyano, (4) C₁₋₆ alkyl optionally having 1 to 3 halogen atoms, (5) C₆₋₁₄ aryl, (6) hydroxy, (7) C₁₋₆ alkoxy optionally having 1 to 3 halogen atoms, (8) C₆₋₁₄ aryloxy, (9) C₇₋₁₆ aralkyloxy, (10) mercapto, (11) C₁₋₆ alkylthio optionally having 1 to 3 halogen atoms, (12) C₆₋₁₄ arylthio, (13) C₇₋₁₆ aralkylthio, (14) amino, (15) mono-C₁₋₆ alkylamino, (16) mono-C₆₋₁₄ arylamino, (17) mono-C₇₋₁₆ aralkylamino, (18) di-C₁₋₆ alkylamino, (19) di-C₆₋₁₄ arylamino, (20) di-C₇₋₁₆ aralkylamino, (21) formyl, (22) C₁₋₆ alkyl-carbonyl, (23) C₆₋₁₄ aryl-carbonyl, (24) carboxyl, (25) C₁₋₆ alkoxy-carbonyl, (26) C₆₋₁₄ aryloxy-carbonyl, (27) carbamoyl, (28) thiocarbamoyl, (29) mono-C₁₋₆ alkyl-carbamoyl, (30) di-C₁₋₆ alkyl-carbamoyl, (31) C₆₋₁₄ aryl-carbamoyl, (32) C₁₋₆ alkylsulfonyl, (33) C₆₋₁₄ arylsulfonyl, (34) C₁₋₆ alkylsulfinyl, (35) C₆₋₁₄ arylsulfinyl, (36) formylamino, (37) C₁₋₆ alkyl-carbonylamino, (38) C₆₋₁₄ aryl-carbonylamino, (39) C₁₋₆ alkoxy-carbonylamino, (40) C₁₋₆ alkylsulfonylamino, (41) C₆₋₁₄ arylsulfonylamino, (42) C₁₋₆ alkyl-carbonyloxy, (43) C₆₋₁₄ aryl-carbonyloxy, (44) C₁₋₆ alkoxy-carbonyloxy, (45) mono-C₁₋₆ alkyl-carbamoyloxy, (46) di-C₁₋₆ alkyl-carbamoyloxy, (47) C₆₋₁₄ aryl-carbamoyloxy, (48) 5- to 7-membered saturated cyclic amino optionally containing one nitrogen atom and, besides carbon atom, one or two kinds of 1 to 4 hetero atoms selected from nitrogen atom, sulfur atom and oxygen atom, (49) a 5- to 10-membered aromatic heterocyclic group containing, besides carbon atom, one or two kinds of 1 to 4 hetero atoms selected from nitrogen atom, sulfur atom and oxygen atom, (50) C₁₋₃ alkylenedioxy, (51) C₃₋₇ cycloalkyl] or a salt thereof.

2. The compound of claim 1, wherein R1 is i) a hydrogen atom, ii) a C₁₋₆ alkyl group optionally substituted by substituent(s) selected from halogen atom, hydroxy, mono-C₁₋₆ alkylamino, di-C₁₋₆ alkylamino, C₁₋₆ alkoxy, C₇₋₁₆ aralkyloxy, C₃₋₇ cycloalkyl and 5- or 6-membered heterocyclic group, iii) a C₂₋₆ alkenyl group or iv) a C₇₋₁₆ aralkyl group optionally substituted by C₁₋₆ alkoxy.

3. The compound of claim 1, wherein m is 1.

4. The compound of claim 1, wherein A is i) a C₆₋₁₄ aryl group optionally substituted by substituent(s) selected from C₁₋₆ alkyl optionally substituted by halogen, C₁₋₆ alkoxy, cyano and halogen atom, ii) a 5- or 6-membered heterocyclic group optionally substituted by substituent (s) selected from C₁₋₆ alkyl, C₁₋₆ alkoxy, cyano and halogen atom, iii) a 2,3-dihydro-1H-inden-1-yl group or iv) a 1,2,3,4-tetrahydronaphthalen-1-yl group.

5. The compound of claim 1, which is selected from
N-(2,3-dihydro-1H-inden-1-yl)-2,3-dimethyl-1H-pyrrolo[2,3-c]pyridine-7-amine,
N-(4-fluoro-2-methylbenzyl)-2,3-dimethyl-1-propyl-1H-pyrrolo[2,3-c]pyridine-7-amine,
and
N-[7-(2,3-dimethyl-1H-pyrrolo[2,3-c]pyridyl)]benzamide.

6. A production method of a compound represented by the formula: 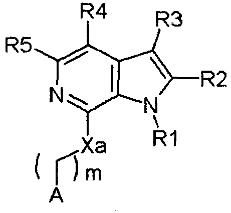 Wherein R1, R2, R3, R4, R5, m and A are as defined in claim 1
Xa is O, or 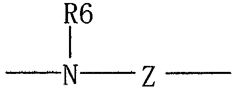 [wherein R6 is a hydrogen atom or a C₁₋₆ alkyl group, and Z is a bond or -CO-],or a salt thereof, which comprises reacting a compound represented by the formula: 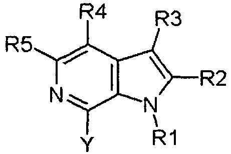 wherein Y is a leaving group, and other symbols are as defined in claim 1, or a salt thereof, with a compound represented by formula: 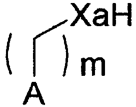 wherein Xa is as defined above, and m and A are as defined in claim 1, or a salt thereof.

7. A compound represented by the formula: 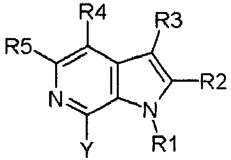 wherein Y is a leaving group, and other symbols are as defined in claim 1, or a salt thereof.

8. A pharmaceutical agent comprising the compound of claim 1.

9. A proton pump inhibitor comprising a compound represented by the formula (I): 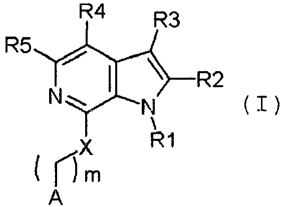 as defined in claim 1, or a salt thereof.

10. The pharmaceutical agent of claim 8, which is an agent for the treatment or prophylaxis of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress; a Helicobacter pylori eradication agent; or a suppressant of upper gastrointestinal hemorrhage due to peptic ulcer, acute stress ulcer, hemorrhagic gastritis or invasive stress.

11. Use of the compound of claim 1 for the production of an agent for the treatment or prophylaxis of peptic ulcer, Zollinger-Ellison syndrome, gastritis, reflux esophagitis, gastroesophageal reflux unaccompanied by esophagitis (Symptomatic Gastroesophageal Reflux Disease (Symptomatic GERD)), NUD (Non Ulcer Dyspepsia), gastric cancer, stomach MALT lymphoma, ulcer caused by a non-steroidal anti-inflammatory agent or hyperacidity and ulcer due to a postoperative stress; a Helicobacter pylori eradication agent; or a suppressant of upper gastrointestinal hemorrhage due to peptic ulcer, acute stress ulcer, hemorrhagic gastritis or invasive stress.

## Patentansprüche

1. Verbindung, die durch die Formel (I) 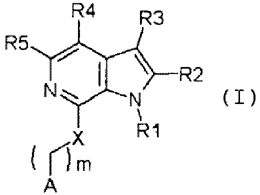
dargestellt wird, wobei R1 Folgendes ist: (i) ein Wasserstoffatom oder (ii) eine Kohlenwasserstoffgruppe, die aus einer C₁₋₆-Alkylgruppe, einer C₂₋₆-Alkenylgruppe, einer C₂₋₆-Alkinylgruppe, einer C₃₋₇-Cycloalkylgruppe, einer C₆₋₁₄-Arylgruppe, einer C₇₋₁₆-Aralkylgruppe und einer durch die Formel 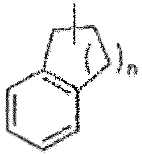 dargestellten Gruppe, wobei n = 1 oder 2 ist, ausgewählt ist, von denen jede gegebenenfalls mit einem oder mehreren Substituenten substituiert ist, die ausgewählt sind aus (1) Halogenatom, (2) Nitro, (3) Cyano, (4) Hydroxy, (5) C₁₋₆-Alkoxy, das gegebenenfalls 1 bis 3 Halogenatome aufweist, (6) C₆₋₁₄-Aryloxy, (7) C₇₋₁₆-Aralkyloxy, (8) Mercapto, (9) C₁₋₆-Alkylthio, das gegebenenfalls 1 bis 3 Halogenatome aufweist, (10) C₆₋₁₄-Arylthio, (11) C₇₋₁₆-Aralkylthio, (12) Amino, (13) Mono-C₁₋₆-alkylamino, (14) Mono-C₆₋₁₄-arylamino, (15) Mono-C₇₋₁₆-aralkylamino, (16) Di-C₁₋₆-alkylamino, (17) Di-C₆₋₁₄-arylamino, (18) Di-C₇₋₁₆-aralkylamino, (19) Formyl, (20) C₁₋₆-Alkylcarbonyl, (21) C₆₋₁₄-Arylcarbonyl, (22) Carboxy, (23) C₁₋₆-Alkoxycarbonyl, (24) C₆₋₁₄-Aryloxycarbonyl, (25) Carbamoyl, (26) Thiocarbamoyl, (27) Mono-C₁₋₆-alkylcarbamoyl, (28) Di-C₁₋₆-alkylcarbamoyl, (29) C₆₋₁₄-Arylcarbamoyl, (30) C₁₋₆-Alkylsulfonyl, (31) C₆₋₁₄-Arylsulfonyl, (32) C₁₋₆-Alkylsulfinyl, (33) C₆₋₁₄-Arylsulfinyl, (34) Formylamino, (35) C₁₋₆-Alkylcarbonylamino, (36) C₆₋₁₄-Arylcarbonylamino, (37) C₁₋₆-Alkoxy-carbonylamino, (38) C₁₋₆-Alkylsulfonylamino, (39) C₆₋₁₄-Arylsulfonylamino, (40) C₁₋₆-Alkylcarbonyloxy, (41) C₆₋₁₄-Arylcarbonyloxy, (42) C₁₋₆-Alkoxy-carbonyloxy, (43) Mono-C₁₋₆-alkylcarbamoyloxy, (44) Di-C₁₋₆-alkylcarbamoyloxy, (45) C₆₋₁₄-Arylcarbamoyloxy, (46) einer fünf- bis zehngliedrigen heterocyclischen Gruppe, die neben Kohlenstoffatomen eine oder zwei Arten von 1 bis 4 Heteroatomen enthält, die aus Stickstoffatom, Schwefelatom und Sauerstoffatom ausgewählt sind, (47) C₁₋₃-Alkylendioxy, (48) C₃₋₇-Cycloalkyl,
R2 eine C₁₋₄-Alkylgruppe ist,
R3 eine C₁₋₄-Alkylgruppe ist,
R4 und R5 gleich oder verschieden sind und jeweils ein Wasserstoffatom oder eine C₁₋₆-Alkylgruppe sind,
X eine Bindung, O oder 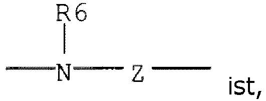 [wobei R6 ein Wasserstoffatom oder eine C₁₋₆-Alkylgruppe ist und Z eine Bindung oder -CO- ist],
m = 0 oder 1 ist,
A Folgendes ist: a) eine Kohlenwasserstoffgruppe, die ausgewählt ist aus: i) einer C₆₋₁₀-Arylgruppe, die gegebenenfalls mit 1 bis 4 Substituenten substituiert ist, die aus C₁₋₆-Alkyl, das gegebenenfalls mit 1 bis 5 Halogenatomen substituiert ist, C₁₋₆-Alkoxy, Cyano und einem Halogenatom ausgewählt sind, ii) einer C₇₋₁₆-Aralkylgruppe, die gegebenenfalls mit 1 bis 4 Substituenten substituiert ist, die aus C₁₋₆-Alkyl, das gegebenenfalls mit 1 bis 5 Halogenatomen substituiert ist, C₁₋₆-Alkoxy, Cyano und einem Halogenatom ausgewählt sind, und (iii) einer Gruppe, die durch die Formel 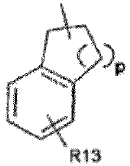 dargestellt wird, wobei p = 1 oder 2 ist und R13 Folgendes ist: i) ein Wasserstoffatom oder ii) eine C₁₋₆-Alkylgruppe, die gegebenenfalls mit 1 bis 4 Substituenten substituiert ist, die aus Hydroxy, Cyano und C₁₋₆-Alkoxy ausgewählt sind;
oder b) eine fünf- bis vierzehngliedrige monocyclische oder bicyclische heterocyclische Gruppe, die neben Kohlenstoffatomen eine oder zwei Arten von 1 bis 4 Heteroatomen enthält, die aus Stickstoffatom, Schwefelatom und Sauerstoffatom ausgewählt sind, die gegebenenfalls mit 1 bis 3 Substituenten substituiert ist, die ausgewählt sind aus (1) Halogenatom, (2) Nitro, (3) Cyano, (4) C₁₋₆-Alkyl, das gegebenenfalls 1 bis 3 Halogenatome aufweist, (5) C₆₋₁₄-Aryl, (6) Hydroxy, (7) C₁₋₆-Alkoxy, das gegebenenfalls 1 bis 3 Halogenatome aufweist, (8) C₆₋₁₄-Aryloxy, (9) C₇₋₁₆-Aralkyloxy, (10) Mercapto, (11) C₁₋₆-Alkylthio, das gegebenenfalls 1 bis 3 Halogenatome aufweist, (12) C₆₋₁₄-Arylthio, (13) C₇₋₁₆-Aralkylthio, (14) Amino, (15) Mono-C₁₋₆-alkylamino, (16) Mono-C₆₋₁₄-arylamino, (17) Mono-C₇₋₁₆-aralkylamino, (18) Di-C₁₋₆-alkylamino, (19) Di-C₆₋₁₄-arylamino, (20) Di-C₇₋₁₆-aralkylamino, (21) Formyl, (22) C₁₋₆-Alkylcarbonyl, (23) C₆₋₁₄-Arylcarbonyl, (24) Carboxy, (25) C₁₋₆-Alkoxycarbonyl, (26) C₆₋₁₄-Aryloxycarbonyl, (27) Carbamoyl, (28) Thiocarbamoyl, (29) Mono-C₁₋₆-alkylcarbamoyl, (30) Di-C₁₋₆-alkylcarbamoyl, (31) C₆₋₁₄-Arylcarbamoyl, (32) C₁₋₆-Alkylsulfonyl, (33) C₆₋₁₄-Arylsulfonyl, (34) C₁₋₆-Alkylsulfinyl, (35) C₆₋₁₄-Arylsulfinyl, (36) Formylamino, (37) C₁₋₆-Alkylcarbonylamino, (38) C₆₋₁₄-Arylcarbonylamino, (39) C₁₋₆-Alkoxycarbonylamino, (40) C₁₋₆-Alkylsulfonylamino, (41) C₆₋₁₄-Arylsulfonylamino, (42) C₁₋₆-Alkylcarbonyloxy, (43) C₆₋₁₄-Arylcarbonyloxy, (44) C₁₋₆-Alkoxycarbonyloxy, (45) Mono-C₁₋₆-alkylcarbamoyloxy, (46) Di-C₁₋₆-alkylcarbamoyloxy, (47) C₆₋₁₄-Arylcarbamoyloxy, (48) fünf- bis siebengliedriges gesättigtes cyclisches Amino, das gegebenenfalls ein Stickstoffatom und neben Kohlenstoffatomen eine oder zwei Arten von 1 bis 4 Heteroatomen enthält, die aus Stickstoffatom, Schwefelatom und Sauerstoffatom ausgewählt sind, (49) eine fünf- bis zehngliedrige aromatische heterocyclische Gruppe, die neben Kohlenstoffatomen eine oder zwei Arten von 1 bis 4 Heteroatomen enthält, die aus Stickstoffatom, Schwefelatom und Sauerstoffatom ausgewählt sind, (50) C₁₋₃-Alkylendioxy, (51) C₃₋₇-Cycloalkyl oder ein Salz davon.

2. Verbindung gemäß Anspruch 1, wobei R1 Folgendes ist: i) ein Wasserstoffatom, (ii) eine C₁₋₆-Alkylgruppe, die gegebenenfalls mit einem oder mehreren Substituenten substituiert ist, die aus einem Halogenatom, Hydroxy, Mono-C₁₋₆-alkylamino, Di-C₁₋₆-alkylamino, C₁₋₆-Alkoxy, C₇₋₁₆-Aralkyloxy, C₃₋₇-Cycloalkyl und einer fünf- oder sechsgliedrigen heterocyclischen Gruppe ausgewählt sind, iii) eine C₂₋₆-Alkenylgruppe oder iv) eine C₇₋₁₆-Aralkylgruppe, die gegebenenfalls mit C₁₋₆-Alkoxy substituiert ist.

3. Verbindung gemäß Anspruch 1, wobei m = 1 ist.

4. Verbindung gemäß Anspruch 1, wobei A Folgendes ist: i) eine C₆₋₁₄-Arylgruppe, die gegebenenfalls mit einem oder mehreren Substituenten substituiert ist, die aus C₁₋₆-Alkyl, das gegebenenfalls mit Halogen substituiert ist, C₁₋₆-Alkoxy, Cyano und Halogenatom ausgewählt sind, ii) eine fünf- oder sechsgliedrige heterocyclische Gruppe, die gegebenenfalls mit einem oder mehreren Substituenten substituiert ist, die aus C₁₋₆-Alkyl, C₁₋₆-Alkoxy, Cyano und Halogenatom ausgewählt sind, iii) eine 2,3-Dihydro-1H-inden-1-yl-Gruppe oder iv) eine 1,2,3,4-Tetrahydronaphthalin-1-yl-Gruppe.

5. Verbindung gemäß Anspruch 1, die aus
N-(2,3-Dihydro-1H-inden-1-yl)-2,3-dimethyl-1H-pyrrolo[2,3-c]pyridin-7-amin,
N-(4-Fluor-2-methylbenzyl)-2,3-dimethyl-1-propyl-1H-pyrrolo[2,3-c]pyridin-7-amin und
N-[7-(2,3-Dimethyl-1H-pyrrolo[2,3-c]pyridyl)]benzamid
ausgewählt ist.

6. Herstellungsverfahren für eine Verbindung, die durch die Formel 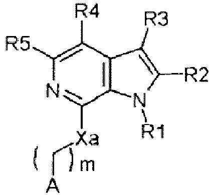 dargestellt wird, wobei R1, R2, R3, R4, R5, m und A wie in Anspruch 1 definiert sind;
Xa Folgendes ist: O oder 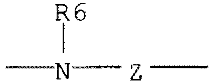 [wobei R6 ein Wasserstoffatom oder eine C₁₋₆-Alkylgruppe ist und Z eine Bindung oder -CO- ist], oder ein Salz davon, umfassend das Umsetzen einer Verbindung, die durch die Formel 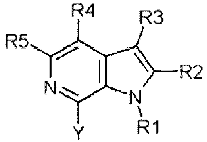 dargestellt wird, wobei Y eine Abgangsgruppe ist und die anderen Symbole wie in Anspruch 1 definiert sind, oder eines Salzes davon mit einer Verbindung, die durch die Formel 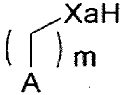 dargestellt wird, wobei Xa wie oben definiert ist und m und A wie in Anspruch 1 definiert sind, oder einem Salz davon.

7. Verbindung, die durch die Formel 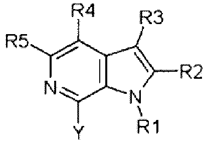 dargestellt wird, wobei Y eine Abgangsgruppe ist und die anderen Symbole wie in Anspruch 1 definiert sind, oder ein Salz davon.

8. Pharmazeutisches Mittel, das die Verbindung gemäß Anspruch 1 umfasst.

9. Protonenpumpenhemmer, der eine Verbindung, die durch die Formel (I) 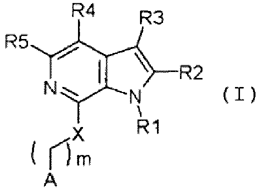 dargestellt wird, wie sie in Anspruch 1 definiert ist, oder ein Salz davon umfasst.

10. Pharmazeutisches Mittel gemäß Anspruch 8, bei dem es sich um ein Mittel für die Behandlung oder Prophylaxe von peptischem Geschwür, Zollinger-Ellison-Syndrom, Gastritis, Refluxösophagitis, gastroösophagealer Refluxkrankheit, die nicht mit einer Ösophagitis verbunden ist (symptomatischer gastroösophagealer Refluxkrankheit (symptomatischer GERD)), NUD (funktioneller Dyspepsie), Magenkrebs, Magen-MALT-Lymphom, Ulcus, der durch ein nichtsteroidales entzündungshemmendes Mittel oder Übersäuerung verursacht ist, und Ulcus aufgrund von postoperativem Stress, ein Mittel zur *Helicobacter-pylori-*Eradikation oder ein Mittel zur Unterdrückung einer oberen gastrointestinalen Blutung aufgrund von peptischem Geschwür, akutem Stress-Ulcus, hämorrhagischer Gastritis oder invasivem Stress handelt.

11. Verwendung der Verbindung gemäß Anspruch 1 zur Herstellung eines Mittels für die Behandlung oder Prophylaxe von peptischem Geschwür, Zollinger-Ellison-Syndrom, Gastritis, Refluxösophagitis, gastroösophagealer Refluxkrankheit, die nicht mit einer Ösophagitis verbunden ist (symptomatischer gastroösophagealer Refluxkrankheit (symptomatischer GERD)), NUD (funktioneller Dyspepsie), Magenkrebs, Magen-MALT-Lymphom, Ulcus, der durch ein nichtsteroidales entzündungshemmendes Mittel oder Übersäuerung verursacht ist, und Ulcus aufgrund von postoperativem Stress, eines Mittels zur *Helicobacter-pylori-*Eradikation oder eines Mittels zur Unterdrückung einer oberen gastrointestinalen Blutung aufgrund von peptischem Geschwür, akutem Stress-Ulcus, hämorrhagischer Gastritis oder invasivem Stress.

## Revendications

1. Composé représenté par la formule (I) : 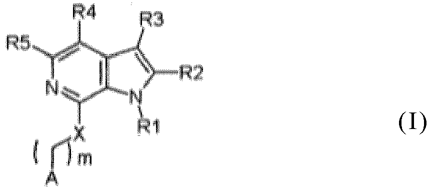 dans laquelle
- R1 représente
i) un atome d'hydrogène,
ii) ou un groupe hydrocarbyle choisi parmi les groupes alkyle en C₁₋₆, alcényle en C₂₋₆, alcynyle en C₂₋₆, cycloalkyle en C₃₋₇, aryle en C₆₋₁₄ et aralkyle en C₇₋₁₆ et les groupes représentés par la formule 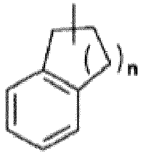 dans laquelle l'indice n vaut 1 ou 2, chacun desquels groupes étant, en option, porteur d'un ou de substituant(s) choisi(s) parmi les suivants : (1) atomes d'halogène, (2) nitro, (3) cyano, (4) hydroxy, (5) alcoxy en C₁₋₆, en option porteur de 1 à 3 atome(s) d'halogène, (6) aryloxy en C₆₋₁₄, (7) aralkyloxy en C₇₋₁₆, (8) sulfhydryle, (9) alkylthio en C₁₋₆, en option porteur de 1 à 3 atome(s) d'halogène, (10) arylthio en C₆₋₁₄, (11) aralkylthio en C₇₋₁₆, (12) amino, (13) mono(alkyle en C₁₋₆)-amino, (14) mono(aryle en C₆₋₁₄)-amino, (15) mono(aralkyle en C₇₋₁₆)-amino, (16) di(alkyle en C₁₋₆)-amino, (17) di(aryle en C₆₋₁₄)-amino, (18) di(aralkyle en C₇₋₁₆)-amino, (19) formyle, (20) (alkyle en C₁₋₆)-carbonyle, (21) (aryle en C₆₋₁₄)-carbonyle, (22) carboxyle, (23) (alcoxy en C₁₋₆)-carbonyle, (24) (aryloxy en C₆₋₁₄)-carbonyle, (25) carbamyle, (26) thiocarbamyle, (27) mono(alkyle en C₁₋₆)-carbamyle, (28) di(alkyle en C₁₋₆)-carbamyle, (29) (aryle en C₆₋₁₄)-carbamyle, (30) (alkyle en C₁₋₆)-sulfonyle, (31) (aryle en C₆₋₁₄)-sulfonyle, (32) (alkyle en C₁₋₆)-sulfinyle, (33) (aryle en C₆₋₁₄)-sulfinyle, (34) formyl-amino, (35) (alkyle en C₁₋₆)-carbonyl-amino, (36) (aryle en C₆₋₁₄)-carbonyl-amino, (37) (alcoxy en C₁₋₆)-carbonyl-amino, (38) (alkyle en C₁₋₆)-sulfonyl-amino, (39) (aryle en C₆₋₁₄)-sulfonyl-amino, (40) (alkyle en C₁₋₆)-carbonyl-oxy, (41) (aryle en C₆₋₁₄)-carbonyl-oxy, (42) (alcoxy en C₁₋₆)-carbonyl-oxy, (43) mono(alkyle en C₁₋₆)-carbamyl-oxy, (44) di(alkyle en C₁₋₆)-carbamyl-oxy, (45) (aryle en C₆₋₁₄)-carbamyl-oxy, (46) hétérocyclique de 5 à 10 chaînons comportant, outre des atomes de carbone, 1 à 4 hétéroatome(s) d'un ou de deux type(s) choisi(s) parmi les atomes d'azote, de soufre et d'oxygène, (47) (alcanediyle en C₁₋₃)-dioxy, (48) cycloalkyle en C₃₋₇,
- R2 représente un groupe alkyle en C₁₋₄,
- R3 représente un groupe alkyle en C₁₋₄,
- R4 et R5 représentent des entités identiques ou différentes, et représentent chacun un atome d'hydrogène ou un groupe alkyle en C₁₋₆,
- X représente une liaison, un chaînon symbolisé par O, ou un fragment de formule 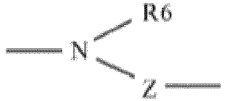 dans laquelle R6 représente un atome d'hydrogène ou un groupe alkyle en C₁₋₆, et Z représente une liaison ou un groupe carbonyle -C(=O)-,
- l'indice m vaut 0 ou 1,
- et A représente
a) un groupe hydrocarbyle choisi parmi
i) un groupe aryle en C₆₋₁₀, en option porteur de 1 à 4 substituant(s) choisi(s) parmi les atomes d'halogène et les groupes cyano, alkyle en C₁₋₆, en option porteurs de 1 à 5 substituant(s) halogéno, et alcoxy en C₁₋₆,
ii) un groupe aralkyle en C₇₋₁₆, en option porteur de 1 à 4 substituant(s) choisi(s) parmi les atomes d'halogène et les groupes cyano, alkyle en C₁₋₆, en option porteurs de 1 à 5 substituant(s) halogéno, et alcoxy en C₁₋₆,
iii) et un groupe de formule 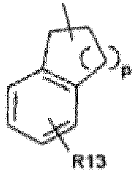 dans laquelle l'indice p vaut 1 ou 2 et R13 représente (i) un atome d'hydrogène ou (ii) un groupe alkyle en C₁₋₆, en option porteur de 1 à 4 substituant(s) choisi(s) parmi les groupes hydroxyle, cyano, et alcoxy en C₁₋₆,
b) ou un groupe hétérocyclique, monocyclique ou bicyclique, comportant de 5 à 14 chaînons et comportant, outre des atomes de carbone, 1 à 4 hétéroatome(s) d'un ou de deux type(s) choisi(s) parmi les atomes d'azote, d'oxygène et de soufre, et en option, porteur de 1 à 3 substituant(s) choisi(s) parmi les suivants : (1) atomes d'halogène, (2) nitro, (3) cyano, (4) alkyle en C₁₋₆, en option porteur de 1 à 3 atome(s) d'halogène, (5) aryle en C₆₋₁₄, (6) hydroxy, (7) alcoxy en C₁₋₆, en option porteur de 1 à 3 atome(s) d'halogène, (8) aryloxy en C₆₋₁₄, (9) aralkyloxy en C₇₋₁₆, (10) sulfhydryle, (11) alkylthio en C₁₋₆, en option porteur de 1 à 3 atome(s) d'halogène, (12) arylthio en C₆₋₁₄, (13) aralkylthio en C₇₋₁₆, (14) amino, (15) mono(alkyle en C₁₋₆)-amino, (16) mono(aryle en C₆₋₁₄)-amino, (17) mono(aralkyle en C₇₋₁₆)-amino, (18) di(alkyle en C₁₋₆)-amino, (19) di(aryle en C₆₋₁₄)-amino, (20) di(aralkyle en C₇₋₁₆)-amino, (21) formyle, (22) (alkyle en C₁₋₆)-carbonyle, (23) (aryle en C₆₋₁₄)-carbonyle, (24) carboxyle, (25) (alcoxy en C₁₋₆)-carbonyle, (26) (aryloxy en C₆₋₁₄)-carbonyle, (27) carbamyle, (28) thiocarbamyle, (29) mono(alkyle en C₁₋₆)-carbamyle, (30) di(alkyle en C₁₋₆)-carbamyle, (31) (aryle en C₆₋₁₄)-carbamyle, (32) (alkyle en C₁₋₆)-sulfonyle, (33) (aryle en C₆₋₁₄)-sulfonyle, (34) (alkyle en C₁₋₆)-sulfinyle, (35) (aryle en C₆₋₁₄)-sulfinyle, (36) formyl-amino, (37) (alkyle en C₁₋₆)-carbonyl-amino, (38) (aryle en C₆₋₁₄)-carbonyl-amino, (39) (alcoxy en C₁₋₆)-carbonyl-amino, (40) (alkyle en C₁₋₆)-sulfonyl-amino, (41) (aryle en C₆₋₁₄)-sulfonyl-amino, (42) (alkyle en C₁₋₆)-carbonyl-oxy, (43) (aryle en C₆₋₁₄)-carbonyl-oxy, (44) (alcoxy en C₁₋₆)-carbonyl-oxy, (45) mono(alkyle en C₁₋₆)-carbamyl-oxy, (46) di(alkyle en C₁₋₆)-carbamyl-oxy, (47) (aryle en C₆₋₁₄)-carbamyl-oxy, (48) cyclique saturé de 5 à 7 chaînons comportant, en option, 1 atome d'azote et comportant, outre des atomes de carbone, 1 à 4 hétéroatome(s) d'un ou de deux type(s) choisi(s) parmi les atomes d'azote, de soufre et d'oxygène, (49) hétérocyclique aromatique de 5 à 10 chaînons comportant, outre des atomes de carbone, 1 à 4 hétéroatome(s) d'un ou de deux type(s) choisi(s) parmi les atomes d'azote, de soufre et d'oxygène, (50) (alcanediyle en C₁₋₃)-dioxy, (51) cycloalkyle en C₃₋₇,
ou sel d'un tel composé.

2. Composé conforme à la revendication 1, dans lequel R1 représente
i) un atome d'hydrogène,
ii) un groupe alkyle en C₁₋₆, en option porteur d'un ou de substituant(s) choisi(s) parmi les atomes d'halogène et les groupes hydroxy, mono(alkyle en C₁₋₆)-amino, di(alkyle en C₁₋₆)-amino, alcoxy en C₁₋₆, aralkyl-oxy en C₇₋₁₆, cycloalkyle en C₃₋₇, et hétérocyclique à 5 ou 6 chaînons,
iii) un groupe alcényle en C₂₋₆,
iv) ou un groupe aralkyle en C₇₋₁₆, en option porteur d'un substituant alcoxy en C₁₋₆.

3. Composé conforme à la revendication 1, dans lequel l'indice m vaut 1.

4. Composé conforme à la revendication 1, dans lequel A représente
i) un groupe aryle en C₆₋₁₄, en option porteur de substituant(s) choisi(s) parmi les atomes d'halogène et les groupes cyano, alkyle en C₁₋₆, en option porteurs de 1 à 5 substituant(s) halogéno, et alcoxy en C₁₋₆,
ii) un groupe hétérocyclique comportant 5 ou 6 chaînons, en option porteur d'un ou de substituant(s) choisi(s) parmi les atomes d'halogène et les groupes cyano, alkyle en C₁₋₆ et alcoxy en C₁₋₆,
iii) un groupe 2,3-dihydro-1H-indén-1-yle,
iv) ou un groupe 1,2,3,4-tétrahydro-napht-1-yle.

5. Composé conforme à la revendication 1, choisi parmi les suivants :
N-(2,3-dihydro-1H-indén-1-yl)-2,3-diméthyl-1H-pyrrolo[2,3-c]pyridine-7-amine,
N-(4-fluoro-2-méthyl-benzyl)-2,3-diméthyl-1-propyl-1H-pyrrolo-[2,3-c]pyridine-7-amine,
N-[7-(2,3-diméthyl-1H-pyrrolo[2,3-c]pyridyl)]-benzamide.

6. Procédé de préparation d'un composé représenté par la formule 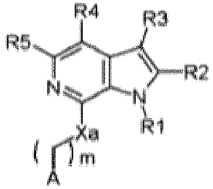 dans laquelle
- R1, R2, R3, R4, R5, m et A ont les significations indiquées dans la revendication 1,
- et Xa représente un chaînon symbolisé par O, ou un fragment de formule 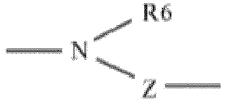 dans laquelle R6 représente un atome d'hydrogène ou un groupe alkyle en C₁₋₆, et Z représente une liaison ou un groupe carbonyle -C(=O)-,
ou d'un sel d'un tel composé, lequel procédé comporte le fait de faire réagir un composé de formule 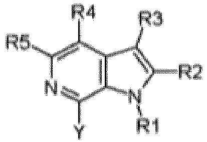 dans laquelle Y représente un groupe partant et les autres symboles ont les significations indiquées dans la revendication 1,
ou un sel d'un tel composé, avec un composé de formule 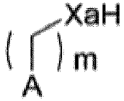 dans laquelle Xa a la signification indiquée ci-dessus et m et A ont les significations indiquées dans la revendication 1,
ou avec un sel d'un tel composé.

7. Composé de formule 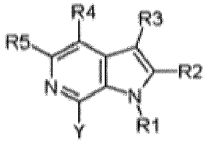 dans laquelle Y représente un groupe partant et les autres symboles ont les significations indiquées dans la revendication 1,
ou sel d'un tel composé.

8. Agent pharmaceutique comprenant un composé conforme à la revendication 1.

9. Inhibiteur de la pompe à protons, comprenant un composé représenté par la formule (I) : 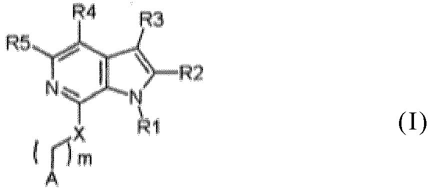 et tel que défini dans la revendication 1, ou un sel d'un tel composé.

10. Agent pharmaceutique conforme à la revendication 8, qui est un agent conçu pour le traitement ou la prophylaxie d'un ulcère gastro-duodénal, d'un syndrome de Zollinger-Ellison, d'une gastrite, d'une œsophagite peptique, d'un reflux gastro-œsophagien non accompagné d'œsophagite (maladie de reflux gastro-œsophagien symptomatique (REG symptomatique)), d'une dyspepsie non-ulcéreuse (DNU), d'un cancer de l'estomac, d'un lymphome de tissu MALT de l'estomac, d'un ulcère causé par un agent anti-inflammatoire non-stéroïdien ou par une hyperacidité, ou d'un ulcère dû à un stress post-opératoire ; un agent d'éradication d'Helicobacter pylori ; ou un agent de suppression de saignements des voies gastro-intestinales supérieures dus à un ulcère gastro-duodénal, un ulcère de stress aigu, une gastrite hémorragique ou un stress invasif.

11. Utilisation d'un composé conforme à la revendication 1 en vue de la fabrication d'un agent conçu pour le traitement ou la prophylaxie d'un ulcère gastro-duodénal, d'un syndrome de Zollinger-Ellison, d'une gastrite, d'une œsophagite peptique, d'un reflux gastro-œsophagien non accompagné d'œsophagite (maladie de reflux gastro-œsophagien symptomatique (REG symptomatique)), d'une dyspepsie non-ulcéreuse (DNU), d'un cancer de l'estomac, d'un lymphome de tissu MALT de l'estomac, d'un ulcère causé par un agent anti-inflammatoire non-stéroïdien ou par une hyperacidité, ou d'un ulcère dû à un stress post-opératoire ; un agent d'éradication d'Helicobacter pylori ; ou un agent de suppression de saignements des voies gastro-intestinales supérieures dus à un ulcère gastro-duodénal, un ulcère de stress aigu, une gastrite hémorragique ou un stress invasif.
